(19) Europäisches Patentamt
European Patent Office
Office européen des brevets

(11) **EP 4 663 207 A1**

(12) **EUROPEAN PATENT APPLICATION**
published in accordance with Art. 153(4) EPC

(43) Date of publication:
**17.12.2025 Bulletin 2025/51**

(21) Application number: **24753680.8**

(22) Date of filing: **08.02.2024**

(51) International Patent Classification (IPC):
***A61K 49/18*** *(2006.01)* ***A61K 9/00*** *(2006.01)*
***A61K 49/12*** *(2006.01)* ***A61K 47/69*** *(2017.01)*

(52) Cooperative Patent Classification (CPC):
**A61K 9/00; A61K 47/69; A61K 49/12; A61K 49/18**

(86) International application number:
**PCT/KR2024/001958**

(87) International publication number:
**WO 2024/167359 (15.08.2024 Gazette 2024/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.02.2023 KR 20230016863**
**09.02.2023 KR 20230017237**
**07.06.2023 KR 20230072904**
**19.10.2023 KR 20230140261**

(71) Applicant: **Inventera Inc.**
**Seoul 06588 (KR)**

(72) Inventors:
- **SHIN, Tae-Hyun**
  **Uiwang-si Gyeonggi-do 16003 (KR)**
- **KIM, Ji-wook**
  **Seoul 07361 (KR)**

(74) Representative: **Schiweck Weinzierl Koch Patentanwälte Partnerschaft mbB**
**Ganghoferstraße 68 B**
**80339 München (DE)**

# (54) LYMPHATIC VESSEL CONTRAST AGENT WITHOUT VENOUS CONTAMINATION

(57) The present invention relates to a formulation for non-intravenous administration, comprising nanoparticles having a hydrodynamic diameter of 2 to 20 nm and a surface charge ranging from -20 mV to 0 mV, the nanoparticles being adjusted in size and charge by hydration of hydrophilic functional groups exposed on the surface thereof, such that the nanoparticles are not permeated into or drained into blood capillaries at the administration site but are selectively drained into terminal lymphatic vessels, wherein the nanoparticles: (i) are themselves therapeutic agents; and/or (ii) serve as carriers for other therapeutic agents, and wherein the nanoparticles are engineered to provide a drug modality that, upon administration into a tissue site rather than via intravenous injection, is selectively drained into lymphatic vessels, thereby avoiding drainage into blood capillaries and thus prolonging residence time at the administration site.

According to the present invention, the nanoparticles with the adjusted hydrodynamic diameter and surface charge are configured to be completely cleared from the administration site within one week after administration without residual accumulation. When designed as a T1 MRI contrast agent, the nanoparticles enable selective imaging of peripheral and/or central lymphatic vessels without venous contamination when administered to perivascular tissue.

FIG. 1

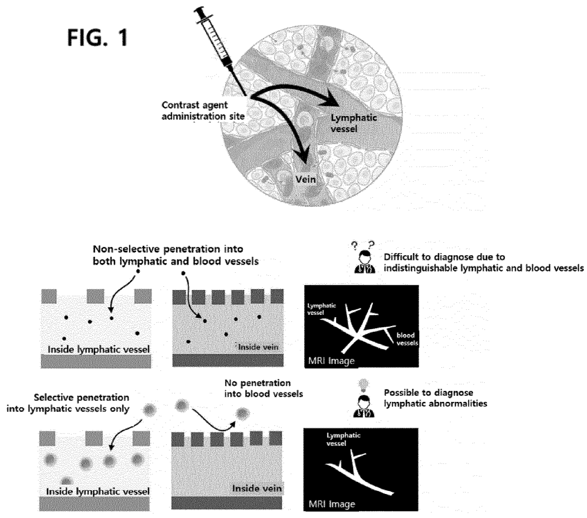


EP 4 663 207 A1

## Description

### TECHNICAL FIELD

The present invention relates to a formulation for non-intravenous administration, comprising nanoparticles having a hydrodynamic diameter of 2 to 20 nm and a surface charge ranging from -20 mV to 0 mV, the nanoparticles being adjusted in size and charge by hydration of hydrophilic functional groups exposed on the surface thereof, such that the nanoparticles are not permeated into or drained into blood capillaries at the administration site but are selectively drained into terminal lymphatic vessels, wherein the nanoparticles: (i) are themselves therapeutic agents; and/or (ii) serve as carriers for other therapeutic agents, and wherein the nanoparticles are engineered to provide a drug modality that, upon administration into a tissue site rather than via intravenous injection, is selectively drained into lymphatic vessels, thereby avoiding drainage into blood capillaries and thus prolonging residence time at the administration site.

According to the present invention, the nanoparticles with the adjusted hydrodynamic diameter and surface charge are configured to be completely cleared from the administration site within one week, preferably within 72 hours, after administration without residual accumulation. When designed as a T1 MRI contrast agent, the nanoparticles enable selective imaging of peripheral and/or central lymphatic vessels without venous contamination when administered to perivascular tissue.

### BACKGROUND ART

Nanomaterials exhibit novel physical and chemical properties that differ from those of bulk materials due to their reduced dimensions. Extensive research into nanomaterials has enabled not only control over particle size, but also the composition and morphology of such materials, thereby allowing the realization of superior physicochemical properties at the nanoscale.

Among the diverse types of nanoparticles, magnetic nanoparticles have been utilized in a wide range of nanobiotechnology applications, including the separation of biological materials, magnetic resonance imaging (MRI) diagnostics, magneto-biosensors incorporating giant magnetoresistance (GMR) sensors, microfluidic systems, drug and gene delivery, and magnetic hyperthermia therapy.

In particular, magnetic nanoparticles may be used as contrast agents for magnetic resonance imaging (MRI).

Magnetic resonance lymphangiography (MRL) is a medical imaging technique used to visualize the lymphatic system and assess its function.

Leveraging the advantages of magnetic resonance imaging (MRI), MRL has emerged as a valuable modality for imaging the lymphatic system. MRL offers several benefits, including high-resolution imaging, three-dimensional reconstruction, and coverage of broad anatomical regions, and has demonstrated clinical utility in surgical planning following cancer therapy and in monitoring the status of lymphedema.

To achieve enhanced contrast, a variety of contrast agents have been employed in MRL. Among them, gadolinium (Gd)-based chelate contrast agents are most widely used in clinical settings. However, due to their small molecular size, these agents undergo non-selective diffusion into the venous system, resulting in venous contamination. Because the lymphatic vessels and veins cannot be distinctly visualized, it is difficult to accurately visualize lymphatics using conventional Gd-chelate contrast agents.

To address this limitation, various nanoparticulate and macromolecular Gd-based contrast agents have been proposed in addition to low-molecular-weight Gd chelates. Nevertheless, Gd use must be restricted in patients with renal impairment to avoid the risk of nephrogenic systemic fibrosis (NSF). Furthermore, Gd deposition in the brain and other tissues has raised safety concerns. Ultrasmall iron oxide nanoparticles, such as ferumoxytol, have demonstrated potential for vascular imaging, making them attractive alternatives to Gd-based formulations. Despite this promise, successful implementation of ferumoxytol in standard MRL remains rare. In the only reported study, both Gd-based agents and ferumoxytol were administered; while the Gd-based agent enhanced both lymphatic and venous structures, ferumoxytol suppressed venous signals. In addition, ferumoxytol has not received FDA approval for imaging indications due to the risk of severe hypersensitivity reactions.

Lymphatic vessels return excess interstitial fluid to the bloodstream. These vessels originate as small, thin-walled endothelial channels within the interstitium, which progressively coalesce into larger conduits. The initial lymphatic vessels, also referred to as peripheral lymphatics, resemble blood capillaries but contain numerous endothelial junctions that function as unidirectional microvalves known as primary lymphatic valves. Anchoring filaments tether the initial lymphatics to the surrounding connective tissue. Larger collecting lymphatic vessels have walls similar to those of small veins and are composed of endothelium and sparse smooth muscle. Similar to veins, the larger lymphatic vessels contain secondary valves that prevent retrograde lymphatic flow. Lymph nodes are located along the paths of collecting lymphatic vessels. Ultimately, the major lymphatic trunks drain into the left and right subclavian veins.

Lymphedema is a condition characterized by the accumulation of fluid and proteins in tissues due to an imbalance

between the volume of interstitial fluid filtered from the blood and the capacity of the lymphatic vessels to transport it. Although it most commonly affects the extremities, lymphedema can also occur in other body regions, including the face, back, abdomen, chest, and genitalia.

Lymphedema results from the interstitial accumulation of lymphatic fluid due to injury or dysfunction of lymphatic vessels or lymph nodes. Imaging evaluation is performed when lymphedema is clinically ambiguous or when a more definitive diagnosis is necessary for prognosis and treatment planning. However, direct visualization of lymphatic vessels is technically challenging because they are extremely small-typically 0.4 to 0.8 mm in diameter-and transparent. To address this limitation, magnetic resonance lymphangiography (MRL), utilizing contrast agents capable of visualizing lymphatic vessels and lymphedematous tissues, has become widely adopted in clinical practice.

Surgical interventions for lymphedema have increased in recent years, and correspondingly, the use of MRL to evaluate lymphatic structures has also risen. Significant advancements have been made, ranging from conventional T2-weighted imaging to three-dimensional (3D) imaging techniques. Among these, 3D spoiled gradient echo sequences and their variants are commonly employed, and fat suppression techniques-particularly the mDixon method-have gained prominence for optimizing image contrast.

Lymphedema has been reported to occur in more than 20% of patients with breast, cervical, or ovarian cancer, and in approximately 15.5% of all cancer patients.

Computed tomography (CT) and magnetic resonance imaging (MRI) are useful for distinguishing lymphedema from conditions such as lipedema or subcutaneous fat thickening, as well as for identifying characteristic honeycomb patterns of lymphedema, lymphatic obstruction secondary to tumor metastasis, the extent of swelling, and the presence of vascular stenosis or occlusion.

In 2000, Koshima et al. introduced supermicrosurgical techniques that enabled anastomosis of lymphatic vessels smaller than 0.8 mm to venules. Since then, surgical treatment of lymphedema has regained attention, and reconstructive procedures aimed at alleviating lymphedema have become increasingly common. The most frequently performed procedures include lymphatico-venular anastomosis (LVA) and vascularized lymph node transfer. Early surgical intervention for secondary lymphedema is generally associated with improved therapeutic outcomes.

Microsurgical lymphatic reconstruction involves connecting the lymphatic vessels and/or lymph nodes at the site of lymphatic stasis to physiologically functioning lymphatic vessels or veins. Although long-term studies have demonstrated efficacy in treating limb lymphedema, the effectiveness is limited primarily to early-stage disease. Furthermore, the procedure requires a high degree of microsurgical expertise, and as a result, is not widely performed.

Imaging evaluation of lymphedema is performed when the clinical diagnosis is uncertain or when a more definitive assessment is needed to guide prognosis and treatment planning. Differential diagnoses in cases of unclear lymphedema include pathologic obesity, lipodystrophy, endocrine dysfunction, venous insufficiency, unrecognized trauma, recurrent infections, neoplasms, and venous malformations. With the increasing number of surgical interventions for lymphedema, clinical demand for MRI has also grown significantly.

Clinically available imaging modalities include bioelectric impedance spectroscopy, lymphoscintigraphy, indocyanine green lymphography (hereinafter referred to as ICGL), and magnetic resonance imaging (MRI). Direct lymphangiography was commonly used during the 1970s and 1980s, prior to the advent of computed tomography (CT) and MRI, and was developed primarily to visualize lymphatic vessels and detect lymph node metastases. The lymphatic vessels serve as conduits for the transport of bodily fluids other than blood. A blue dye was injected into the interdigital skin between the toes to identify lymphatic vessels, followed by skin incision and insertion of a 30-gauge needle, through which iodinated oil was slowly injected to obtain continuous X-ray images. Lymph node visualization was typically achieved 24 hours post-injection. However, this procedure has largely been replaced by modern imaging techniques due to its invasiveness and associated risks, including patient discomfort and rare but serious complications such as pulmonary embolism and pulmonary edema.

Bioelectric impedance spectroscopy estimates the volume of intracellular fluid in the limbs by measuring electrical resistance. Lymphoscintigraphy is the most commonly used diagnostic method for lymphedema and is employed to evaluate lymph node uptake and assess lymphatic flow or dermal backflow. However, its resolution and specificity are inferior to those of ICGL and MRI. MRI demonstrates higher resolution than lymphoscintigraphy in detecting lymphatic vessel abnormalities, whereas lymphoscintigraphy exhibits greater specificity for visualizing lymph nodes, allowing for detection of a larger number of nodes. Indocyanine green (ICG) is commonly used in sentinel lymph node biopsy. ICGL involves subcutaneous injection of ICG followed by near-infrared fluorescence imaging of superficial lymphatic vessels. Compared to lymphoscintigraphy, ICGL is less invasive and more cost-effective. However, ICGL is limited in its ability to visualize deeper lymphatic structures, for which MRI is more suitable. ICG is also frequently used intraoperatively as a visually traceable contrast agent during lymphatico-venular anastomosis (LVA), allowing for real-time fluorescence imaging.

Among available modalities, MRI provides the highest contrast resolution and spatial resolution for visualizing lymphatic vessels and lymph nodes. Due to its low resolution, lymphoscintigraphy is inadequate for delineating individual lymphatic vessels. ICGL, while useful for visualizing superficial lymphatics, has limited penetration depth (less than 2 cm)

and cannot capture deeper structures. In contrast, magnetic resonance lymphangiography (MRL) enables visualization of lymphatic vessels regardless of depth or subcutaneous tissue composition. In cases where lymphatics are not visualized, the condition is often consistent with chronic lymphedema, and MRL may reveal associated features such as tissue fibrosis and fat hypertrophy. MRL can be performed using either 1.5 Tesla or 3.0 Tesla MRI systems.

In magnetic resonance lymphangiography (MRL), lymphatic vessels typically appear tortuous, beaded, irregular, and discontinuous in shape, and they generally become more conspicuous over time, allowing for distinction from veins. Dermal backflow can be diagnosed as an area demonstrating progressive and diffuse spread of gadolinium (Gd)-based contrast agent into the dermal tissue.

Gd-based contrast agents are administered via intradermal injection into the interdigital spaces of the hands and feet, typically distributed across four interdigital sites. A mixture of 4 to 5 mL of contrast agent with 0.5 to 1 mL of lidocaine or mepivacaine is commonly used. While gadolinium-based contrast agents are commonly used at a standard concentration of 0.5 mmol/mL, higher concentrations, such as 1.0 mmol/mL, may also be employed in some cases. Experimental contrast agents are currently under investigation. For example, gadofosveset, which binds to human serum albumin (HSA), has demonstrated superior performance compared to gadolinium-diethylenetriamine pentaacetic acid (Gd-DTPA) in preclinical imaging studies of lymphatic vessels and lymph nodes. The gadofosveset-HSA complex, being a macromolecular contrast agent, is considered comparable to agents used in nuclear medicine imaging such as sulfur colloid and albumin. In addition, Gd-labeled dendrimers have also shown promising results as macromolecular contrast agents for MRL in animal models.

A recently proposed imaging strategy utilizes both ferumoxytol and Gd-DTPA to suppress venous enhancement and selectively visualize lymphatic structures. Ferumoxytol, originally developed as a therapeutic agent for iron-deficiency anemia, requires slow intravenous infusion over 15 minutes or more due to the risk of severe hypersensitivity reactions. As such, its use for imaging purposes remains off-label and is subject to regulatory restrictions.

Contrast agents are pharmaceutical substances used to enhance the visibility of organs or tissues during imaging studies such as computed tomography (CT) scans, magnetic resonance imaging (MRI), and angiography. These agents are typically approved for use in connection with specific medical conditions or for designated imaging modalities.

Off-label use of a contrast agent refers to its use in a manner that is not consistent with its approved indications. This may include use in a different medical condition, use with an imaging modality other than that for which it was approved, or use at a concentration or dosage that deviates from the approved formulation, such as when the contrast agent is diluted manually at the point of care in a clinical setting. Off-label use is not uncommon and may be beneficial in certain situations, particularly when no alternative approved contrast agent is available for the diagnostic objective. However, such use is accompanied by inherent risks, including the possibility of contamination during dilution and subsequent septic complications, as the safety and efficacy of the contrast agent under these conditions are not fully established.

In current clinical practice, certain gadolinium (Gd)-based vascular contrast agents are being used off-label for lymphatic imaging. In these cases, the contrast agent, which is originally formulated at a concentration of approximately 500 mM, must be diluted more than tenfold to be suitable for lymphatic application. Such bedside dilution procedures present risks of dilution errors or contamination of the injectable solution, thereby increasing the likelihood of infection. Moreover, unless the agent is directly administered into the lymphatic vessels, its small molecular size allows it to non-selectively permeate both lymphatic and blood vessels, resulting in venous contamination and preventing clear distinction between the two systems (see FIG. 1).

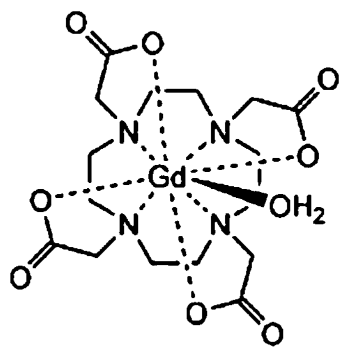

Furthermore, it has been observed that repeated and prolonged administration of current MRI contrast agents can lead to increased signal intensity in brain tissues due to gadolinium deposition. Such intracerebral gadolinium accumulation has been shown to correlate with both the cumulative dose and frequency of administration, and can occur even in individuals without underlying medical conditions. In addition to the brain, gadolinium deposition has also been reported in bones, visceral organs, and skeletal muscle. Notably, linear gadolinium-based contrast agents are known to

exhibit a higher propensity for tissue retention, and their use has been increasingly restricted worldwide.

## DETAILED DESCRIPTION OF THE INVENTION

## TECHNICAL PROBLEM

Conventional contrast agents are administered directly into lymphatic vessels prior to MR imaging. However, it is technically challenging to inject contrast agents directly into peripheral lymphatic vessels that are less than 0.8 mm in diameter.

To address the lack of MRI contrast agents approved for lymphatic indications, despite the urgent need for lymphatic-specific contrast agents, the present invention provides nanoparticles engineered with a hydrodynamic diameter and/or surface charge adjusted such that, when administered into tissue surrounding blood vessels (see FIG. 1), the nanoparticles are not permeated into or drained into blood capillaries at the administration site but are selectively drained into terminal lymphatic vessels only.

## TECHNICAL SOLUTION

According to a first aspect of the present invention, there is provided a formulation for non-intravenous administration, comprising nanoparticles having a hydrodynamic diameter of 2 to 20 nm and a surface charge ranging from -20 mV to 0 mV, the nanoparticles being adjusted in size and charge by hydration of hydrophilic functional groups exposed on the surface thereof, such that the nanoparticles are not permeated into or drained into blood capillaries at the administration site but are selectively drained into terminal lymphatic vessels, wherein the nanoparticles: (i) are themselves therapeutic agents; and/or (ii) serve as carriers for other therapeutic agents, and wherein the nanoparticles are engineered to provide a drug modality that, upon administration into a tissue site rather than via intravenous injection, is selectively drained into lymphatic vessels, thereby avoiding drainage into blood capillaries and thus prolonging residence time at the administration site.

According to a first aspect of the present invention, there is provided a formulation for non-intravenous administration, comprising nanoparticles having a hydrodynamic diameter of 2 to 20 nm and a surface charge ranging from -20 mV to 0 mV, the nanoparticles being adjusted in size and charge by hydration of hydrophilic functional groups exposed on the surface thereof, such that the nanoparticles are not permeated into or drained into blood capillaries at the administration site but are selectively drained into terminal lymphatic vessels, wherein the nanoparticles: (i) are themselves therapeutic agents; and/or (ii) serve as carriers for other therapeutic agents, and wherein the nanoparticles are engineered to provide a drug modality that, upon administration into a tissue site rather than via intravenous injection, is selectively drained into lymphatic vessels, thereby avoiding drainage into blood capillaries and thus prolonging residence time at the administration site.

According to a second aspect of the present invention, there is provided a contrast agent composition engineered for non-intravenous administration, comprising nanoparticles having a hydrodynamic diameter of 2 to 20 nm and a surface charge of -20 mV to 0 mV, wherein the nanoparticles are configured such that they are substantially excluded from entering or being drained into blood capillaries at the administration site and are selectively drained into lymphatic vessels, such that, upon administration to tissue other than via intravenous injection, the composition enables selective imaging of peripheral and central lymphatic vessels without venous contamination.

According to a third aspect of the present invention, there is provided a method of providing diagnostic information regarding lymphatic diseases without venous contamination, comprising administering the contrast agent composition of the second aspect of the present invention.

According to a fourth aspect of the present invention, there is provided a formulation for non-intravenous administration, comprising polysaccharide-crosslinked colloidal particles having: a hydrodynamic diameter of 2 to 20 nm, preferably 10 nm or less, more preferably 8 nm or less, and most preferably 6 nm or less; and • a surface charge ranging from -20 mV to 0 mV, wherein the polysaccharide-crosslinked colloidal particles are formed by intra- and/or inter-molecular crosslinking of 1 to 3 branched polysaccharides or 2 to 30 cyclic polysaccharides at hydroxyl groups of constituent monosaccharide building blocks using a crosslinker, such that the particles are resistant to enzymatic hydrolysis in vivo; and wherein the formulation is engineered to provide a drug modality that is selectively drained into lymphatic vessels when administered to tissue or a body cavity other than via intravenous injection, thereby avoiding drainage into blood capillaries and thus prolonging residence time at the administration site to 60 minutes or more.

The present invention will now be described.

As described above, gadolinium-based contrast agents (GBCAs) are currently used for MR lymphangiography (MRL). However, GBCAs simultaneously visualize both lymphatic vessels and normal veins, making it difficult to distinguish between them. Normal veins often obscure lymphatic vessels and impair the diagnostic value of MRL-a phenomenon referred to as *venous contamination.*

To overcome this limitation, Examples 1 to 3 utilize INV-001, a non-gadolinium nanoparticulate MRI contrast agent for MRL, which consists of a dextran-based core and an iron oxide shell. Due to its hydrodynamic size of approximately 4 nm-smaller than the renal filtration threshold-intracutaneously injected INV-001 is not absorbed into venous circulation but is instead taken up exclusively via the lymphatic system and subsequently excreted in the urine. Accordingly, Example 1 demonstrated the potential of INV-001 as an effective contrast agent for venous contamination-free MRL to improve diagnosis and monitoring of lymphatic status.

Specifically, all animals used in Example 1 were male Sprague-Dawley (SD) rats weighing 250-300 g. The prepared contrast agent was administered intradermally/subcutaneously into the fourth digits of both hind limbs using a 30-gauge needle. MRL was performed every 16 minutes using a 3D time-of-flight (TOF) sequence with a saturation band on a 9.4-T preclinical MRI scanner.

As a result, unlike Gd-DOTA, which showed venous contamination in most animals regardless of the injection dose and condition, INV-001 exhibited no venous contamination. In the case of Gd-DOTA, the popliteal lymph nodes and lymphatic vessels reached peak enhancement at 16 minutes post-injection but were rapidly washed out thereafter. In contrast, INV-001 reached maximum enhancement between 16 and 32 minutes post-injection, enabling prolonged visualization of the popliteal lymph nodes and lymphatic vessels. INV-001 administered at 0.45 μmol (15 mM, 30 μL) and 0.75 μmol (15 mM, 50 μL) provided excellent visualization of popliteal lymph nodes and lymphatic vessels without venous contamination, interstitial leakage, or lymph node swelling, and received high qualitative scores in image analysis.

Therefore, the iron-based T1 contrast agent INV-001 enables prolonged enhancement of popliteal lymph nodes and lymphatic vessels in MRL without venous contamination.

In Example 3, the novel contrast agent INV-001, composed of a dextran core and an iron oxide shell, demonstrated contrast enhancement and visualization of lymphatic vessels and lymph nodes without venous contamination via MRL in beagle dogs.

Using optimized concentrations and dosing of INV-001 for MRL, the study clearly demonstrated selective visualization of lymph nodes and lymphatic vessels without venous contamination in healthy beagle dogs, confirming the excellent MRL efficacy of this novel dextran core-iron oxide shell formulation.

Based on quantitative and qualitative analyses, the optimal concentration was determined to be 15 mM, and the recommended doses for MRL were 0.056 and 0.112 mg Fe/kg. Clinically, the typical concentration of gadolinium-based MRI contrast agents used for MRL is 500-1,000 mmol/L, with a recommended dose of 0.1-0.2 mL/kg. However, Example 3 achieved selective visualization of lymphatic vessels at lower doses of 0.067 and 0.13 mL/kg without venous contamination, offering a significant clinical advantage. These findings are of substantial academic importance and contribute to understanding the appropriate dosing parameters for MRL using INV-001 in preclinical studies involving beagle dogs.

Therefore, INV-001, which differs in composition from conventional gadolinium-based MRI contrast agents (containing 0.84 mg/mL iron and 28.0 mg/mL dextran), enables lymphatic vessel-specific imaging without venous contamination following intradermally/subcutaneously administration.

In Example 7, it was confirmed that the dextran-crosslinked nanoparticles used in Example 1-formed by intra- and/or inter-molecular crosslinking of 1 to 3 dextran or dextran derivatives at hydroxyl groups of glucose building blocks using a crosslinker-can be extended to polysaccharide-crosslinked colloidal particles. These are formed by intra- and/or inter-molecular crosslinking of 1 to 3 branched polysaccharides or 2 to 30 cyclic polysaccharides at the hydroxyl groups of their constituent monosaccharide building blocks using a crosslinker.

The present invention is based on this finding.

The non-intravenous formulation of the present invention comprises nanoparticles having a hydrodynamic diameter of 2 to 20 nm and a surface charge ranging from -20 mV to 0 mV, the size and charge of which are adjusted via hydration of hydrophilic functional groups exposed on the nanoparticle surface, such that the nanoparticles are not permeated into or drained into blood capillaries at the administration site but are selectively drained into terminal lymphatic vessels only. The nanoparticles (i) are themselves therapeutic agents and/or (ii) serve as carriers for other therapeutic agents. Upon administration into a tissue site rather than via intravenous injection, the nanoparticles are selectively drained into lymphatic vessels, thereby extending the residence time at the administration site and providing a unique drug modality.

According to the present invention, when such size-controlled nanoparticles serve as MRI contrast agents that are not permeated into or drained into blood capillaries at the administration site but are selectively drained into terminal lymphatic vessels only, the formulation can provide high-resolution imaging that delineates individual lymphatic vessels regardless of depth or subcutaneous tissue condition at the administration site (see FIGS. 1 and 14).

Accordingly, the contrast agent composition for non-intravenous administration of the present invention is further characterized in that, upon administration into target tissue rather than intravenously, it enables selective imaging of peripheral and central lymphatic vessels without venous contamination. The formulation contains nanoparticles having a hydrodynamic diameter of 2 to 20 nm and a surface charge of -20 mV to 0 mV, adjusted such that they are not permeated into or drained into blood capillaries at the administration site but are selectively drained into terminal lymphatic vessels only.

The nanoparticles of the present invention are further characterized in that their surface-exposed hydrophilic functional groups, as well as their hydrodynamic diameter and/or surface charge, are engineered in a manner that enables them to be drained into the lymphatic vessels connected to the body's circulatory system without causing lymph node congestion. As a result, the nanoparticles can be eliminated via venous circulation without systemic accumulation and/or without triggering inflammatory responses.

**[Modality of Nanoparticles]**

According to the present invention, the nanoparticles-having a hydrodynamic diameter of 2 to 20 nm and a surface charge ranging from -20 mV to 0 mV, adjusted via hydration of hydrophilic functional groups exposed on the surface thereof-are not drained into blood capillaries at the administration site but are selectively drained into terminal lymphatic vessels only. The nanoparticles serve as therapeutic agents themselves and/or as carriers for other therapeutic agents.

As used herein, the term "therapeutic agent" refers to any substance, excluding food or medical devices, that is administered for the purpose of diagnosing, curing, alleviating, treating, or preventing a disease, or otherwise affecting the structure or function of the body. For example, this includes any chemical or biological substance that exerts an effect on physiological processes or metabolism within the body.

In the present specification, the term "therapeutic agent" encompasses not only molecular-level therapeutic agents but also functional nanoparticles engineered for in vivo administration, provided they satisfy the foregoing definition. Contrast agents are also considered a subclass of therapeutic agents.

As used herein, "drug modality" refers to the various modes by which a therapeutic agent can be delivered to the body, including oral, topical, intravenous, intramuscular, and intradermal/subcutaneous routes. The selected modality may influence the rate of absorption and metabolism of the agent, which in turn affects its therapeutic efficacy. Accordingly, appropriate selection of the route of administration constitutes a critical aspect of both effective pharmacotherapy and diagnostic imaging.

According to the present invention, nanoparticles engineered to have a controlled hydrodynamic diameter and surface charge through hydration of hydrophilic functional groups exposed on the surface thereof, such that they do not enter blood capillaries at the injection site but are selectively drained only into terminal lymphatic vessels, can be administered into peripheral regions of the body to be drained into lymphatic vessels or directly injected into lymphatic vessels. Once drained into the lymphatic vessels, the nanoparticles may be absorbed into the circulatory system without being removed by lymphocytes or macrophages in the lymph nodes.

Both peripheral lymphatic vessels and blood capillaries are components of the circulatory system.

Peripheral lymphatic vessels form part of the lymphatic system and function to collect lymphatic fluid, waste products, and cellular debris from tissues and return them to the circulatory system. These lymphatic vessels are generally larger in diameter and have thicker walls than blood capillaries. Moreover, lymphatic vessels are equipped with unidirectional valves that prevent backflow and facilitate fluid transport toward the circulatory system.

In contrast, blood capillaries are the smallest blood vessels in the body and form a dense network of microtubules connecting arterioles and venules. Capillaries account for approximately 90% of the body's vasculature, spanning about 95,000 kilometers, and are present in all organs, serving as the primary site for the exchange of oxygen, nutrients, and waste between the blood and tissues. Capillaries have a diameter typically ranging from 30 to 40 $\mu$m, with narrower segments around 8 $\mu$m, just wide enough for a single red blood cell to pass through, thereby providing an extensive surface area for molecular exchange. Composed of a single layer of endothelial cells, blood capillaries enable facile diffusion and exchange of substances with surrounding tissues.

The circulatory system is responsible for the flow of bodily fluids such as blood and lymph, supplying organs throughout the body with nutrients, oxygen, and energy, and delivering carbon dioxide and metabolic waste products generated from vital activities to the respiratory or urinary systems for excretion from the body.

Blood circulation is driven by the pumping action of the heart. Circulating blood performs functions such as transporting oxygen, supplying nutrients, removing metabolic waste products, maintaining body temperature, and delivering hormones.

A substantial amount of ions, nutrients, organic waste, dissolved gases, and water is exchanged through the blood capillaries, most of which is reabsorbed into the blood capillaries. The volume of fluid that exits and reenters the blood capillaries is nearly equal, with only a portion absorbed through the lymphatic vessels. This portion flows into the lymphatic vessels and ultimately returns to the blood circulation.

A lymphatic vessel is a transparent, one-way, blind-ended vessel located primarily beneath the skin or mucosal layers, responsible for transporting fluid and substances into the vascular system. Lymphatic vessels carry lymph from tissues to lymph nodes, and from the lymph nodes back to the vascular system. After transporting lymphatic fluid from tissues to lymph nodes, the lymphatic vessels return the fluid to the venous circulation through two collecting ducts. Lymphatic vessels are more permeable than blood capillaries, allowing them to absorb macromolecules-including

antigens and cells-more readily than blood capillaries. Meanwhile, lymph nodes are distributed throughout the body along the lymphatic vessels and are the sites where lymphatic vessels and lymphatic pathways are connected. Lymph nodes discharge into collecting ducts, which in turn drain their contents into the two subclavian veins located beneath the clavicles. These veins converge to form the superior vena cava, a large vein that returns blood from the upper body to the heart.

Lymph is a pale yellow fluid that contains less protein but more lipid components than blood, and is rich in lymphocytes and leukocytes. As lymph circulates throughout the body via the lymphatic vessels, it delivers nutrients to cells and removes waste products. Lymphocytes play a key role in immune responses, defending the body against invading bacteria, viruses, and other pathogens.

After circulating through the body via arteries, blood returns through the veins, during which a portion of the fluid remains between cells as interstitial fluid-an extracellular fluid formed by plasma filtered through blood capillaries into the tissue. When this interstitial fluid drains into terminal lymphatic vessels, it is referred to as lymph. Lymph enters the lymphatic vessels at a very slow rate and eventually flows back into the bloodstream. As it seeps out of the walls of blood capillaries and bathes the body's tissue cells, lymph removes waste products from the tissues.

As lymph circulates throughout the body via lymphatic vessels, it delivers nutrients to cells and transports carbon dioxide ($CO_2$), damaged cells, cancer cells, bacteria, and other waste materials. After completing this process, the lymph reenters the bloodstream at the junction of the internal jugular vein and the subclavian vein. During its passage through the lymph nodes, lymphocytes within the lymph nodes interact with foreign substances to eliminate or destroy them. This is why lymph nodes become swollen in response to acute inflammation, as they filter and trap invading foreign substances.

Accordingly, it is preferable that the nanoparticles engineered to be absorbed into lymphatic vessels upon administration into peripheral regions of the body, or to be directly injected into lymphatic vessels according to the present invention, are not removed by lymphocytes or macrophages in the lymph nodes but instead absorbed into the circulatory system.

The total length of blood vessels in the human body is approximately 120,000 kilometers, enabling circulation equivalent to traveling around the Earth two and a half times in under one minute.

The nanoparticles according to the present invention are intended for in vivo administration and, therefore, are considered a type of drug.

Once a drug enters the body, elimination processes are initiated. The primary pathways for drug elimination include: (1) hepatic metabolism, (2) biliary excretion, and (3) urinary excretion.

Elimination encompasses both biotransformation (drug metabolism) and excretion. Excretion refers to the removal of the intact drug from the body.

As blood circulates through the vasculature, it is filtered by the kidneys, where waste is excreted through the bladder in the form of urine. The size of the glomerular filtration pores in the kidney is generally known to be approximately 6 to 8 nm. In theory, substances within or below this size range should be capable of passing through the glomerular filter; however, rigid materials such as inorganic nanoparticles can effectively pass through only when their size is 5.5 nm or smaller. In other words, both the size and rigidity of a substance play critical roles in its renal clearance via glomerular filtration.

A high proportion of nanoparticles retained in organs such as the liver and spleen indicates unsatisfactory excretion performance.

Accordingly, the nanoparticles of the present invention may (a) comprise polysaccharide-crosslinked colloidal particles formed by intra- and/or inter-molecular crosslinking of 1 to 3 branched polysaccharides or 2 to 30 cyclic polysaccharides at the hydroxyl groups of their constituent monosaccharide units with a crosslinking agent, such that they are not hydrolyzed by endogenous enzymes; or (b) be surface-coated with the aforementioned polysaccharide-crosslinked colloidal particles. In either case, hydration of hydrophilic functional groups exposed on the surface of the nanoparticle imparts deformability (squeezability) during interstitial transport in vivo. Furthermore, once absorbed into the circulatory system via the lymphatic vessels after administration, the nanoparticles may be engineered for selective delivery to a target site by modulating vascular permeability and in vivo absorption, and for controllable renal excretion rate and timing, thereby regulating circulation duration or degree of clearance. As such, the nanoparticles are advantageous for use in the diagnosis and/or treatment of diseases without in vivo accumulation.

According to the present invention, nanoparticles engineered to avoid drainage into blood capillaries at the injection site but to be selectively drained only into terminal lymphatic vessels-preferably without causing lymph node congestion-are rendered deformable (squeezable) via hydration of hydrophilic functional groups exposed on the surface thereof, with their hydrodynamic diameter and/or surface charge being controlled. By engineering the nanoparticles to be impermeable to blood capillaries, they are drained from the injection site into lymphatic vessels, absorbed into the circulatory system, and thereafter cleared through (1) hepatic metabolism, (2) biliary excretion, or (3) urinary excretion (see Example 2), without undergoing leakage across blood vessel walls or diffusion into interstitial fluid during circulation.

For example, according to the present invention, nanoparticles comprising a spherical dextran-crosslinked core and a discontinuous shell in which divalent or trivalent iron ions are coordinated to hydrophilic functional groups derived

from a crosslinking agent on the surface of the core may be engineered such that, upon in vivo administration, they do not accumulate in tissues but are fully absorbed into the circulatory system via lymphatic drainage and subsequently collected in urine via renal filtration (see Example 4, FIGS. 6 to 10).

The nanoparticles described in Example 4 are shown to be excreted 100% in urine after in vivo administration without accumulation in normal tissues, indicating that they are eliminated exclusively via (1) urinary excretion, without undergoing (2) hepatic metabolism or (3) biliary excretion.

Accordingly, the nanoparticles of the present invention may be collected via urinary excretion at an amount of 80% or greater, preferably 90% or greater, and more preferably 95% or greater, relative to the administered dose.

Therefore, since the nanoparticles of the present invention may be absorbed, distributed, and/or excreted in an intact form-without metabolic degradation-along their in vivo pharmacokinetic pathways and subsequently collected in urine, they are also amenable to reuse or recycling. In addition, they may be used to collect biological information from the injection site, lymphatic system, or bloodstream via adsorption or similar mechanisms.

**[Administration Site, Residence Time, and Excretion of Nanoparticles]**

As illustrated in FIG. 1, which conceptually shows the administration site and in vivo absorption pathway of the contrast agent, the nanoparticles of the present invention are administered not intravenously but to a tissue region adjacent to a blood vessel.

The tissue region adjacent to a blood vessel where the nanoparticles of the present invention are administered may be connective tissue (FIG. 1). Connective tissue constitutes the majority of vertebrate bodies and contains cells (e.g., fibroblasts, leukocytes, adipocytes) embedded in a non-cellular extracellular matrix.

The tissue region adjacent to a blood vessel where the nanoparticles of the present invention are administered may also be a body cavity formed in epithelial tissue. For example, the joint cavity and intrathecal space are included.

According to the present invention, nanoparticles having a hydrodynamic diameter of 2 to 20 nm and a surface charge of -20 mV to 0 mV, which are regulated via hydration of hydrophilic functional groups exposed on the surface thereof such that they are not absorbed into or discharged through blood capillaries at the administration site but are selectively discharged only through terminal lymphatic vessels, can be excreted exclusively via the lymphatic system. Accordingly, their residence time at the administration site is extended, which can induce interstitial space enhancement, anatomical space distension, and/or lymph node enlargement (see Examples 1 and 5).

According to the present invention, nanoparticles engineered to have a hydrodynamic diameter of 2 to 20 nm and a surface charge of -20 mV to 0 mV, controlled through hydration of hydrophilic functional groups exposed on their surface, are selectively drained into terminal lymphatic vessels without entering blood capillaries at the administration site. These nanoparticles are characterized in that they are absorbed into the body via lymphatic drainage without accumulating at the administration site, and the absorbed nanostructures are subsequently excreted from the body.

For example, the nanoparticles of the present invention may be engineered such that, upon administration into an articular cavity or an intrathecal space, they are absorbed into the circulatory system via lymphatic drainage and subsequently excreted in urine through renal filtration.

The nanostructure INV-002 described in Example 4 is a particle having a hydrodynamic diameter of approximately 4 nm, formed by coordination of iron to a dextran-crosslinked core. The number average molecular weight of INV-002 is approximately 32 kDa, which is about half that of serum albumin, and its hydrodynamic diameter is approximately 4 nm, indicating that INV-002 meets the size criteria for venous drainage and is optimal for intra-articular injection.

An articulation refers to a joint between two or more bones, where the bones are in contact and function as a critical structure enabling bodily movement. Between the bones forming a joint lies a thin layer of hyaline cartilage referred to as articular cartilage. The joint also includes a synovial membrane, a pouch-like structure composed of synoviocytes that secrete a viscous fluid known as synovial fluid. The synovial fluid contains proteins, electrolytes, and hyaluronic acid, serving to nourish the joint surfaces and reduce friction. Synovial fluid also contains white blood cells and lymphocytes.

Blood vessels extend only up to the interface between the fibrous layer of the joint capsule and the synovial membrane, and therefore do not directly supply nutrients to the articular cartilage.

The joint cavity is filled with synovial fluid, a viscous liquid that reduces friction between the articular cartilages of synovial joints during movement. Synovial fluid is known to contain various molecules, including hyaluronic acid, proteins, and enzymes. Substances present in the synovial fluid can be drained and eliminated through lymphatic vessels or venous vessels connected to the systemic circulation.

Although the exact size limitations for drainage are not clearly defined, the synovium is generally known to be permeable to relatively small substances such as albumin (molecular weight: approximately 66.5 kDa) and IgG (molecular weight: approximately 150 kDa). Therefore, for uniform distribution, effective MR arthrography, and subsequent elimination of the contrast agent, smaller-sized contrast agents (less than approximately 7 nm) are preferred. The nanoparticles of the present invention may be engineered to satisfy these criteria simultaneously (see Example 4).

Intrathecal injection refers to a method of administering a drug through a needle into the spinal canal, including the vertebral canal and the subarachnoid space. The vertebral canal is formed by the continuous alignment of the vertebral foramina located in each vertebra. It houses the spinal cord, meninges, blood vessels, and parts of the peripheral nerves, and serves to protect these structures. Because intrathecal injection delivers the drug into the subarachnoid space, the administered drug reaches the cerebrospinal fluid (CSF) located between the arachnoid mater and the pia mater.

The nanoparticles of the present invention may also be engineered to allow uniform distribution within the vertebral canal and subarachnoid space upon intrathecal injection, enabling effective MR cisternography or MR myelography and subsequent excretion of the contrast agent (see Example 5 and FIG. 22).

**[Nanoparticles Exhibiting T1-MRI Contrast Effects]**

Magnetic resonance imaging (MRI) is a technique that visualizes differences in signal intensity acquired from the nuclear magnetic resonance phenomenon of hydrogen nuclei, which constitute the majority of biological tissues, based on tissue-specific variations in resonance behavior.

MRI measures the relaxation of hydrogen nuclear spins in water molecules, which is categorized into T1 and T2 relaxations.

The process in which nuclear spins, excited from longitudinal magnetization, return to their original state is referred to as T1 relaxation or longitudinal relaxation. Since T1 relaxation involves the transfer of energy from hydrogen nuclei to surrounding molecules (lattice), it is also called spin-lattice relaxation.

The substance with the longest T1 relaxation time is water, with a relaxation time of approximately 3 seconds. Although pure water is not typically present in the human body, non-mucoid fluids in the body contain molecules that move rapidly, resulting in slow energy transfer and long relaxation times. Fluids containing only electrolytes and lacking large molecules, such as cerebrospinal fluid (CSF) or urine, exhibit long T1 relaxation times of 2 to 3 seconds.

The relaxation time of a substance depends on the size of the molecules containing protons. Small molecules, such as water, move rapidly, which leads to low relaxation efficiency and thus longer relaxation times. Fatty acids present in adipose tissue have restricted motion, resulting in high T1 relaxation efficiency, and therefore exhibit the shortest T1 relaxation times among human tissues. Notably, T1 relaxation time increases as the strength of the magnetic field increases.

In MRI, contrast agents do not directly affect the signal themselves; rather, they exert an indirect effect by altering the local environment, thereby modifying the T1 and T2 relaxation times of surrounding tissues. Magnetic materials influence tissue relaxation times in MRI.

MRI contrast agents are categorized as either T1 or T2 contrast agents, and they serve to enhance either T1 or T2 signals. In MRI, T1 and T2 refer to the spin-lattice relaxation time and the spin-spin relaxation time, respectively, after nuclear spins are excited, and they result in distinct contrast enhancement effects.

The mechanism by which paramagnetic substances enhance contrast involves shortening the T1 and T2 relaxation times of hydrogen nuclei present in water molecules surrounding the paramagnetic substance in vivo, thereby increasing signal intensity and producing a contrast enhancement effect. This is caused by interactions between the unpaired electrons of the paramagnetic substance and the hydrogen nuclei of nearby water molecules.

In MRI, tissues that have a high proton density, a short longitudinal relaxation time (T1), and a long transverse relaxation time (T2) generate strong signals. T1 relaxation refers to the process in which hydrogen nuclei, having absorbed radio frequency (RF) pulses, release the absorbed energy and return to thermal equilibrium once the RF pulse is turned off. This energy release is most efficient when the Larmor frequency matches the molecular tumbling frequency of the surrounding tissue. Therefore, to shorten the T1 relaxation time using an MR contrast agent, the vibrational frequency of the agent must closely match the precessional frequency, the agent must have a high magnetic moment, and it must approach the hydrogen nuclei closely.

Because the molecular tumbling frequency of gadolinium ($Gd^{3+}$) matches the precessional frequency of protons, T1 relaxation is accelerated. Thus, the unpaired electrons of $Gd^{3+}$ interact with nearby protons in a resonant manner, facilitating rapid relaxation of the protons and thereby shortening the longitudinal relaxation time (T1), which results in an increased signal intensity.

When administration of a contrast agent shortens the T1 relaxation time of a tissue, the signal intensity of that tissue is enhanced. A contrast agent that produces such an effect is defined as a positive contrast agent.

T1 contrast agents are composed of paramagnetic substances capable of inducing spin-lattice relaxation and, in the presence of such agents, typically generate bright or positive contrast signals in comparison to water.

The nanoparticles of the present invention can be designed and synthesized as T1-MRI contrast agents having an $r_2/r_1$ ratio of less than 5 and an $r_1$ value of 1.5 or greater.

To evaluate whether the nanoparticles of the present invention possess contrast enhancement properties suitable for use as T1 MRI contrast agents, the spin-spin relaxivity coefficient ($r_2$) and spin-lattice relaxivity coefficient ($r_1$) are measured, and the ratio of $r_2$ to $r_1$ ($r_2/r_1$ ratio) is calculated. The $r_2/r_1$ ratio serves as a metric for determining whether the

nanoparticles are more appropriate for use as T1 MRI contrast agents or T2 MRI contrast agents. Conventional T1 MRI contrast agents exhibit an $r_2/r_1$ ratio of less than 5; if this value is 5 or greater, the agent is generally unsuitable for use as a T1 MRI contrast agent.

Through the use of the nanoparticles of the present invention, which are squeezable and precisely tunable in terms of swelling ratio, overall size, and/or surface charge, it is possible to visualize peripheral or central lymphatic vessels via T1-weighted MRI imaging for up to 1 hour following intradermal or subcutaneous administration.

For this purpose, the volume administered via intradermal or subcutaneous injection may range from 2 mL to 15 mL. Considering the effectiveness of imaging and the maximum administrable volume per injection site via intradermal or subcutaneous routes, the injection volume per administration point may be 2 mL or less.

The concentration of the magnetic nanomaterials is preferably in the range of 5 mM to 30 mM to ensure effective lymphatic imaging.

If an excessive dose of contrast agent is administered due to overly high concentration or injection volume, the contrast agent absorbed into the lymphatic system may migrate back into the venous circulation via the thoracic duct, potentially resulting in venous enhancement.

The nanoparticles of the present invention may be magnetic materials of nanometer size that exhibit T1-MRI contrast effects. Such magnetic materials exhibiting T1-MRI contrast effects at the nanoscale may be paramagnetic or superparamagnetic.

Iron oxide nanoparticles, when reduced to a few nanometers in size, undergo a magnetic property transition from superparamagnetism to paramagnetism, which results in enhanced T1 contrast effects relative to T2 contrast effects. Accordingly, they are emerging as biocompatible and novel T1 contrast agents. Furthermore, iron oxide has fewer toxicity-related concerns compared to other contrast agents, and ultimately degrades into bioavailable iron components in vivo, thereby offering a potential solution to the issues associated with gadolinium.

According to the present invention, the magnetic material of nanometer size that exhibits T1-MRI contrast effects may be, for example, manganese ferrite nanoparticles. Manganese ferrite nanoparticles may be synthesized at a low temperature of 100°C or below via a co-precipitation method in the presence of a polymer resin as a surface stabilizer, thereby yielding monodisperse nanoparticles in terms of composition and/or size. In addition, manganese ferrite nanoparticles synthesized via a low-temperature co-precipitation method in the presence of a surface stabilizer can be uniformly produced as particles having an average diameter of 1 to 3 nm. Furthermore, for the purpose of controlling the biodistribution and aggregation behavior of the manganese ferrite nanoparticles in physiological environments, the composition ratio between manganese and iron may be adjusted and/or various types of surface stabilizers may be employed.

In the present invention, the nanoparticles exhibiting MRI contrast effects may be (1) nanometer-sized nonmagnetic scaffolds on whose surface iron, manganese, or gadolinium is chemically bound, or (2) nanometer-sized oxides of iron, manganese, or gadolinium coated with water-soluble molecules such that they exhibit T1-MRI contrast effects. Non-limiting examples of such water-soluble molecules include surface stabilizers such as the polysaccharide-crosslinked colloidal particles described below (e.g., dextran-crosslinked nanoparticles (CDex)), citric acid, and polyacrylic acid (PAA).

The nonmagnetic scaffold may be a material that exposes hydrophilic groups on its surface, such as a polysaccharide, a protein (e.g., albumin, aprotinin, or lysozyme), or an inorganic material (e.g., silica or gold (Au)).

Non-limiting examples of polysaccharides include various polysaccharides known in the art that contain hydrophilic chemical functional groups, such as dextran, cellulose, starch, glycogen, chitosan, stachyose, sucrose, xylan, arabinan, hexosan, fructan, galactan, mannan, agaropectin, alginic acid, carrageenan, hemicellulose, hypromellose, chitin, agarose, dextrins, carboxymethyl cellulose, glycogen dextran, carbodextran, cyclodextrin, pullulan, or derivatives thereof.

To prepare the nonmagnetic scaffold in particulate form, various nanoparticle preparation methods known in the art may be employed.

For example, a method of preparing silica nanoparticles is disclosed in WO 2014/107055. Silica nanoparticles may be prepared by forming reverse micelles using an appropriate surfactant [e.g., a nonionic surfactant such as poly(oxyethylene) nonylphenyl ether], adding a silica precursor such as tetraethoxysilane, and allowing the reaction to proceed at room temperature.

An organic polymer-based nonmagnetic scaffold may be prepared by crosslinking the organic polymer through a reaction with a crosslinker having hydrophilic functional groups.

The crosslinker having hydrophilic functional groups includes not only those in which hydrophilic functional groups are inherently present in the crosslinker prior to the reaction, but also those in which hydrophilic functional groups are formed as a result of chemical structural changes in the crosslinker after the reaction.

Non-limiting examples of crosslinkers include epoxide crosslinkers, amine crosslinkers, crosslinkers having an anhydride group, polyisocyanate crosslinkers, and combinations thereof.

Non-limiting examples of the epoxide crosslinkers include 1-chloro-2,3-epoxypropane (epichlorohydrin), 1,4-cyclohexanedimethanol diglycidyl ether, 1,4-butanediol diglycidyl ether, bisphenol-F diglycidyl ether, isocyanuric acid tris-(2,3-epoxypropyl) ester, neopentyl glycol diglycidyl ether, triphenylolmethane triglycidyl ether, and bisphenol-A diglycidyl ether.

Non-limiting examples of the amine crosslinkers include ethylenediamine, 1,3-propanediamine, diethylenetriamine, triethylenetetramine, tetraethylenepentamine, aminoethylpiperazine, 4,7,10-trioxa-1,13-tridecanediamine, 2,2'-(ethylenedioxy)diethylamine, 1,3-bis(aminomethyl)cyclohexane, 1,3-bis(4-aminophenoxy)benzene, 4,4'-methylenebis(cyclohexylamine), and 5-amino-1,3,3-trimethylcyclohexanemethylamine.

Non-limiting examples of the crosslinkers having an anhydride group include 2,2-bis-[4-(phthalic anhydride-4-oxyphenyl)]propane, butanetetracarboxylic dianhydride, 4,4'-oxybis(phthalic anhydride), benzophenone-3,3',4,4'-tetracarboxylic dianhydride, and biphenyl-3,3',4,4'-tetracarboxylic dianhydride.

Non-limiting examples of the polyisocyanate crosslinkers include 1,3-bis(1-isocyanato-1-methylethyl)benzene, 1,3-bis(isocyanatomethyl)cyclohexane, hexamethylene diisocyanate, toluene-2,4-diisocyanate, trimethylhexamethylene diisocyanate, methylene bis(phenyl isocyanate), 4,4'-diisocyanatodicyclohexylmethane, and isophorone diisocyanate.

When the nonmagnetic scaffold is an inorganic material that lacks hydrophilic groups exposed on its surface, the surface may be modified with a water-soluble molecule containing hydrophilic groups.

The water-soluble molecule may serve as a surface stabilizer that binds to the nanoparticle through its hydrocarbon chain region and provides surface charge to the nanoparticle via its hydrophilic functional groups.

The surface stabilizer may comprise a hydrophilic functional group such as $-COOH$, $-NH_2$, $-SH$, $-CONH_2$, $-PO_3H$, $-OPO_4H_2$, $-SO_3H$, $-OSO_3H$, $-N_3$, $-NR_3OH$ ($R = C_nH_{2n+1}$, $0 \leq n \leq 16$), -OH, -SS-, $-NO_2$, -CHO, -COX (X = F, Cl, Br, I), -COOCO-, -CONH-, or -CN. As a result, nanoparticles whose surfaces are modified with the surface stabilizer may form ionic bonds, covalent bonds, hydrogen bonds, or metal-ligand coordination bonds in vivo.

Meanwhile, a method for preparing iron oxide nanoparticles is exemplified in Example 6.

**[Polysaccharide-crosslinked colloidal particles based on branched or cyclic polysaccharides]**

According to the present invention, nanoparticles having a hydrodynamic diameter of 2 to 20 nm and a surface charge ranging from -20 mV to 0 mV, the size and charge of which are adjusted by hydration of hydrophilic functional groups exposed on the surface thereof such that the nanoparticles are not absorbed into or drained into blood capillaries at the administration site but are selectively drained into terminal lymphatic vessels only, are:

(a) polysaccharide-crosslinked colloidal particles formed by intramolecular and/or intermolecular crosslinking, via crosslinkers at the -OH functional groups of the monosaccharide building blocks, of 1 to 3 branched polysaccharides or 2 to 30 cyclic polysaccharides, so as to resist hydrolysis by enzymes in vivo; or

(b) nanoparticles coated on their surface with the above-described polysaccharide-crosslinked colloidal particles.

Polysaccharides are a group of compounds in which multiple monosaccharides of identical or different structures are linked by enzyme-sensitive glycosidic bonds, and are widely found in the cell walls of animals, plants, and microorganisms.

These polysaccharides may carry neutral, positive, or negative charges, may have linear or branched molecular structures, and may vary in molecular weight from several hundred to several thousand Daltons. Due to these physicochemical properties, polysaccharides significantly influence the biodistribution of carrier drug molecules in vivo.

The polysaccharide-crosslinked colloidal particles of the present invention may be formed by crosslinking 1 to 3 branched polysaccharides or 2 to 30 cyclic polysaccharides using one or more crosslinkers in an aqueous solvent.

In this case, the -OH functional groups of the monosaccharide building blocks of the branched or cyclic polysaccharides are modified by a first crosslinker, and the resulting modified groups, when spatially adjacent, are crosslinked either directly or via a second crosslinker, intramolecularly and/or intermolecularly, to form the polysaccharide-crosslinked particles. When the first and/or second crosslinker is an amine-based crosslinker, amine groups derived from the crosslinker may be exposed on the surface of the resulting particles.

As the molecular weight of the branched or cyclic polysaccharides used in the synthesis increases, the hydrodynamic diameter of the resulting polysaccharide-crosslinked colloidal particles also increases. Therefore, the hydrodynamic size of the particles of the present invention can be controlled within the range of 2 to 20 nm.

The polysaccharide-crosslinked colloidal particles synthesized using various branched or cyclic polysaccharides, when administered in vivo (e.g., via intravenous injection or local administration), were confirmed to be excreted in the urine through renal filtration after entering the vasculature of the nephrons via systemic circulation. This excretion was validated as an MRI signal observed in the bladder (see Example 7).

The branched or cyclic polysaccharides to be crosslinked may be homopolysaccharides or heteropolysaccharides.

Non-limiting examples of branched or cyclic polysaccharides include, as illustrated in FIG. 23, dextran,

cyclodextrin, maltodextrin, and inulin.

With respect to dextran-crosslinked nanoparticle systems, the entire contents of Korean Patent Application No. 10-2021-0104405 are incorporated herein by reference.

Dextran is a polysaccharide derived from the condensation of glucose and is a complex branched glucan, as shown in the following structural formula. It is a microbially derived branched poly-α-D-glucoside predominantly composed of glycosidic bonds at the C-1 → C-6 positions.

The main chain of the polymer is composed of α(1→6)-linked glucose monomers, and the branching points are connected via α(1→3) glycosidic bonds.

Dextran was initially discovered as a microbial product in wine, but its large-scale production became feasible only after processes using bacterial cultures were developed. Dextran is currently synthesized from sucrose by specific lactic acid bacteria belonging to the genus Lactobacillus.

Dextran is approved by the U.S. Food and Drug Administration (FDA) as a biocompatible material.

As used herein, the term "dextran" includes various derivatives thereof. Non-limiting examples of dextran derivatives include carboxymethyl dextran (CM-dextran), dextran sulfate, and diethylaminoethyl dextran (DEAE-dextran).

There is an increasing industrial demand for dextran molecules of various specific molecular weights for diverse applications. For example, dextran with a molecular weight ranging from 70,000 to 100,000 Da is used as a plasma substitute. Dextran of approximately 40,000 Da is most likely used to improve blood circulation by reducing blood viscosity and inhibiting erythrocyte aggregation. Smaller dextran sulfates, such as those around 10,000 Da, are used as iron transport agents or anticoagulants.

Inulin is an energy-storage polysaccharide found in plants of the Compositae family and consists of a linear chain of fructosyl groups linked by β-2,1 glycosidic bonds and terminated by an α-D-1,2 glucopyranoside ring at the reducing end. Inulin is used as an excipient in pharmaceutical formulations under the GRAS regulatory classification, and its water-soluble form is utilized to test renal glomerular filtration. A specific semicrystalline particulate form known as delta inulin exhibits immunomodulatory properties and has been used as a vaccine adjuvant. Although inulin is not digestible by humans, it can be metabolized by bifidobacteria in the gut, providing not only a probiotic effect but also additional benefits such as colon-targeted drug delivery. Modifications of inulin for medical use typically involve periodate oxidation followed by functionalization via amination reactions or reactions with anhydrides.

According to the present invention, the polysaccharide-crosslinked colloidal particles formed by intramolecular and/or intermolecular crosslinking of 1 to 3 branched polysaccharides or 2 to 30 cyclic polysaccharides via hydroxyl groups of their monosaccharide building blocks using a crosslinker can be synthetically tuned to exhibit diverse physicochemical properties, including size, surface charge, and morphology.

The polysaccharide-crosslinked colloidal particles of the present invention are formed by intramolecular and/or intermolecular crosslinking of 1 to 3 complex branched polysaccharides or 2 to 30 cyclic polysaccharides in an aqueous medium via the hydroxyl groups of their monosaccharide building blocks using a crosslinker, thereby enabling various improvements over conventional polymer coatings widely used for nanomaterials and pharmaceutical agents.

According to the present invention, polysaccharide-crosslinked colloidal particles compactly formed via intramolecular and/or intermolecular crosslinking of complex branched or cyclic polysaccharides in an aqueous solution at the hydroxyl groups of their monosaccharide building blocks can be readily controlled to exhibit desired hydrodynamic size and swelling degree. As such, they allow predictable modulation of mobility within biological fluids and can be engineered to be squeezable for transport within various anatomical structures. For example, in the polysaccharide-crosslinked colloidal particles of the present invention, the degree of substitution of the crosslinker may be controlled to 10% to 40% of the available functional groups on the polysaccharide, and 2% to 98% of the crosslinker molecules may be configured such that one terminal group does not participate in crosslinking and remains exposed on the outer surface. Through such control, the swelling behavior and compressibility of the colloidal particles can be precisely tuned as desired.

Additionally, by adjusting at least one of the following parameters-namely, the molecular weight of the polysaccharide, the length of the polysaccharide backbone, the type of crosslinker used during crosslinking, the amount and rate of crosslinker administration during synthesis, and the type and degree of subsequent functional group modification-it is possible to finely control the overall size and surface charge of the resulting polysaccharide-crosslinked colloidal particles and/or the in vivo administrable complexes thereof, thereby imparting desired blood circulation time, biodistribution, and excretion pharmacokinetics.

For renal excretion, the average molecular weight of the dextran or dextran derivatives used may be 10,000 Da or less, and the molecular weight of spherical dextran-crosslinked nanoparticles formed by crosslinking dextran-based molecules through a crosslinker may be 35,000 Da or less.

The polysaccharide-crosslinked colloidal particles of the present invention are nanoparticles in which complex branched polysaccharides are intramolecularly and/or intermolecularly crosslinked at the -OH functional groups of their monosaccharide building blocks in an aqueous phase. The branched polysaccharides may form dimers or trimers through intermolecular crosslinking, or a single branched polysaccharide molecule may be intramolecularly crosslinked via a crosslinker to form the colloidal particle. The degree of crosslinking and/or the molecular weight of the branched

polysaccharide can be adjusted to control the compressibility, and preferably, the particles may be compact and spherical.

In particular, polymer solutions increase in viscosity with increasing concentration, and this also applies to aqueous solutions containing polysaccharide-crosslinked colloidal particles. According to the Mark-Houwink-Sakurada (MHS) equation, which relates the viscosity of a polymer solution to the molecular weight of the polymer, it is well known that viscosity is directly proportional to molecular weight. Excessive viscosity in injectable formulations may increase the risk of vascular occlusion or vascular damage due to high injection pressure. Therefore, for drug formulations utilizing the dextran-crosslinked nanoparticles of the present invention as multivalent linker systems or drug carriers, it is preferable to use dextran-based molecules with an average molecular weight of 10,000 Da or less, more preferably around 5,000 Da or less. When such low-molecular-weight dextran molecules are crosslinked to form spherical dextran-crosslinked nanoparticles with a molecular weight of approximately 15,000 Da or less, an injectable formulation with appropriate viscosity for in vivo administration can be achieved.

For example, when the average molecular weight of the branched or cyclic polysaccharides is adjusted to be relatively low, the resulting polysaccharide-crosslinked colloidal particles of the present invention can be used to prepare the in vivo administrable complex at high concentration. This allows precise control of the formulation's viscosity by adjusting the degree of dilution and enables a reduction in the injection volume relative to the desired drug dose.

For instance, when dextran-based molecules with an average molecular weight of 10,000 Da or less are crosslinked in an aqueous phase at the -OH groups of glucose building blocks, 1 to 3 dextran-based molecules are crosslinked intramolecularly and/or intermolecularly to form spherical nanoparticles. Accordingly, dextran-crosslinked nanoparticles prepared from dextran-based molecules with an average molecular weight of 10,000 Da or less may have a molecular weight of 35,000 Da or less. The in vivo administrable complex modified therewith may be engineered as a nanostructure having a hydrodynamic diameter of 10 nm or less, preferably 5 nm or less, for renal excretion. Generally, a nanostructure must have a hydrodynamic diameter of 6 to 8 nm or less in order to be naturally excreted through the kidney.

Therefore, by diversely designing the synthetic method of polysaccharide-crosslinked colloidal particles such as dextran-crosslinked nanoparticles, it is possible not only to regulate the biodistribution and excretion of target substances such as functional nanoparticles or drugs without causing immune rejection or toxicity, but also, in certain cases, to allow the conjugated polysaccharide-crosslinked colloidal particles of the present invention to be excreted in vivo together with the target substances, while remaining stable in plasma without aggregation, and without being metabolized or degraded in vivo after administration. Thus, they may be collected via urine or feces and reused. For example, the polysaccharide-crosslinked colloidal particles of the present invention may be engineered to be absorbed into the bloodstream after in vivo administration and excreted via the kidney without extravasation through the vascular wall.

In the present invention, the functional groups derived from the crosslinker and exposed on the surface of the polysaccharide-crosslinked colloidal particles may be the terminal functional groups of the crosslinker itself or modified/substituted versions thereof. For example, the crosslinker-derived functional groups exposed on the surface may include functional groups in which at least a portion of the terminal groups of the crosslinker are substituted with other functional groups.

In the present invention, the hydrophilic functional groups may be derived from: (i) functional groups of branched or cyclic polysaccharides that did not participate in the crosslinking reaction; (ii) terminal functional groups of crosslinkers that did not participate in crosslinking; and/or (iii) terminal groups of crosslinkers exposed after the reaction and subsequently modified.

Non-limiting examples of crosslinker-derived or hydrophilic functional groups exposed on the surface of the polysaccharide-crosslinked colloidal particles include amino groups, carboxyl groups, hydroxyl groups, and/or thiol groups. Reactive functional groups such as amine, thiol, carboxyl, and hydroxyl groups facilitate not only surface modification but also chemical conjugation with biopharmaceuticals and various types of small-molecule drugs, including ligands that specifically bind to cell receptors, antibodies or antibody fragments, antigenic peptides, and nucleic acids (DNA, RNA, or their fragments). In some cases, they may be cleaved in the acidic tumor microenvironment ($pH \leq 7$) or by hydrolytic enzymes, thereby releasing active drug compounds. Non-limiting examples of acid-labile linkages that can be cleaved under acidic conditions ($pH \leq 7$), such as those found in tumor tissues, include carbonate and ester bonds.

When the exposed functional group on the surface of the polysaccharide-crosslinked colloidal particles is positively charged (e.g., an amine group), cytotoxicity similar to that of other cationic polymers may arise. This issue can be mitigated by substituting some or all of the amine groups with carboxyl, methyl, or ethyl groups.

Non-limiting examples of hydrophilic functional groups capable of forming coordination bonds with metal ions (e.g., iron ions) include amine, thiol, carboxyl (carboxylate and carboxylic acid), and hydroxyl groups.

The terminal hydroxyl, amine, and non-coordinating carboxylate groups of the polysaccharide-crosslinked colloidal particles confer water solubility to the conjugates of the present invention. Accordingly, hydration of the hydrophilic groups exposed on the surface of the polysaccharide-crosslinked colloidal particles enhances the dispersion stability of the conjugates in body fluids. As a result, conjugates modified with the polysaccharide-crosslinked colloidal particles can remain stably dispersed in body fluids without precipitation or aggregation, even when a large amount of drug is conjugated, thereby retaining their intended function.

Furthermore, the present invention enables the surface charge of the polysaccharide-crosslinked colloidal particles and that of the modified target substance (e.g., a functional nanoparticle or drug) to be freely controlled by adjusting the type and/or degree of substitution of the functional groups derived from the crosslinker and exposed on the surface of the particles.

In addition, by precisely tuning the molecular weight, size, and surface charge of the polysaccharide-crosslinked colloidal particles, the overall size and charge of the particles or the resulting in vivo administrable complexes can be tailored to meet the desired pharmacokinetic profile, including blood circulation time, renal clearance, and hepatic clearance.

According to the present invention, the polysaccharide-crosslinked colloidal particles obtained via crosslinking reactions and, optionally, further modification of the terminal groups of the crosslinkers exposed after the reaction, can bind to targeting molecules and/or drugs through coordination bonding, covalent bonding, hydrogen bonding, and/or electrostatic interactions via the functional groups exposed on their surface. In particular, the ability of the crosslinker-derived functional groups on the surface of the polysaccharide-crosslinked colloidal particles to coordinate with metals facilitates the surface modification of various functional metal nanoparticles that are otherwise difficult to functionalize, thereby allowing the tuning of their physicochemical properties. For example, divalent or trivalent iron ions may coordinate with carboxylic acid or carboxylate groups derived from the crosslinker on the surface of the polysaccharide-crosslinked colloidal particles. Therefore, the polysaccharide-crosslinked colloidal particles of the present invention can be used to functionalize the surface of metal or metal oxide nanoparticles (e.g., iron oxide nanoparticles) via coordination or covalent bonding, thereby enabling their use as diagnostic agents such as contrast agents.

**[Method for Producing In Vivo Administrable Nanoparticles]**

According to the present invention, nanoparticles that are engineered to have a hydrodynamic diameter of 2 to 20 nm and a surface charge of -20 mV to 0 mV-such that they are not absorbed into or drained into blood capillaries at the administration site but are selectively drained only into terminal lymphatic vessels-may include polysaccharide-crosslinked colloidal particles formed by intramolecular and/or intermolecular crosslinking of 1 to 3 branched polysaccharides or 2 to 30 cyclic polysaccharides in an aqueous phase at the -OH functional groups of their monosaccharide building blocks using a crosslinker, wherein hydration of hydrophilic functional groups exposed on the surface enables modulation of the hydrodynamic diameter.

Meanwhile, the polysaccharide-crosslinked colloidal particles of the present invention may be provided, without limitation, by a method comprising the steps described below, and may be extended to include branched or cyclic polysaccharides in place of dextran or dextran derivatives in the dextran-crosslinked nanoparticles (CDex) (see Example 7).

Meanwhile, the dextran-crosslinked nanoparticles of the present invention may, without limitation, be produced by a method comprising the following steps:

Step 1 of preparing an aqueous solution of dextran or a dextran derivative;
Step 2 of dropwise adding an epoxide and an alkaline aqueous solution at room temperature;
Step 3 of dropwise adding divalent or higher-valent polyamines at room temperature to form dextran-crosslinked nanoparticles bearing terminal amine groups;
Step 4 of precipitating the resulting product;
Step 5 of redispersing the precipitated product in water;
optionally, Step 6 of recovering the dextran-crosslinked nanoparticles bearing terminal amine groups by dialysis;
Step 7 of adding an organic acid anhydride to the dextran-crosslinked nanoparticles bearing terminal amine groups, so as to substitute at least a portion or all of the terminal amine groups with carboxylic acid and/or carboxylate groups;
optionally, Step 8 of purifying a solution of dextran-crosslinked nanoparticles bearing carboxylic acid and/or carboxylate groups to obtain water-dispersible dextran-crosslinked nanoparticles;
optionally, Step 9 of adding an iron precursor (e.g., iron chloride) or an aqueous solution of iron oxide nanoparticles to the water-dispersible dextran-crosslinked nanoparticles prepared in the previous step, to obtain (i) dextran-crosslinked nanoparticles having a shell formed of divalent or trivalent iron ions, or (ii) complexes in which the surface of iron oxide nanoparticles is surface-modified with the dextran-crosslinked nanoparticles; and
optionally, Step 10 of purifying or concentrating the nanostructures obtained in the previous step by ultrafiltration.

To prevent unintended increases in hydrodynamic size due to swelling of dextran molecules, the present invention provides a method in which dextran monomers are crosslinked intra- and/or intermolecularly in an aqueous phase. It was discovered that, during the crosslinking of dextran by a crosslinker, two to three dextran molecules form a spherical core through intramolecular and/or intermolecular crosslinking. This spherical core may be a dextran-crosslinked nanoparticle formed by intra- and/or intermolecular crosslinking of the hydroxyl groups (-OH) of glucose building

blocks within the dextran molecules using a crosslinker.

The number of dextran molecules subjected to crosslinking in the dextran-crosslinked nanoparticles can be controlled to be the same or different, depending on the synthesis conditions and/or purification process.

For example, the present invention enables the production of dextran-crosslinked nanoparticles by reacting (i) an epoxide that modifies the hydroxyl groups of the glucose building blocks and (ii) a crosslinker with an aqueous solution of dextran or a dextran derivative at room temperature through Steps 2 and 3, without the need for expensive catalysts. In this case, crosslinking occurs via the hydroxyl groups of the glucose building blocks in dextran.

In Step 2, the epoxide used to modify the hydroxyl groups of the glucose building blocks is not particularly limited as long as it enables subsequent reaction with a crosslinker. Preferably, the epoxide is a halo alkyl oxirane, such as epichlorohydrin.

In Step 3, the polyamine may be replaced with any crosslinker capable of forming a covalent bond with the epoxide-derived functional groups that have modified the hydroxyl groups of the glucose building blocks. Such variants also fall within the scope of the present invention.

Steps 2 and 3 are economically advantageous because the crosslinking reaction at the hydroxyl groups of the glucose building blocks proceeds without requiring any additional expensive catalyst.

In Step 4, a large amount of an organic solvent having a dielectric constant between 15 and 50 may be added to induce precipitation of the product.

As described above, the dextran-crosslinked nanoparticles of the present invention are synthesized in an aqueous solution at room temperature without the use of surfactants and do not require a ligand exchange step. Such water compatibility is advantageous for biological applications.

Furthermore, the dextran-crosslinked nanoparticles obtained by the above-described manufacturing method may be in the form of a colloidal dispersion in water and can be adjusted to be isotonic depending on the degree of concentration, even without the use of additional excipients. For example, through Step 10, the resulting formulation may satisfy both non-pyrogenicity and sterility without any additional processing, thereby allowing the formulation to be used directly as an injectable pharmaceutical composition.

In Step 9, non-limiting examples of hydrophilic functional groups that can coordinate with iron ions include hydroxyl, carboxylic acid, carboxylate, and amine groups.

In addition, all disclosures of Korean Patent Application No. 10-2021-0104405 are hereby incorporated into the present invention and this specification in their entirety, particularly with respect to dextran-crosslinked nanoparticles having a shell composed of divalent or trivalent iron ions.

The polysaccharide-crosslinked colloidal particles having a shell composed of divalent or trivalent iron ions, as engineered through Step 9, may be designed and synthesized to function as T1 MRI contrast agents that generate bright signals in MRI imaging. Accordingly, after administration in vivo, the position of the conjugate surface-modified with the polysaccharide-crosslinked colloidal particles such as dextran-crosslinked nanoparticles may be tracked by MRI imaging.

The polysaccharide-crosslinked colloidal particles of the present invention may be engineered and synthesized to prolong the retention of contrast enhancement during MRI, thereby enabling longer scan times and enhancing the spatial resolution of MRI. This feature allows high-resolution imaging of the entire vascular system in vivo. Accordingly, the polysaccharide-crosslinked colloidal particles of the present invention are capable of visualizing microvasculature that is clinically significant but difficult to observe using conventional contrast agents. For example, when imaging the rat brain using dextran-crosslinked nanoparticles at a spatial resolution of 0.078 mm $\times$ 0.078 mm $\times$ 0.078 mm, microvasculature with a thickness of approximately 0.078 mm could be clearly visualized. Given that the spatial resolution of the most commonly used 3 Tesla (3T) MRI scanners in clinical settings is approximately 1 mm, the resolution achieved using the dextran-crosslinked nanoparticles of the present invention represents an approximately 13-fold improvement.

In summary, the polysaccharide-crosslinked colloidal particles of the present invention may serve as T1 or T2 MRI contrast agents when divalent or trivalent iron ions are coordinated to the crosslinker-derived functional groups exposed on the particle surface.

In addition, iron oxide nanoparticles engineered to function as T1 or T2 MRI contrast agents may be surface-modified with dextran-crosslinked nanoparticles in Step 9. This modification renders the nanoparticles squeezable, enabling them to move within in vivo structures without aggregation upon in vivo administration, thereby maintaining their functionality as T1 or T2 MRI contrast agents.

Therefore, the conjugates surface-modified with the polysaccharide-crosslinked colloidal particles according to the present invention can serve as MRI contrast agents. After administration in vivo, their location can be monitored by MRI imaging to assess: phagocytosis by macrophages, metabolic degradation, circulation in the bloodstream, delivery to parenchymal cells via blood capillaries, tissue accumulation, renal excretion into urine, excretion into feces, absorption into the vascular system, leakage through vessel walls, potential for recovery and reuse via excretion, and intracellular uptake.

**[Absorption, Distribution, and Excretion Profile of Nanoparticles in the Human Body]**

According to the present invention, nanoparticles engineered to have a hydrodynamic diameter of 2 to 20 nm and a surface charge of -20 mV to 0 mV by hydrating hydrophilic functional groups exposed on their surface-thereby preventing penetration into or drainage into blood capillaries at the administration site and enabling selective drainage only into terminal lymphatic vessels-function as a type of drug with tunable physicochemical properties (e.g., size, surface charge, morphology) and controllable synthesis parameters.

Accordingly, the pharmacokinetics-i.e., the continuously changing concentration of the drug in blood and tissues over time as it is absorbed, distributed, metabolized, and excreted from the body-of such nanoparticles, including their biodistribution and excretion pattern in the human body, is primarily governed by the precisely controlled physicochemical properties of the nanoparticles.

After being drained into lymphatic vessels, the nanoparticles of the present invention may enter the vascular circulation and (i) be absorbed by hepatocytes, subsequently secreted into the bile duct and excreted via feces, and/or (ii) be excreted into the bladder via urine.

Furthermore, the physiological processes by which organs and tissues in the body respond to foreign materials such as nanoparticles-namely, absorption in the gastrointestinal tract, distribution to peripheral tissues via blood flow driven by cardiac output, and elimination via hepatic or renal clearance-result in changes in nanoparticle concentrations that rise and fall within the bloodstream and various tissues over time.

Such circulation and biodistribution of nanoparticles are not only dependent on the diverse physicochemical properties of the nanoparticles, but may also be affected by aggregation of the nanoparticles in vivo.

According to the present invention, nanoparticles having a hydrodynamic diameter adjusted to 2 to 20 nm and a surface charge adjusted to -20 mV to 0 mV through hydration of hydrophilic functional groups exposed on their surfaces are engineered such that they are not absorbed into or drained into blood capillaries at the administration site, but are selectively drained into terminal lymphatic vessels. These nanoparticles can reside at the administration site for two hours or more and are preferably excreted via urine through the kidneys.

As demonstrated in Example 4, when NEMO-103 was used as an MR contrast agent to perform magnetic resonance arthrography (MRA), it was confirmed that the agent produced a strong T1 contrast effect along the joint boundaries with joint expansion, which persisted for up to 120 minutes, and was completely drained into surrounding terminal lymphatic vessels within 24 hours.

Non-limiting examples of physical properties of nanoparticles engineered for in vivo administration include particle size, surface charge, magnetic hysteresis profile, and the $r_2/r_1$ ratio.

Non-limiting examples of chemical properties of such nanoparticles include the type and distribution of surface functional groups.

The size of the nanoparticles of the present invention is critical, as it significantly influences cellular uptake and circulation within the bloodstream.

The particle size affects both the degree and kinetics of accumulation.

The presence of surface charge affects opsonization, recognition by cells of the mononuclear phagocyte system (MPS), and circulation in the bloodstream. The surface charge of nanoparticles is determined by the materials, composition, and surface stabilization strategies employed.

Accordingly, the nanoparticles of the present invention are engineered to have a surface charge in the range of -20 mV to 0 mV, such that opsonization is inhibited and aggregation in vivo is prevented and/or phagocytic uptake by macrophages is selectively enabled or suppressed.

For example, positively charged nanoparticles tend to exhibit increased cellular binding and uptake due to electrostatic interactions with negatively charged cell membranes.

The hydrophobicity of the final nanoparticles also affects their interactions with cells. Meanwhile, the conjugation of hydrophilic polymers such as polyethylene glycol (PEG) may delay the adsorption of serum proteins and opsonins.

As described above, nanoparticles may be engineered to control their in vivo distribution (1) the vascular system, and their excretion via either (2) the liver or (3) renal elimination into the bladder, depending on their size, surface charge, and surface functionalization. For example, nanoparticles smaller than approximately 6-8 nm may be excreted through the kidneys, whereas larger nanoparticles are primarily sequestered in the liver. When surface charges are present, serum proteins may adsorb onto the nanoparticle surface, forming a protein corona, which increases phagocytic uptake by macrophages, including Kupffer cells. Conversely, the closer the surface charge of nanoparticles is to neutrality, the more the formation of the protein corona is suppressed, leading to extended circulation time in the bloodstream.

The nanoparticles of the present invention are capable of finely controlling their surface properties through the type and/or density of functional groups exposed on the surface.

Meanwhile, the surface modification efficiency of nanoparticles through the hydration of hydrophilic functional groups exposed on the surface can be estimated by measuring the chemical quantification of such groups, the increase in surface charge, or the enhancement of surface hydrophilicity. One commonly used method to evaluate surface

modification is to measure the zeta potential of an aqueous dispersion containing the nanoparticles. Zeta potential reflects the electrostatic potential at the slipping plane of the particles and is influenced by both the particle composition and the dispersion medium. The primary purpose of measuring zeta potential is to predict colloidal stability. Interparticle interactions play a crucial role in colloidal stability, and zeta potential measurements help quantify these interactions. Zeta potential serves as an index of electrostatic repulsion or attraction between particles depending on their surface charge; since most aqueous colloidal systems are stabilized by electrostatic repulsion, a higher magnitude of repulsion reduces the likelihood of particle aggregation. Generally, nanoparticles having a zeta potential greater than ±10 mV are considered stable in aqueous media, as the surface charge can prevent aggregation. However, nanoparticles bearing high surface charges may become unstable in solutions with high ionic strength, such as physiological fluids, due to electrolyte-mediated aggregation. When both positively charged (e.g., amine) and negatively charged (e.g., carboxylate) functional groups are present on the surface, resulting in an overall near-neutral zeta potential (i.e., forming a zwitterionic surface), such nanoparticles have been reported to exhibit improved stability both in aqueous solution and under in vivo conditions such as within the bloodstream.

In addition, when administered in vivo, nanoparticles interact with biomolecules such as proteins via electrostatic interactions or non-specific binding, primarily governed by their surface charge. When proteins become adsorbed onto the nanoparticle surface through such interactions, the likelihood of recognition by immune cells is increased. Therefore, it is necessary to control the surface charge of the nanoparticles to a desired range, for example, from -20 mV to 0 mV.

As described above, the polysaccharide-crosslinked colloidal particles of the present invention, such as dextran-crosslinked nanoparticles, can precisely regulate their surface charge through the type and/or density of terminal functional groups derived from the crosslinker that are exposed on the surface (see FIG. 3).

For example, a combination of positively charged amines and negatively charged carboxylates can generate a zwitterionic surface with an overall charge close to neutral. Due to the terminal functional groups derived from the crosslinker, the surface charge of the dextran-crosslinked nanoparticles may be in the range of -30 mV to +10 mV, preferably -20 mV to 0 mV, within a range that is non-toxic and maintains stability in physiological fluids.

Accordingly, the nanoparticles of the present invention can be engineered to provide desired in vivo pharmacokinetic properties to the in vivo administrable complex of the present invention, by precisely controlling their hydrodynamic diameter and/or surface charge. In particular, by adjusting the surface charge to a range of -20 mV to 0 mV through the hydration of hydrophilic functional groups exposed on the nanoparticle surface, aggregation-induced lymph node congestion can be prevented.

In addition to influencing colloidal stability in aqueous media, the surface charge of nanoparticles also affects their interactions with proteins in vivo. Generally, nanoparticles larger than 30 nm are readily recognized by the immune system and are subsequently cleared via phagocytosis by macrophages. Even if the nanoparticles are small in size, excessive positive or negative surface charge can result in binding with blood components, primarily proteins, leading to an increase in hydrodynamic size and subsequent phagocytic clearance.

The surface charge of the nanoparticles according to the present invention plays a significant role in determining their pharmacokinetics and in vivo behavior. For example, when serum proteins bind non-specifically to the surface of nanoparticles through electrostatic interactions-i.e., via opsonization-the resulting nanoparticle-protein complexes are more likely to be recognized by the mononuclear phagocyte system (MPS), leading to enhanced phagocytic uptake and accumulation in organs. Even if the original nanoparticles are within the renal filtration size threshold, complexation with proteins increases their size, making renal excretion unfeasible. Therefore, to avoid unintended organ accumulation and enable renal clearance, it is essential to impart anti-opsonization characteristics to the nanoparticles.

Accordingly, the nanoparticles of the present invention may be engineered to control blood circulation time and renal excretion capability by adjusting the surface charge to a range of -30 mV to +10 mV, preferably -20 mV to 0 mV, thereby minimizing non-specific adsorption of serum proteins.

The nanoparticles of the present invention may be engineered to induce or evade opsonization by modulating their surface charge to be positive, negative, or neutral (zwitterionic) through hydrophilic functional groups exposed on their surfaces.

The nanoparticles of the present invention may be designed to prevent binding with plasma proteins such as albumin by adjusting their surface charge to a range of -30 mV to +10 mV, and preferably from -20 mV to 0 mV.

When nanoparticles are exposed to actual in vivo environments-such as blood flow, interstitial fluid, or the extracellular matrix-proteins present in these environments form a "protein corona" on the surface of the nanoparticles. Through this corona effect, proteins non-specifically adsorb onto the nanoparticle surface (commonly referred to as opsonization), which in turn determines the effective size, colloidal stability, surface properties, cellular uptake, biodistribution, intracellular distribution, pharmacokinetics, tissue distribution, and potential toxicity of the nanoparticles.

Accordingly, the nanoparticles of the present invention may be surface-modified with water-soluble ligands or coated with amphiphilic polymers to exhibit bright T1 signals in MRI and to be partially excreted through the kidneys.

Because the nanoparticles of the present invention are designed to (i) evade phagocytosis by macrophages and (ii) function as T1 MRI contrast agents that produce bright signals in MRI images (as demonstrated in Examples 1 through

7), they enable in vivo tracking via MRI after administration. Specifically, the fate of the nanoparticles can be monitored to determine whether they (i) are phagocytosed by macrophages, (ii) are metabolically degraded, (iii) circulate in the bloodstream, (iv) circulate through the lymphatic system, (v) are delivered to parenchymal cells through blood capillaries, (vi) accumulate in tissues, (vii) are excreted via the kidneys into urine, (viii) are absorbed into the vascular system after in vivo administration, (ix) extravasate through blood vessel walls, or (x) can be recovered from urine for reuse.

The nanoparticles of the present invention may be designed to be administered into nonvascular tissues, such as the joint cavity or spinal cavity, and subsequently drained via lymphatic vessels, absorbed into the vascular circulation without extravasation through blood vessel walls, and finally excreted in urine through renal filtration (as demonstrated in Examples 1 through 7).

The nanoparticles of the present invention are designed to be completely cleared from the body without accumulation or adverse effects at the administration site, including blood vessels, the joint cavity, and the spinal cavity, and to be safely eliminated via renal excretion. Therefore, the present invention demonstrates successful pharmacokinetic elimination via the kidneys even when the nanoparticles are administered to nonvascular tissues or body cavities (e.g., joint cavity, spinal cavity), thereby expanding the potential for in vivo applications of the nanoparticles.

The nanoparticles of the present invention may be engineered such that, after being drained via the lymphatic vessels at the administration site, they are not eliminated by lymphocytes in the lymph nodes during the drainage of lymph through the lymph nodes, and thereby can be absorbed into the bloodstream without causing acute inflammation or swelling at the administration site and/or in the lymph nodes.

Additionally, the nanoparticles of the present invention may be designed to be excreted in an intact state in the urine without undergoing metabolic degradation along the absorption, distribution, and/or excretion pathways following in vivo administration, thereby allowing potential reuse or recycling.

**[Hydrophilic Functional Groups Exposed on the Surface and Hydrodynamic Diameter of Nanoparticles]**

The nanoparticles of the present invention can be precisely controlled in terms of hydrodynamic diameter and surface properties through the type and/or density of hydrophilic functional groups exposed on their surface.

Surprisingly, in lymphangiography, it was discovered that contrast agents designed in accordance with the present invention are absorbed exclusively into lymphatic vessels without penetrating blood capillaries, even when their hydrodynamic diameter ranges from 2 nm to 4 nm. That is, by adjusting the hydrodynamic diameter of the contrast agent to be greater than at least 2 nm, it is possible to prevent diffusion into interstitial tissue and subsequent entry into veins, thereby overcoming the drawback of simultaneous visualization of both lymphatic and venous vessels.

Accordingly, the nanoparticles of the present invention may be engineered to have a hydrodynamic diameter in the range of 2 to 20 nm, preferably 10 nm or less, more preferably 8 nm or less, and even more preferably 6 nm or less, depending on physicochemical parameters such as size, surface charge, and shape. Such control enables the provision of a finely tuned formulation modality that, upon administration to perivascular tissues, is selectively drained into lymphatic vessels without unintended venous uptake.

In addition, the nanoparticles of the present invention may be engineered to be squeezable through hydration of hydrophilic functional groups exposed on their surface, such that their hydrodynamic diameter can be regulated to ensure selective drainage into terminal lymphatic vessels without entering blood capillaries at the administration site and without causing lymph node congestion.

Furthermore, the nanoparticles of the present invention may be designed to be completely excreted from the body without residual retention at the administration site within one week, preferably within 72 hours, and more preferably within 24 hours.

The nanoparticles of the present invention may also be engineered to be excreted from the body via hepatic or renal elimination pathways.

The non-vascular administration route of the formulation of the present invention may be direct injection via intradermal, subcutaneous, or intralymphatic routes.

Accordingly, when the nanoparticles of the present invention are administered intradermally or subcutaneously, the size of the injection needle may typically range from 21 to 31 gauge.

For effective lymphatic vessel imaging, the following conditions should be satisfied: (1) the nanoparticles must selectively penetrate into terminal lymphatic vessels at the administration site without entering blood capillaries; (2) to allow efficient drainage, the nanoparticles that reach the lymph nodes must not be sequestered or accumulated by macrophages residing in the lymph nodes; and (3) the nanoparticles that enter the venous circulation via the thoracic duct must not extravasate back out of the veins. Accordingly, the nanoparticles of the present invention are preferably engineered to have a hydrodynamic diameter ranging from 2 to 20 nm, depending on their physicochemical properties such as size, surface charge, and shape, so as to satisfy the above conditions.

In addition, nanoparticles with a hydrodynamic diameter adjusted in accordance with the present invention may improve tolerability without eliciting allergic reactions.

The hydrodynamic diameter of a nanoparticle, also referred to as the "hydrated diameter," denotes the size of the particle in solution, which is influenced by factors such as particle shape, surface charge, and interactions with other particles and the surrounding solvent. The hydrodynamic diameter may be measured using various techniques such as dynamic light scattering (DLS) or nanoparticle tracking analysis (NTA). This diameter is a critical parameter that governs nanoparticle behavior in biological systems, including in vivo mobility and biodistribution, interactions with cells and tissues, and clearance from the body. Furthermore, the hydrodynamic diameter may affect the pharmacokinetics and pharmacodynamics of drugs encapsulated within the nanoparticles. Therefore, precise control and measurement of the hydrodynamic diameter is crucial for drug development and delivery applications.

Depending on their size, nanoparticles are taken up by different organs, thereby determining their biodistribution in the body. Nanoparticles with a diameter of 50 nm or greater are rapidly sequestered by Kupffer cells in the liver and tend to accumulate in the hepatic tissue.

However, even small nanoparticles may be unintentionally cleared by the reticuloendothelial system (RES) if they lack sufficient colloidal stability, as poorly dispersed nanoparticles tend to aggregate readily.

For example, nanoparticles having a hydrodynamic diameter in the range of 1 to 30 nm may circulate in the bloodstream for an extended period without undergoing phagocytic uptake by phagocytes. Furthermore, in order to allow for natural excretion via the kidneys, the nanoparticle size typically needs to be 6 to 8 nm or smaller. If the diameter exceeds this range, renal excretion from the body becomes difficult. Accordingly, when the nanoparticles of the present invention are intended to be excreted via urine through the kidneys, they may be engineered to have a total hydrodynamic diameter of 8 nm or less, preferably 6 nm or less, with a uniform size distribution.

Conversely, when urinary excretion is suppressed, the circulation time of the nanoparticles in the bloodstream may be prolonged, thereby maintaining a high plasma drug concentration for an extended duration and improving the delivery efficiency to the reticuloendothelial system (RES). This may enhance drug delivery to organs with a well-developed RES, such as the liver and spleen. In such cases, the nanoparticles of the present invention may be engineered to have a total hydrodynamic diameter of 8 nm or greater with a uniform size distribution, in order to prolong systemic circulation or enhance RES-targeted delivery.

**[Selective Exclusion from Blood Capillaries]**

According to the present invention, nanoparticles that are selectively drained into terminal lymphatic vessels without entering blood capillaries at the administration site, and that do not cause lymph node congestion, may be engineered to exhibit exclusion from blood capillaries through hydration of hydrophilic functional groups exposed on their surfaces. Such nanoparticles may be designed to be squeezable and to have a hydrodynamic diameter ranging from 2 to 20 nm, preferably 10 nm or less, more preferably 8 nm or less, and most preferably 6 nm or less, with a surface charge controlled to be in the range of -20 mV to 0 mV. The hydration of surface-exposed hydrophilic functional groups contributes to the capillary-impermeable characteristics of the nanoparticles.

Vascular capillary structures vary significantly in terms of the exposure of the basement membrane through slit-like gaps between endothelial cells. In organs such as the liver and spleen, most of the basement membrane is exposed through large discontinuous fenestrations, allowing the passage of large plasma proteins. In contrast, the capillary structure of the brain is continuous, with no slit junctions. Closely aligned endothelial cells form tight junctions, constituting the blood-brain barrier (BBB). For a drug to enter the brain, it must traverse or be actively transported across the capillary endothelium of the central nervous system (CNS).

The intercellular gaps (fenestrations) between cells constituting the vascular wall are generally known to be approximately 2 nm in size. Therefore, materials smaller than 2 nm can extravasate through capillary walls. By adjusting the size of a material, its vascular permeability can be controlled. The nanoparticles of the present invention may thus be engineered to be capillary-impermeable, such that after being discharged from the administration site into lymphatic vessels and subsequently absorbed into the vascular circulation, they circulate within the bloodstream without leaking through the walls of blood capillaries or diffusing into interstitial fluids.

The chemical properties of a drug strongly influence its ability to traverse cellular membranes. Lipophilic drugs can diffuse through lipid bilayers and thus are capable of passing across all cell surfaces. In contrast, hydrophilic drugs do not readily penetrate cell membranes and must pass through slit junctions between cells.

In one embodiment of the present invention, the nanoparticles are engineered such that a hydrophilic functional group-either a terminal group of the crosslinker or a group derived from the crosslinker-is exposed on the surface of the polysaccharide-crosslinked colloidal particles and oriented toward the surrounding aqueous environment. As a result, the nanoparticles behave as hydrophilic drugs and are designed to be impermeable to cellular membranes and, more specifically, incapable of penetrating the slit junctions between endothelial cells in blood capillaries-that is, the nanoparticles are engineered to exhibit vascular capillary impermeability.

For example, the polysaccharide-crosslinked colloidal particles of the present invention may be engineered to be filtered through the kidneys but not to permeate the vascular walls of normal capillaries, thereby being selectively drained

only into lymphatic vessels.

The normal capillaries that the polysaccharide-crosslinked colloidal particles of the present invention cannot penetrate are those possessing continuous endothelium with tight junctions and a basal lamina. Accordingly, although the particles are designed to be filtered in the kidneys, they may also be filtered through the capillaries of the liver and spleen.

The polysaccharide-crosslinked colloidal particles of the present invention may be designed to be filtered in the kidneys but not to permeate the vascular walls of normal capillaries, and to be selectively drained into lymphatic vessels, by engineering the hydrodynamic diameter to be within the range of 2 to 10 nm, preferably 8 nm or less, more preferably 6 nm or less, and by exposing carboxyl (-COOH) functional groups on the surface so that the surface charge is within the range of -20 mV to 0 mV.

Upon local administration, the polysaccharide-crosslinked colloidal particles of the present invention may be retained at the administration site for an extended period of time, thereby inducing interstitial space enhancement, anatomical space distension, and/or lymph node enlargement. Furthermore, prolonged retention at the target administration site may enhance drug efficacy (see Examples 1 to 5).

The polysaccharide-crosslinked colloidal particles of the present invention may be engineered to have a hydrodynamic diameter of 10 nm or less, preferably 8 nm or less, and more preferably 6 nm or less, and a surface charge in the range of -20 mV to 0 mV, such that they are filtered through the kidneys and excreted via urine following vascular circulation. As a result, the excretion time may be 48 hours or less, preferably 24 hours or less, with an excretion rate of 90% or greater.

The polysaccharide-crosslinked colloidal particles of the present invention are designed to be readily excreted in urine, thereby avoiding hepatic metabolism and exhibiting no hepatotoxicity.

For example, when the hydrodynamic diameter of the polysaccharide-crosslinked colloidal particles is in the range of 2 nm to 10 nm, preferably 8 nm or less, and more preferably 6 nm or less, and the surface charge is controlled to be within the range of -20 mV to 0 mV, the particles can be filtered through the kidneys but are unable to permeate the vascular walls of normal capillaries, thereby being selectively drained into lymphatic vessels. Accordingly, upon local administration, the ease of urinary excretion may reduce adverse effects associated with prolonged systemic retention or long-term accumulation (see Examples 5 and 7).

For example, by controlling at least one of the following: the molecular weight of the polysaccharide such as dextran or a dextran derivative (e.g., carboxymethyl dextran), the chain length of the polysaccharide backbone, the type of crosslinker used during crosslinking, the amount and rate of crosslinker addition during synthesis, additional surface modification with functional groups, the molecular weight of the resulting polysaccharide-crosslinked colloidal particles, and the surface charge, the final size and charge of the polysaccharide-crosslinked colloidal particles may be finely tuned. As such, the particles may be engineered either to avoid phagocytosis by macrophages or to be actively taken up by macrophages, and to be excreted via urine or feces through renal or hepatic clearance mechanisms. In addition, desired blood circulation time, biodistribution, and pharmacokinetic profiles may be imparted to the particles.

**[Nanoparticles Selectively Drained into Terminal Lymphatic Vessels Without Entering Blood Capillaries]**

The nanoparticles of the present invention may be engineered such that their hydrophilic functional groups exposed on the surface are hydrated, thereby adjusting their hydrodynamic diameter to 2 to 20 nm and their surface charge to the range of -20 mV to 0 mV. This enables the nanoparticles to be selectively drained into terminal lymphatic vessels without entering blood capillaries at the administration site.

The lymphatic system is an essential component of the immune system and includes organs such as the thymus, bone marrow, spleen, tonsils, appendix, and Peyer's patches in the small intestine, which generate and process specialized white blood cells that fight infections and cancer. The lymphatic system comprises (i) lymphatic vessels with thin walls, (ii) lymph nodes, and (iii) two collecting ducts.

Lymphatic vessels are distributed throughout the body and are larger than blood capillaries (the smallest blood vessels connecting arterioles and venules) but smaller than the smallest veins. Most lymphatic vessels contain valves similar to those of veins, which allow lymph-a coagulating fluid-to flow continuously in one direction toward the heart. Lymphatic vessels drain fluid (lymph) from tissues throughout the body and transport it to lymph nodes before returning it to the venous circulation via the two collecting ducts.

Lymph originates from interstitial fluid that diffuses through the extremely thin walls of blood capillaries into the intercellular space. Most of this fluid is reabsorbed into blood capillaries, while the remaining portion enters the lymphatic vessels and is ultimately returned to the venous system. Lymph also contains (i) proteins, electrolytes, nutrients, and other substances that the fluid delivers to tissues, and (ii) various other components including damaged cells, cancer cells, and foreign materials such as bacteria and viruses that have entered the interstitial fluid.

Lymph nodes are small, bean-shaped organs that function as collection centers for lymph. All lymph passes through strategically located lymph nodes, where damaged cells, cancer cells, and foreign substances are filtered. In addition, lymph nodes contain white blood cells, such as lymphocytes and macrophages, that are specialized to

phagocytose and destroy damaged cells, cancer cells, infectious microorganisms, and foreign materials. Accordingly, one of the primary functions of the lymphatic system is to eliminate damaged cells from the body and prevent the spread of infections and cancer. Some lymph nodes are clustered beneath the skin, particularly in the neck, axillae, and groin, while others are located deeper within the body, such as in the abdominal cavity.

Lymph nodes drain into collecting ducts, which in turn discharge their contents into the two subclavian veins located beneath the clavicles. These veins converge to form the superior vena cava, a major vein that returns blood from the upper body to the heart.

The nanoparticles of the present invention may be engineered to be injected into distal regions of the body such that they are absorbed into lymphatic vessels or directly administered into the lymphatic vessels. In such cases, whether administered at distal sites or directly into lymphatic vessels, the nanoparticles can flow through the lymphatic system and be absorbed into the vascular circulation without being eliminated by lymphocytes or macrophages in the lymph nodes.

Furthermore, when the nanoparticles of the present invention function as MRI contrast agents, absorption or injection into the lymphatic vessels allows MRI imaging of abnormalities in lymphatic flow pathways, such as lymphatic obstruction, lymphedema, swollen lymph nodes, lymphadenitis, lymphoma, or metastasis of tumors to nearby lymph nodes.

**[Contrast Agents for Medical Imaging Techniques Used to Visualize the Lymphatic System and Its Function]**

The non-intravenous injectable formulation of the present invention may provide contrast agents for medical imaging techniques used to visualize the lymphatic system and its associated functions.

In this context, the nanoparticles contained in the non-intravenous injectable formulation of the present invention may serve as a contrast agent for effective imaging of disease diagnosis and biological phenomena at the molecular and cellular levels.

The nanoparticles of the present invention can also be drained into lymphatic vessels having a diameter of 0.1 mm or greater.

When nanoparticles with tunable physicochemical properties-such as size, surface charge, and morphology-are used as contrast agents, it becomes possible to predict various in vivo interactions across molecular, cellular, and organ levels based on the acquired imaging data. It also enables the prediction of chemical or physical functions of biological entities, organs, cells, and biochemical molecules present within the body.

Moreover, the nanoparticles of the present invention are engineered such that they are not discharged into blood capillaries at the administration site but are instead selectively drained into terminal lymphatic vessels. These nanoparticles possess hydrophilic functional groups exposed on their surfaces, which enable hydration-mediated compressibility and control over hydrodynamic diameter, thereby minimizing lymph node congestion. Consequently, such nanoparticles can be utilized to assess lymphatic flow, drainage, and congestion.

According to the present invention, when nanoparticles engineered to be compressible through hydration of hydrophilic functional groups exposed on their surfaces, and having a controlled hydrodynamic diameter, are used as contrast agents-such that they are not discharged into blood capillaries at the administration site but are selectively drained only into terminal lymphatic vessels without inducing lymph node congestion-it becomes possible to selectively visualize peripheral and/or central lymphatic vessels without venous contamination when administered into perivascular tissue regions rather than via intravenous injection.

Peripheral lymphatic vessels and central lymphatic vessels are two types of lymphatic vessels that play essential roles in the lymphatic system.

Peripheral lymphatic vessels are located throughout the body's tissues and function to collect excess interstitial fluid and debris from the tissues and transport them to the central lymphatic vessels. These vessels have small diameters and semi-permeable thin walls, allowing fluids and debris to enter but preventing their exit. As they progress toward the central lymphatic vessels, they merge to form larger lymphatic vessels

In contrast, central lymphatic vessels are located near the heart and are larger lymphatic vessels responsible for transporting lymph from the peripheral vessels into the bloodstream. These vessels have thicker walls and operate under higher pressure, facilitating the pumping of lymph through the lymphatic network. Central lymphatic vessels also contain specialized valves that prevent backflow of lymph and help regulate its flow through the lymphatic system.

In one aspect of the present invention, the nanoparticles may be magnetic nanoparticles, in which case they can be used as diagnostic agents for magnetic resonance imaging (MRI).

MRI imaging technology offers advantages such as excellent soft tissue contrast and the absence of radiation-related risks.

Medical imaging techniques that utilize contrast agents to visualize the lymphatic system and its functions include magnetic resonance lymphangiography (MRL), ultrasound lymphangiography, computed tomography (CT) lymphangiography, positron emission tomography (PET) lymphangiography, and lymphoscintigraphy.

The selection of the imaging modality depends on various factors, including the nature of the condition being assessed, the overall health status of the patient, and the availability of equipment and clinical expertise.

[Formulation]

The term "drug formulation" refers to the process of combining various chemical and physical components to produce a final drug or pharmaceutical product. Drug formulation is a critical aspect of drug development, as it determines the stability, bioavailability, and efficacy of the drug. The formulation process involves the selection of appropriate components and the determination of their optimal ratios to ensure a safe and effective final product. Components used in drug formulation may include active pharmaceutical ingredients (APIs), excipients, and other substances that improve the stability, solubility, and bioavailability of the drug. Formulation may also affect the physical characteristics of the drug, such as appearance, taste, and texture, which can influence patient compliance and adherence.

Different types of formulations are used for different routes of administration, including oral, topical, intravenous, intramuscular, and subcutaneous. Formulations may also influence the release profile of the drug, i.e., the rate at which the drug is released into the body and the duration of its therapeutic effect. Therefore, careful consideration of drug formulation is essential to achieve optimal therapeutic outcomes and to minimize potential side effects.

In the present invention, nanoparticles engineered to have a hydrodynamic diameter of 2 to 20 nm and a surface charge of -20 mV to 0 mV through hydration of hydrophilic functional groups exposed on their surface-such that they are selectively drained into terminal lymphatic vessels without entering blood capillaries at the administration site-can be formulated without requiring further dilution to achieve optimal contrast enhancement (see Example 5). Accordingly, since the formulation can be provided as a pre-diluted vial product, it is relatively free from issues such as infection, contamination, and contrast agent concentration heterogeneity.

According to the present invention, nanoparticles engineered to have a hydrodynamic diameter of 2 to 20 nm and a surface charge of -20 mV to 0 mV through hydration of hydrophilic functional groups exposed on their surface-such that they are selectively drained into terminal lymphatic vessels without entering blood capillaries at the administration site-can be administered via intralymphatic injection, subcutaneous injection, intramuscular injection, intraperitoneal administration, endothelium-targeted delivery, topical administration, intradermal injection, intrapulmonary administration, or rectal administration.

In the present invention, it is preferable that the non-vascular administration method is selected from intradermal injection, subcutaneous injection, or direct intralymphatic injection.

Subcutaneous injection provides more rapid drug efficacy than oral administration, although the absorption is slower than that of intramuscular injection. The absorbable volume is generally within 2 cc. Provided that blood circulation is sufficient, the administered drug can be almost completely absorbed into the tissue, and the onset of action generally occurs within 30 minutes.

Meanwhile, intradermal injection refers to the administration of a small volume of solution into the dermis, the layer immediately beneath the epidermis. Due to the risk of severe hypersensitivity caused by rapid drug absorption, the dermis is selected as it has limited blood supply and slower absorption. Typically, a volume less than 0.1 mL is injected. Since the dermis lacks blood vessels, it exhibits the slowest absorption among parenteral routes. Inaccurate dosing may lead to adverse effects, and administration may cause inflammation, bleeding, or allergic reactions at the injection site.

**[MR Technique]**

An MRI sequence refers to a specific setting of the MRI system-namely, pulse sequences and pulsed field gradients-used to acquire a particular image. A gradient-echo (GRE) sequence is typically used to obtain T1-weighted images, whereas a fast spin-echo (FSE) sequence is employed for acquiring T2-weighted images.

When the nanoparticles of the present invention serve as contrast agents that produce T1-MRI contrast enhancement, the non-vascular injectable formulation of the present invention may be used in magnetic resonance lymphangiography (MRL).

Magnetic resonance lymphangiography is typically performed using the largest available phased-array coil to acquire images. For the lower extremities, images are generally acquired in four separate regions from the feet to the pelvis. For the upper extremities, imaging is typically divided into three regions, spanning from the hands to the axillary area. However, depending on the capability of the MRI system and coils, the lower extremities are more commonly imaged in three segments up to the inguinal region. Although the latest MRI systems are equipped with coils capable of covering the entire lower extremities for continuous imaging, when only a single coil is used, imaging must be performed in separate segments.

In magnetic resonance lymphangiography (MRL), high-resolution isotropic image acquisition is essential. For image evaluation, post-processing techniques such as multiplanar reformation and maximum intensity projection (MIP) are applied, with MIP images being particularly essential for assessing lymphatic vessels. In MRL, three-dimensional images are typically acquired using isovoxel settings, although in some cases, images may be acquired in the coronal plane with a 2 mm slice thickness. When axial images are reconstructed with a 3 mm slice thickness, they are generally presented as baseline images reconstructed in the axial or coronal plane to provide anatomical context. The use of

isovoxel imaging enables advantageous reconstruction across multiple planes.

According to Lohrmann et al., maximum contrast enhancement was observed at approximately 45 minutes after contrast agent injection for the lower extremities and at 55 minutes for the upper extremities. Furthermore, venous structures exhibited decreasing signal intensity over time, whereas lymphatic vessels demonstrated increasing signal intensity as time progressed. According to Liu et al., the diameter of the observed lymphatic vessels was less than 2 mm in 31% of cases, 2-5 mm in 50%, and 5-8 mm in 19%. In terms of the number of visualized lymphatic vessels, 40% of cases showed 1-2 vessels, 36% showed 3-20 vessels, and 24% showed more than 20 vessels.

Conventional MRL imaging techniques include heavily T2-weighted turbo spin echo images (T2WI), and fat-suppressed T1-weighted three-dimensional spoiled gradient echo (SPGR, GE), fast low-angle shot (FLASH, Siemens), or T1-weighted fast field echo (T1FFE, Philips). Heavily T2WI has traditionally been used and can be implemented as 3D T2WI. This technique visualizes lymphatic vessels with stagnant fluid, and since the use of subcutaneous injection of Gd-based contrast agents is limited, one advantage is that lymphatic vessels may be visualized without the need for contrast injection. However, this method only visualizes dilated lymphatic vessels and fails to depict normal vessels, thereby limiting its clinical applicability.

T1-weighted 3D imaging, which employs contrast enhancement, is a key feature of MRL and enables clear visualization of lymphatic vessels. Nevertheless, it is not widely used due to the relatively long acquisition time, which may approach 30 minutes to cover the entire upper or lower extremity. Intermediate-weighted 3D imaging provides inferior visualization of lymphatic vessels compared to 3D T1-weighted imaging.

SPGR or FLASH sequences have been traditionally used and are widely applied in various published studies. The Volumetric Interpolated Breath-hold Examination (VIBE) and the Controlled Aliasing In Parallel Imaging Results IN Higher Acceleration (CAIPIRINHA) techniques are primarily employed in abdominal or thoracic MRI and for evaluating central lymphatic vessels. VIBE is a gradient-echo sequence similar to SPGR, while CAIPIRINHA is an advanced parallel imaging technique that reduces aliasing artifacts in three-dimensional imaging. Compared to conventional parallel imaging methods, CAIPIRINHA offers improved signal-to-noise ratio and reduced imaging artifacts. VIBE, which is a modified version of FLASH, has been reported to provide superior image quality of peripheral veins relative to FLASH. In VIBE imaging, due to the short repetition time (TR), most tissues appear dark, whereas contrast agents exhibit high signal intensity.

Fat suppression imaging is essential for MR lymphangiography because nonuniform fat suppression can obscure contrast-enhanced lymphatic vessels, resulting in suboptimal image quality. Among fat suppression techniques, the multi-echo Dixon (mDixon) method is a recent advancement and is particularly effective for achieving uniform fat suppression. The T1FFE sequence incorporating the mDixon method has recently garnered attention for its performance. The acquisition times for VIBE, CAIPIRINHA, FLASH, and T1FFE-mDixon sequences are comparable. The T1FFE-mDixon technique, which enables uniform fat suppression with sufficient signal-to-noise ratio at 1 mm isovoxel resolution, represents the most recent advancement and is expected to see expanded clinical application.

Magnetic resonance lymphangiography (MRL) is currently considered the most advanced imaging modality for visualizing lymphatic vessels and lymphedema, with its use increasingly widespread. T2-weighted imaging (T2WI) remains limited to visualizing edema and anatomical structures. The three-dimensional imaging platform underlying MRL has evolved from the SPGR sequence to its advanced variants, including VIBE, CAIPIRINHA, and the T1FFE-mDixon technique. Maximum intensity projection (MIP) is an essential post-processing technique in MRL that allows comprehensive visualization of the lymphatic vessels.

**[Lymphedema and Lymphatic Disorders]**

The nanoparticles of the present invention, having a hydrodynamic diameter of 2 to 20 nm and a surface charge of -20 mV to 0 mV through hydration of hydrophilic functional groups exposed on the surface, are engineered to be selectively drained into terminal lymphatic vessels without infiltrating or entering blood capillaries at the administration site. When such nanoparticles contain at least one element selected from the group consisting of iron, manganese, and gadolinium, they not only serve as contrast agents for effective imaging of disease diagnosis and biological phenomena at the molecular and cellular levels but also allow the formulation for non-vascular administration to be used in the assessment of lymphatic flow, drainage, and congestion.

Accordingly, the contrast agent composition for non-vascular administration according to the present invention enables imaging of lymphatic vessels, lymphatic ducts, and/or lymph nodes.

Furthermore, when the nanoparticles of the present invention function as MRI contrast agents, their absorption or injection into the lymphatic vessels enables magnetic resonance imaging (MRI) to visualize abnormalities in lymphatic circulation, including lymphatic obstruction, lymphedema, swollen lymph nodes, lymphadenitis, lymphoma, and the migration of tumors in other organs to regional lymph nodes.

Moreover, the contrast agent composition for non-vascular administration of the present invention may be used to provide information necessary for diagnosing lymphatic diseases without venous contamination. Prior to the discharge

of the nanoparticles into the venous circulation via the lymphatic vessels, vascular enhancement is excluded, allowing vascular imaging noise to be eliminated. Thus, lymphatic obstruction may be visualized. In addition, the formulation may provide information necessary for the prevention or early diagnosis of lymphedema.

Preferably, the non-vascular administration formulation of the present invention employs nanoparticles engineered to avoid dermal backflow in lymphatic vessels or lymph node congestion at lymphedematous sites.

Lymphedema occurs when the volume of interstitial fluid, which is filtered from blood capillaries and enters primary lymphatic vessels, exceeds the transport capacity of the lymphatic system. Primary lymphatic vessels lack muscle cells except for the valves that direct lymph flow in a unidirectional manner. Therefore, lymph is transported from the primary to secondary lymphatic vessels by external mechanical forces such as contraction and relaxation of limb muscles or compression and decompression of the skin, functioning similarly to a pump-this mechanism is referred to as the "lymphatic pump." The lymphatic pump can also be activated by subtle physiological motions, including cardiac pulsation, respiration, and intestinal peristalsis. Lymphedema may result from an increased volume of interstitial fluid due to conditions such as deep vein thrombosis, varicose veins, hypoalbuminemia, or heart failure. It may also arise from decreased lymph transport capacity due to congenital lymphatic malformations, lymphatic vessel damage caused by surgery, radiation therapy, infection, or trauma, or impaired lymphatic pump function associated with prolonged immobilization. If lymphedema persists chronically, tissue fibrosis may occur, leading to morphological deformities. Patients may experience sensations of heaviness, stiffness, and pain depending on the severity of the edema. These symptoms can further reduce physical activity, leading to diminished lymphatic pump function and progressively worsening lymphatic transport capacity, thereby exacerbating the edema.

Since lymphedema becomes difficult to treat once subcutaneous tissue fibrosis has progressed over time, prevention and early diagnosis are critical. When a patient presents with edema, a comprehensive evaluation should be conducted to identify any systemic diseases or medications that may cause or exacerbate the condition. This includes obtaining a detailed medical history, performing a physical examination, and conducting basic screening tests such as complete blood count (CBC), liver function tests (LFTs), renal function tests, electrolyte panel, serum albumin, thyroid function tests, fasting blood glucose, inflammatory markers (erythrocyte sedimentation rate and C-reactive protein), brain natriuretic peptide (BNP), hepatitis panel, urinalysis, chest radiography, and electrocardiography (ECG). When the underlying cause of lymphedema remains unclear, additional diagnostic procedures such as bioelectrical impedance analysis, Doppler ultrasonography, lymphoscintigraphy, computed tomography (CT), magnetic resonance imaging (MRI), genetic testing, and filariasis screening should be performed to determine the etiology of the edema.

Peripheral lymphedema is a condition that may improve with medical management, but it often progresses to chronic edema, leading to recurrent infections, changes resembling elephantiasis, severe disability, and, in rare cases, serious complications such as lymphangiosarcoma (Stewart-Treves syndrome). Peripheral lymphedema is primarily associated with treatments for breast cancer and gynecologic malignancies. The incidence of upper extremity lymphedema following breast cancer surgery varies widely, ranging from 2% to 65%, and it typically arises after lymph node dissection or lymphatic radiotherapy. In the case of gynecologic cancers, the incidence is approximately 11.1% to 50%.

Lymph node dissection or radiotherapy can damage or obstruct central lymphatic vessels, inhibiting centripetal lymph drainage, increasing intralymphatic pressure, and inducing pathological changes in the lymphatic system. The resulting lymphedema leads to the accumulation of protein-rich fluid in the interstitial tissue, which triggers inflammatory responses, promotes adipocyte proliferation and fibrotic tissue deposition, and increases susceptibility to infection.

The lymphatic system not only plays a passive role in transporting fluid, fatty acids, and immune cells but also actively regulates immune responses. It is influenced by changes in the immune microenvironment, and impaired immune responses may lead to lymphatic dysfunction.

## ADVANTAGEOUS EFFECTS

According to the present invention, the nanoparticles with a controlled hydrodynamic diameter can be excreted from the body without any residual presence at the administration site within one week after administration. When engineered as a T1-MRI contrast agent, the nanoparticles enable selective imaging of peripheral and/or central lymphatic vessels upon administration to perivascular tissues, without venous contamination.

The lymphatic contrast agent according to the present invention can highlight lymphatic vessels and facilitate the assessment of lymphatic flow, drainage, and congestion. Magnetic resonance lymphangiography (MRL) can provide clinically significant information in specific cases, particularly in the evaluation of lymphatic disorders such as lymphedema, and in the preoperative assessment of lymphatic vessel damage or lymphatic drainage patterns. In such clinical contexts, MRL may assist in guiding clinical decision-making and treatment planning.

## BRIEF DESCRIPTION OF THE DRAWINGS/FIGURES

**FIG. 1** is a schematic diagram illustrating the infiltration pathway of a contrast agent into lymphatic vessels or veins following intradermal/subcutaneous injection; and a schematic representation of the lymph-selective contrast enhancement mechanism of the lymphatic contrast agent according to the present invention, which enables lymphatic vessel imaging without venous contamination.

**FIG. 2** is a schematic representation of the experimental workflow of Example 1: (a) Magnetic resonance lymphangiography (MRL) timeline; (b) Methylene blue staining performed 2 days after MRL (yellow triangle: popliteal lymph node (LN)); (c) Region of interest (ROI) designated for analysis (red circle: popliteal LN; blue circle: muscle; yellow circle: background; red arrow: lymphatic vessel; blue arrow: muscle; yellow arrow: background).

**FIG. 3** shows the characteristics of INV-001 used in Example 1. (a) The structure of INV-001 consists of a dextran core and an iron oxide shell. (b) The hydrodynamic size of INV-001. (c) T1-weighted (T1-WI), T2-weighted (T2-WI), T1 mapping, and T2 mapping images of a serially diluted INV-001 phantom. (d) T1 and T2 relaxation rates as a function of concentration and their linear fitting. The slope of the line indicates the $r_1$ and $r_2$ relaxivities.

**FIG. 4** compares the effects of Gd-DOTA and INV-001 on popliteal lymph node (LN) and lymphatic vessel enhancement in Example 1. (a) 3D time-of-flight (TOF) scan images taken before (0 minutes), 16 minutes, and 96 minutes after intradermal injection of Gd-DOTA or INV-001. (b) Signal-to-noise ratio (SNR) and contrast-to-noise ratio (CNR) analysis of the popliteal LN and lymphatic vessels (LV) after administration of Gd-DOTA or INV-001.

**FIG. 5** compares the presence or absence of venous contamination after administration of Gd-DOTA and INV-001 in Example 1. MRL images using Gd-DOTA and INV-001, and methylene blue-stained images for lymphatic confirmation. Lymph nodes and lymphatic vessels were visualized 16 minutes after administration of different concentrations into the hindlimb: (a, e) Gd-DOTA and INV-001 at 2.25 μmol (75 μL, 30 mM), (b, f) 1.125 μmol (75 μL, 15 mM), (c, g) 0.45 μmol (30 μL, 15 mM), (d, h) Methylene blue staining. Yellow triangles, yellow arrows, and red arrows indicate lymph nodes, lymphatic vessels, and the saphenous vein, respectively.

**FIG. 6** shows the results of a dose optimization study conducted in Example 1 to minimize interstitial space enhancement and lymph node enlargement. Various concentrations and volumes were injected into the hindlimb, and visualization of the popliteal lymph nodes (LNs) and lymphatic vessels (LVs) was performed 16 minutes after injection.

**FIG. 7** shows the qualitative scoring results of Gd-DOTA and INV-001 in Example 1. (a, b) Qualitative scores for each group under various injection conditions. (c) MRL images taken 16 minutes after intradermal injection of Gd-DOTA or INV-001 and methylene blue staining. Yellow triangles, yellow arrows, and red arrows indicate lymph nodes, lymphatic vessels, and the saphenous vein, respectively.

**FIG. 8** shows a comparison of concentration and viscosity at maximum concentration between the nanostructure for intradermal/subcutaneous/intravenous injection (coded as INV-001) and the nanostructure for intra-articular injection (coded as INV-002), synthesized in Example 2.

**FIG. 9** is a series of MRL images of the mouse hindlimb captured at 16-minute intervals over 96 minutes after administration of INV-001.

**FIG. 10** illustrates venous contamination as verified by methylene blue staining (left), and comparison of venous contamination in MRL images following intradermal injection of INV-001 and Dotarem (right).

**FIG. 11** shows an MRL image of the mouse hindlimb taken 24 hours after intradermal injection of INV-001, demonstrating that the administered INV-001 was completely cleared.

**FIG. 12** illustrates the experimental design for dose setting in the beagle dog MRL study using INV-001.

**FIG. 13** illustrates the setting of regions of interest (ROIs) for SNR and CNR analysis in the beagle dog MRL study using INV-001.

**FIG. 14** shows the MRL images at various concentrations corresponding to doses of 0.028 mg/kg (a), 0.056 mg/kg (b), and 0.112 mg/kg (b) in the beagle dog MRL study using INV-001.

**FIG. 15** shows the quantitative analysis of $SNR_{LN}$, $SNR_{LV}$, $CNR_{LN}$, and $CNR_{LV}$ values over time according to the dose and injection volume in the beagle dog MRL study using INV-001.

**FIG. 16** shows MRL images demonstrating reproducibility at doses of 0.028 mg/kg, 0.056 mg/kg and 0.112 mg/kg in the beagle dog MRL study using INV-001.

**FIG. 17** shows quantitative analysis results confirming reproducibility at doses of 0.056 mg/kg and 0.112 mg/kg, including time-dependent changes in $SNR_{LN}$, $SNR_{LV}$, $CNR_{LN}$, and $CNR_{LV}$ $SNR_{ln}$, $SNR_{lv}$, $CNR_{ln}$, and $CNR_{lv}$ in the beagle dog MRL study using INV-001.

**FIG. 18** is a photograph showing intradermal injection of the INV-001 contrast agent into the hindlimb of a rat using a 31-gauge syringe in Example 2-13.

**FIG. 19** illustrates the intradermal injection site in the beagle dog MRL study using INV-001.

**FIG. 20** shows the T1 (A), T2 (B), and T2* (C) relaxation values in the liver and kidneys before and 48 hours after administration in the beagle dog MRL study using INV-001, indicating that the administered agent was completely excreted without accumulation.

**FIG. 21** shows MRL images before administration, 30 minutes after administration, and 48 hours after administration

in the beagle dog MRL study using INV-001, demonstrating that no residual INV-001 remained at the injection site at 48 hours.

**FIG. 22** shows magnetic resonance arthrography (MRA) efficacy data obtained by injecting INV-002 into the rat joint cavity. (a) and (b) are schematic illustrations of intra-articular anatomical structures. (c) and (d) are MRI images of rat joints in the INV-002-injected group and the saline-injected control group. (e) shows a comparison of T1 MRI signal intensities before and after INV-002 injection. (f) shows a comparison of the contrast-to-noise ratio (CNR) for intra-articular tissues before and after administration.

**FIG. 23** shows additional MRA efficacy data obtained by injecting INV-002 into the rat joint cavity. (a) and (b) are MRI images of rat joints in the INV-002-injected group and the gadolinium-based contrast agent (Dotarem)-injected control group. (c) shows the time-dependent comparison of T1 MRI signal intensities in the joint cavity after injection of INV-002 and Dotarem.

**FIG. 24** presents urine analysis data after intra-articular injection of INV-002 or saline in rats. (a) shows a comparison of iron content in urine samples collected after injection. (b) shows a visual comparison of urine sample color.

**FIG. 25** shows trace iron analysis data in rat joint tissues using Perls' Prussian blue immunohistochemical staining. (a) is a microscopic image of joint tissue from a rat injected with INV-002. (b) is a microscopic image of joint tissue from a saline-injected control rat.

**FIG. 26** shows T1 MRI images over time after injection of INV-002 into the rat spinal canal.

**FIG. 27** presents body weight trend data measured for 14 days after intra-articular injection of INV-002 in rats.

**FIG. 28** shows blood chemistry data analyzed after intra-articular injection of INV-002 in rats.

**FIG. 29** compares the structure (a), MRI phantom image (b), and relaxivity (d) of the dextran-crosslinked nanoparticle-based T1 MRI contrast agent NEMO-103 of Example 4-2 with those of a representative clinical gadolinium-based contrast agent (GBCA), Dotarem (c, e).

**FIG. 30** shows the imaging phase timeline of magnetic resonance arthrography (MRA) used in the clinical comparison study between the dextran-crosslinked T1 MRI contrast agent NEMO-103 (iron-based contrast agent, IBCA) and GBCA.

**FIG. 31** illustrates the clinical comparison study protocol between NEMO-103 and GBCA.

**FIG. 32** shows MRA images captured at 30 minutes (a) and 60 minutes (b) after administration of NEMO-103 in humans, and a comparative analysis in terms of contrast-to-noise ratio (CNR) (c), distension (d), and overall image quality (e).

**FIG. 33** shows MRA images captured at 30 minutes after administration of NEMO-103 (a) and a gadolinium-based contrast agent (GBCA, b) in humans, with comparative evaluation in terms of CNR (c), distension (d), and overall image quality (e).

**FIG. 34** shows MRA images captured approximately 60 minutes after administration of NEMO-103 (a, c) and GBCA (b, e) in humans, with comparative assessment of CNR (c), distension (d), and overall image quality (e).

**FIG. 35** shows the surface charges of C-DNPs synthesized by adjusting the amount of succinic anhydride to substitute the surface-exposed functional groups with carboxyl groups.

**FIG. 36** illustrates the electron microscopy image of iron oxide nanoparticles (IONPs) (a); the observed size of the IONPs from electron microscopy (4 nm, b); the size of IONPs coated with C-DNP-3, C-DNP-5, and C-DNP-10 substituted with carboxyl groups (c); and the size of IONPs coated with C-CMDNP-10 (d).

**FIG. 37** illustrates the surface charge of iron oxide nanoparticles coated with C-DNPs having surface charges controlled.

**FIG. 38** shows (a) the colloidal stability and (b) the change in hydrodynamic diameter over time of C-DNP-coated iron oxide nanoparticles under various physiological conditions (salt concentration and pH).

**FIG. 39** illustrates (a) the colloidal stability and (b) the hydrodynamic diameter of iron oxide nanoparticles depending on the amount of dextran (molecular weight: 10 kDa) used for coating.

**FIG. 40** shows electrophoresis results demonstrating the anti-opsonization effect of iron oxide nanoparticles coated with C-DNP-5 having surface charges of +5 mV (a), -3 mV (b), and -20 mV (c), according to one embodiment of the present invention.

**FIG. 41** presents MRI images showing the pharmacokinetics of IONPs coated with C-DNPs designed to exhibit the following properties through size and surface charge control: hydrodynamic diameter of 7 nm and surface charge of -3 mV (a); hydrodynamic diameter of 11 nm and surface charge of -3 mV (b); and hydrodynamic diameter of 11 nm and surface charge of -30 mV (c).

**FIG. 42** shows the structural formulas of exemplary branched polysaccharides or cyclic polysaccharides that are suitable for use in the polysaccharide-crosslinked colloidal particles.

**FIG. 43** illustrates confirmation of the formation of crosslinked structures using a UV-Vis spectrophotometer for Dextran T-5 (a), Maltodextrin (b), α-cyclodextrin (c), β-cyclodextrin (d), and Inulin (e).

**FIG. 44** shows MRI phantom images and $r_1$ and $r_2$ relaxivity values (f) of materials synthesized by introducing iron into the crosslinked structures of Dextran T-5 (a), Maltodextrin (b), α-cyclodextrin (c), β-cyclodextrin (d), and Inulin (e).

**FIG. 45** shows T1-weighted MRI images demonstrating vascular enhancement along with signal-to-noise ratios (SNRs) before and after administration, obtained from mice intravenously injected with materials synthesized by introducing iron into crosslinked structures synthesized from Dextran T-5, Maltodextrin, α-cyclodextrin, β-cyclodextrin, and Inulin.

**FIG. 46** shows T1-weighted MRI images obtained from mice intravenously injected with materials synthesized by introducing iron into crosslinked structures synthesized from Dextran T-5 (a), Maltodextrin (b), α-cyclodextrin (c), β-cyclodextrin (d), and Inulin (e), thereby confirming renal excretion.

## Mode for carrying out the invention

Hereinafter, the present invention will be described in further detail with reference to specific examples. However, these examples are provided for the purpose of illustrating the technical features of the invention more clearly and are not intended to limit the scope of the invention in any way.

## Example 1: Magnetic Resonance Lymphangiography (MRL)

This example compares the pharmacokinetics of a gadolinium-based contrast agent and an iron oxide-based contrast agent within the lymphatic structure using magnetic resonance lymphangiography (MRL).

Gadolinium (Gd)-based contrast agents are commonly used in contrast-enhanced MRL. However, overcoming the issue of venous contamination remains a significant challenge. This example evaluates the MRL efficacy of a newly developed iron-based contrast agent (INV-001) that is specifically designed to achieve clear lymphatic imaging without venous contamination. In addition, this study aims to identify the optimal dose, including the injection volume and concentration, required for successful visualization of the popliteal lymph node and surrounding lymphatic vessels.

### 1-1. Preparation of Gd-DOTA and INV-001

Gd-DOTA (Gd-DOTA, DOTAREM, Guerbet, Roissy CdG Cedex, France) was used as a Gd-based contrast agent. The Gd-DOTA stock solution, provided at a concentration of 500 mM, was diluted with normal saline before its administration.

INV-001 was synthesized as follows. A 20 mM solution of dextran T5 (average molecular weight: 5,000 Da) was mixed with a sodium hydroxide solution and epichlorohydrin, followed by the addition of diethylenetriamine. The crosslinked dextran was purified using a 3-kDa molecular weight cut-off ultrafiltration filter. The terminal amine groups were further modified into carboxyl groups. After ultrafiltration, the carboxyl-modified dextran was reacted with an iron chloride solution for 1 hour, and the final product was purified and concentrated via ultrafiltration.

### 1-2. Hydrodynamic Size and MR Relaxivity of INV-001

The hydrodynamic size of INV-001 was measured to be 3.6 nm (FIG. 3b). Subsequently, the $T_1$ and $T_2$ relaxation rates ($1/T_1$ and $1/T_2$) of INV-001 were measured at 9.4 T (FIG. 3c). The longitudinal and transverse relaxivities ($r_1$ and $r_2$) were calculated at various INV-001 concentrations (0.125, 0.25, 0.5, and 1.0 mM). The $r_1$ was determined to be 2.61 $mM^{-1}\cdot s^{-1}$ and the $r_2$ was 4.33 $mM^{-1}\cdot s^{-1}$ (FIGS. 3d and 3e).

The contrast agent INV-001 has a size larger than 2 nm, which is the maximum size capable of intravasation, and therefore cannot enter blood vessels. Accordingly, in principle, when administered intradermally or subcutaneously, it can be used as a lymphatic-specific contrast agent that permeates into lymphatic vessels without venous contamination.

### 1-3. Comparison of SNR and CNR Between INV-001 and Gd-DOTA

The signal-to-noise ratio (SNR) and contrast-to-noise ratio (CNR) of the popliteal lymph node and surrounding lymphatic vessels were analyzed at a dose of 1.125 μmol (75 μL, 15 mM) of INV-001 and Gd-DOTA. Through sequential MRI imaging, dynamic patterns of SNR and CNR and peak time points in the popliteal lymph node and lymphatic vessels were obtained (FIG. 4a). In the group receiving 1.125 μmol Gd-DOTA, peak enhancement of the popliteal lymph node was observed at 16 minutes ($SNR_{LN}$: 26.96 ± 10.78; $SNR_{LV}$: 11.15 ± 5.83; $CNR_{LN}$: 21.61 ± 10.04; $CNR_{LV}$: 7.89 ± 5.15). In the 1.125 μmol INV-001 group, peak enhancement appeared between 16 and 32 minutes during extended visualization ($SNR_{LN}$: 23.49 ± 8.64; $SNR_{LV}$: 16.05 ± 3.52; $CNR_{LN}$: 15.87 ± 11.05; $CNR_{LV}$: 12.58 ± 3.37) (FIG. 4b).

### 1-4. Observation of Venous Contamination, Interstitial Space Enhancement, and Lymph Node Enlargement

Following injection of the contrast agents into the hind limb, magnetic resonance lymphangiography (MRL) was

performed using 3D TOF under saturation band conditions to distinguish lymphatic vessels. For Gd-DOTA, venous contamination was observed in the 2.25 μmol (75 μL, 30 mM) and 1.125 μmol (75 μL, 15 mM) groups, except for the 0.45 μmol (30 μL, 15 mM) group (FIGs. 5a and 5b, red arrows). In contrast, in all INV-001 groups, the popliteal lymph node and lymphatic vessels were clearly visualized without venous contamination (FIGs. 5e, 5f, and 5g, yellow arrows). Methylene blue was injected two days after MRL to verify the anatomical structures (FIGs. 5d and 5h).

### 1-5. Qualitative Image Analysis for Various Injection Parameters

Qualitative image analyses under various injection conditions were performed using Gd-DOTA and INV-001. In all groups, MRL provided good visualization of the popliteal lymph node and lymphatic vessels (FIG. 6). The qualitative image analysis was conducted according to the criteria shown in Table 1 (Criteria for qualitative score).

[Table 1]

| Description | Qualitative score | | | |
|---|---|---|---|---|
| | 0 | 1 | 2 | 3 |
| Venous contamination | Present | Absent | - | - |
| Dermal backflow | Present | Absent | - | - |
| Lymph node congestion | Present | Absent | - | - |
| Visualization | Poor | Moderate | Good | Excellent |

When varying the injection concentration, INV-001 at 0.75 μmol (75 μL, 10 mM), 1.125 μmol (75 μL, 15 mM), and 1.5 μmol (75 μL, 20 mM), and Gd-DOTA at 2.25 μmol (75 μL, 30 mM) received scores of 4.2, 5, 5, and 3.8, respectively (FIG. 7a). When adjusting the injection volume, INV-001 at 0.3 μmol (20 μL, 15 mM), 0.45 μmol (30 μL, 15 mM), and 0.75 μmol (50 μL, 15 mM), and Gd-DOTA at 2.25 μmol (75 μL, 15 mM) received scores of 3.6, 4.4, 4.6, and 3.2, respectively (FIG. 7b). Overall, the qualitative image analysis of INV-001 showed that both lymphatic vessels and lymph nodes were well visualized without venous contamination or dermal backflow. Notably, INV-001 at 0.45 μmol (15 mM, 30 μL) and 0.75 μmol (15 mM, 50 μL) achieved the highest scores of 4.4 and 4.6, respectively (FIGS. 7b and 7c).

[Discussion]

INV-001 is an iron-based nano-MRI contrast agent specifically engineered for magnetic resonance lymphangiography (MRL). In phantom studies, INV-001 demonstrated T1 contrast enhancement comparable to that of gadolinium-based agents (INV-001 ≒ 2.6 $mM^{-1}s^{-1}$ vs. Gd-DOTA ≒ 3.7 $mM^{-1}s^{-1}$). Subsequent in vivo comparisons between INV-001 and the gadolinium-based contrast agent Gd-DOTA showed that administration of 1.125 μmol INV-001 resulted in relatively higher contrast-to-noise ratio (CNR) and signal-to-noise ratio (SNR) in the popliteal lymph node and surrounding lymphatic vessels. INV-001 also exhibited a more prolonged enhancement in the popliteal lymph node compared to Gd-DOTA.

This delayed washout of INV-001 suggests the potential for a wider time window for scanning compared to Gd-DOTA.

Unlike Gd-DOTA, which exhibited venous contamination in most animals at doses of 0.45 μmol or higher, INV-001 did not result in any venous contamination. This distinctive property of INV-001 appears to derive from its optimized hydrodynamic size (3.6 nm), which prevents intravascular infiltration.

The size of a contrast agent has been reported to determine its pharmacological and pharmacokinetic properties, including the rapid clearance of small molecules in MRL. Because gadolinium-based agents are small in size (approximately 1 nm, molecular weight <1,000 g/mol), they are rapidly washed out through the venous system, resulting in venous contamination.

Finally, it was confirmed that INV-001 did not remain at the administration site, in the lymphatic vessels, or in the lymph nodes at 24 or 48 hours post-injection. In addition, statistical analysis comparing T1, T2, and T2* relaxation times in the liver and kidneys before and after injection revealed no significant differences, suggesting that INV-001 was excreted from the body within 24 to 48 hours (data not shown).

In conclusion, this example demonstrates the feasibility of INV-001 as a T1 contrast agent for MRL.

Accordingly, INV-001 may be potentially utilized as a T1 contrast agent for improved diagnosis and monitoring of lymphatic diseases.

**[Conclusion]**

This example demonstrated that the novel contrast agent INV-001 provided prolonged enhancement of the visualization of the popliteal lymph nodes and lymphatic vessels without venous contamination. By optimizing the injection parameters, INV-001 enabled clear visualization of the popliteal lymph nodes and lymphatic vessels, with the highest qualitative scores observed at doses of 0.45 μmol (15 mM, 30 μL) and 0.75 μmol (15 mM, 50 μL). These results highlight the potential of INV-001 as a contrast agent for magnetic resonance lymphangiography (MRL).

**Example 2**

**2-1. Preparation of Contrast Agent for Magnetic Resonance Lymphangiography (MRL)**

Dextran (180 μmol, average molecular weight of 5,000 Da) was dissolved in 9 mL of distilled water, followed by the addition of 75 mmol of epichlorohydrin and 75 mmol of NaOH. Subsequently, 380 mmol of diethylenetriamine was added, and the solution was stirred at room temperature (RT) for 24 hours. After the 24-hour succinylation reaction at RT, the dextran core was purified via ultrafiltration using a 5 kDa molecular weight cutoff (MWCO) filter. An excess amount of ferric chloride hexahydrate ($FeCl_3 \cdot 6H_2O$) solution was then added to the dextran core solution. The pH was adjusted to 8 using 1 M NaOH, and the mixture was reacted at RT for 1 hour. The resulting product was purified by dialysis to yield the nanostructure (code name: INV-001).

The maximum iron concentration of INV-001 upon concentration was 2.6 mg/mL, with a viscosity of 5.3 cP (see FIG. 8).

The hydrodynamic diameter of the synthesized INV-001 was 3.9 nm with a surface charge of -3.3 mV. The $r_2/r_1$ ratio measured at 3 T MRI was 1.22 ($r_1$ = 3.57 $mM^{-1}s^{-1}$, $r_2$ = 4.35 $mM^{-1}s^{-1}$), which is very close to the theoretically ideal value of 1 for $T_1$ MRI contrast agents, indicating that INV-001 is suitable as a $T_1$ MRI contrast agent. The particle size of this contrast agent exceeds the maximum size (2 nm) permissible for intravasation, thereby preventing its entry into blood vessels. Thus, it is theoretically suitable for use as a lymphatic-specific contrast agent that infiltrates the lymphatic vessels without venous contamination when administered intradermally or subcutaneously.

The contrast agent was prepared at metal-based concentrations of 10 mM, 15 mM, and 20 mM. An appropriate amount of mannitol was added to adjust the osmolarity to 280-340 mOsm/kg, rendering the solution isotonic.

**2-2. Method of Administration of Contrast Agent for Magnetic Resonance Lymphangiography**

INV-001 was administered intradermally into the interdigital space of the lower limb of animals using a 31-gauge syringe. The injection volume was set at 10, 20, 30, 40, 50, or 75 microliters per animal, corresponding to a human equivalent dose of 2, 4, 6, 8, 10, or 15 mL, respectively.

**2-3. Animal Preparation for Magnetic Resonance Lymphangiography**

Prior to MRI acquisition, the animals were anesthetized by inhalation of isoflurane delivered in oxygen at a concentration of 0.5-2%. If necessary, local anesthesia with lidocaine was applied to relieve procedural discomfort.

**2-4. Method of Magnetic Resonance Lymphangiography Image Acquisition**

Baseline MRI images were acquired prior to the administration of the contrast agent. Following the injection of INV-001 prepared in Example 2-1, MRI scans were conducted every 16 minutes for a total of 96 minutes. The imaging sequence used was 3D Time-of-Flight Gradient Echo (3D TOF GRE), with images obtained in axial, sagittal, and coronal planes. The spatial resolution was set to approximately 0.3 $mm^3$ voxel size.

**2-5. Analysis of Contrast Enhancement in Magnetic Resonance Lymphangiography**

Maximum intensity projection (MIP) images were generated from the baseline and post-injection MRI data acquired at 16-minute intervals for 96 minutes. Lymphatic vessels and blood vessels exhibiting bright signal enhancement following contrast injection, relative to baseline, were identified. The overall image quality, including vessel discontinuity, was assessed using a four-point scale (-, +, ++, +++). Additionally, the presence of dermal backflow and lymph node congestion was evaluated.

## 2-6. Confirmation of Contrast Agent Absorption into lymphatics at the Intradermal Injection Site

MRL was performed using 3D Time-of-Flight Gradient Echo (3D TOF GRE), and the images were compared with baseline MRI to confirm whether the contrast agent had been absorbed into lymphatic vessels.

In the case of INV-001, lymphatic vessel enhancement was observed starting from 16 minutes post-injection and remained visible until 64 minutes, demonstrating effective lymphatic contrast enhancement (see FIG. 9). Furthermore, fine lymphatic vessels with diameters on the order of 300 μm were clearly visualized.

## 2-7. Analysis of Venous Contamination

After all MRI imaging was completed, the animals were euthanized and shaved the following day. Methylene blue, a dye known to selectively stain lymphatic vessels, was injected intradermally to anatomically identify the lymphatic vessels. This enabled anatomical correlation with MRI images to assess the presence or absence of venous contamination.

When methylene blue was injected intradermally into the lower limb of rats, the location of the lymphatic vessels could be precisely identified, allowing for direct comparison with MRI data to confirm venous contamination (see FIG. 10, left panel). In a comparative analysis between INV-001 and a conventional low-molecular-weight contrast agent, Dotarem (molecular weight: 753.9 g/mol; size <1 nm), INV-001 provided selective enhancement of lymphatic vessels only, whereas Dotarem preferentially enhanced veins more than lymphatic vessels, indicating the occurrence of venous contamination (see FIG. 10, right panel). Since INV-001 has a hydrodynamic diameter of 3.9 nm, which exceeds the commonly accepted maximum size for venous permeation (2 nm), it was confirmed that INV-001 enables selective lymphatic imaging without venous contamination.

## 2-8. Method for Confirming Excretion of Intradermally Injected Contrast Agent (1)

To confirm the clearance of the injected contrast agent, the administration site was evaluated 24 hours after intradermal injection. When INV-001 was injected intradermally into the lower limb of rats at a concentration of 20 mM, no T1 signal attributable to the contrast agent was observed at the injection site after 24 hours on MRI, indicating that the contrast agent had been completely absorbed into the lymphatic system and subsequently excreted (see FIG. 11).

## Example 3. Evaluation of the Efficacy of INV-001 in Magnetic Resonance Lymphangiography (MRL) in Healthy Beagle Dogs

The objective of this example was to verify the efficacy of INV-001 in visualizing lymphatic vessels without venous contamination. Additionally, this study aimed to determine the optimal administration conditions of INV-001 for MRL in beagle dogs.

## 3-1. Animal and experiment designation

All animal experiments were performed in accordance with ARRIVE guidelines and regulations of Institutional Animal Care and Use Committee (IACUC) of Daegu-Gyeongbuk Medical Innovation Foundation (IACUC-No. KMEDI-23050401). In addition, all procedures in this study followed the Animal Protection Act of Republic of Korea and the Guide for the Care and Use of Laboratory Animals. In this study, 2-y-old healthy male beagles (n = 3) that were 9.3 to 10.05 kg were used. After an overnight fast (more than 12 h), the beagles were pre-anesthetized intramuscularly with 0.05 mg/kg of atropine and 2 mg/kg of xylazine. An intravenous line was placed using the aseptic technique into the dorsal vein of the hindfoot, and 2-3 mg/kg of alfaxalone was administrated. When each beagle reached an adequate state of anesthetic, it was intubated with an adequately sized endotracheal tube. The beagle was then placed on the table and connected to the anesthetic machine and ventilator. General anesthesia was maintained with inhaled 1-2% isoflurane delivered through a precision vaporizer. INV-001 was injected intradermally into four dorsal webspace at each hind limb using a small syringe. The injection sites of each dorsal webspace had to be massaged for approximately 2 min after INV-001 administration to facilitate absorption in the lymphatic vessels.

First, we performed a dose-finding study using variable concentrations (7.5, 15, and 30 mM) and administration volumes (0.028, 0.056, and 0.112 mg Fe/kg) to determine the optimal concentration and injection volume of INV-001 for MRL in healthy beagles. Based on the qualitative visualization score analysis of lymphatic vessels and lymph nodes, we increased the administration concentrations and volumes of INV-001 from 7.5 Mm and 0.028 mg Fe/kg ($1^{st}$) to 15 Mm and 0.056 mg Fe/kg ($6^{th}$), respectively, according to the adaptive optimal dose-finding study design (FIG. 12). In the next phase, the reproducibility studies for determined optimal volume (0.056 and 0.112 mg Fe/kg) with a concentration of 15 mM were performed. Lastly, an excretion study of INV-001 was conducted in three healthy beagles. The excretion study

was used to ascertain the presence of INV-001 at various concentrations and administration volumes (15 mM with 0.056 mg Fe/kg, 30 mM with 0.028 mg Fe/kg, and 30 mM with 0.056 mg Fe/kg) in the body within 48 h after administration, utilizing $T_1$, $T_2$, and $T_2$* relaxation time measurements.

### 3-2. MRL acquisition and scan parameters

All of the MRL images were taken with the healthy beagle in the supine position using a 3.0-Tesla (T) whole body clinical magnetic resonance imaging scanner (MAGNETOM Skyra, Siemens Healthcare, Erlangen, Germany) with a maximum gradient strength of 45 mT/m and a slew rate of 200 T/m/s). The MRL images were obtained before and after contrast agent administration. We used two 18-channel body coils (Siemens Healthcare) with 18 integrated preamplifiers to image a wide range of the lower lymphatic vessels from the pelvis to the toes. In addition, the dual echo three-dimensional (3D) volumetric interpolated breath-hold examination (VIBE) sequence with fat suppression (Dixon technique) was used to acquire $T_1$ weighted imaging in the coronal orientation with two stacks. The utilized scan parameters were as follows: field of view = 300 × 300 × 115 mm, repetition time (TR) = 6.06 ms, echo time (TE) =2.46 / 3.69 ms, voxel size = 0.8 × 0.8 × 0.8 mm, acquisition matrix = 384 × 307, acceleration factor = 2, flip angle = 20°, average = 1, and slice thickness/slice gap = 0.8/0 mm. A more detailed summary of the imaging parameters used for MRL is shown in Table 2. After contrast agent administration, MRL imaging was performed in five cycles (30, 40, 50, 60, and 70 min) on a coronal 3D VIBE sequence with fat suppression, except for the administration of 7.5 mM and 0.028 mg Fe/kg. In the case of the administration of 7.5 mM and 0.028 mg Fe/kg, there was no visualization of lymphatic vessels except for the popliteal lymph node. Thus, MRL images were no longer acquired after 50 min. Furthermore, variable TR, turbo spin echo (TSE) with multi-echo, and gradient with multi-echo sequence were used to acquire $T_1$, $T_2$, and $T_2$* relaxation times, respectively. The utilized scan parameters are shown in Tables 2 and 3.

[Table 2]

| (1) Dose finding study for concentration and volume escalation | No. of beagles |
|---|---|
| 1. Concentration (7.5 mM) and volume (0.028 mg Fe/kg) | 1 |
| 2. Concentration (15 mM) and volume (0.028 mg Fe/kg) | 1 |
| 3. Concentration (30 mM) and volume (0.028 mg Fe/kg) | 1 |
| 4. Concentration (30 mM) and volume (0.056 mg Fe/kg) | 1 |
| 5. Concentration (15 mM) and volume (0.056 mg Fe/kg) | 1 |
| 6. Concentration (15 mM) and volume (0.112 mg Fe/kg) | 1 |
| (2) Reproducibility study for determined volume (with conc. of 15 mM) | No. of beagles |
| 1. Concentration (15 mM) and volume (0.056 mg Fe/kg) | 3 |
| 2. Concentration (15 mM) and volume (0.112 mg Fe/kg) | 3 |
| (3) Excretion study (48-hour delay) | 3 |
| Total | 15 |

[Table 3]

| **3D $T_1$ VIBE sequence** | |
|---|---|
| TR (ms) | 6.06 |
| TE (ms) | 2.46 / 3.69 |
| [a]Acceleration factor | 2 |
| Phase encoding direction | Right to Left |
| Phase oversampling | 30% |
| Flip angle | 20° |
| Bandwidth (Hz/pixel) | 690 |
| FOV (mm) | 300 × 300 |
| Acquisition matrix | 384 × 307 |

(continued)

| 3D $T_1$ VIBE sequence | |
|---|---|
| Number of slices | 144 |
| Scan orientation | Coronal plane |
| Slice thickness / gap (mm) | 0.8/0 |
| Fat suppression | Dixon technique |

**a:** A parallel imaging technique known as generalized autocalibrating partially parallel acquisitions (GRAPPA) with an acceleration factor of 2 was used. MR lymphangiography was acquired in two stacks to visualize the extensive lower lymphatic vessels. VIBE: volumetric interpolated breath-hold examination; TR: repetition time; TE: echo time; GRE: gradient echo; FOV: field of view.

### 3-3. Quantitative analysis of image quality

The quantitative assessment for the degree of contrast enhancement in both lymph nodes and lymphatic vessels were performed using the signal-to-noise ratio (SNR) and contrast-to-noise ratio (CNR). The background region of interest (ROI) was drawn at an area where there were no anatomical structures in the beagles (FIG. 13). The SNR and CNR values were calculated using Image J (Bethesda, MD, USA; http://rsbweb.nih.gov/ij/) with the following equation by one reader:

$$SNR_{LN} = SI_{LN} / \text{Mean } SD_{background}$$

$$CNR_{LN} = (SI_{LN} - SI_{muscle}) / \text{Mean } SD_{background}$$

$$SNR_{LV} = SI_{LV} / \text{Mean } SD_{background}$$

$$CNR_{LV} = (SI_{LV} - SI_{muscle}) / \text{Mean } SD_{background}$$

where $SI_{LN}$, $SI_{LV}$, $SI_{muscle}$, and $SD_{background}$ indicate the signal intensities of the popliteal lymph node, lymphatic vessels, muscles, and standard deviation of background, respectively. In addition, the relaxation time of $T_1$, $T_2$, and $T_2$* in the acquired map images were calculated using MATLAB (R2016b; MathWorks, Natick, MA, USA).

### 3-4. Qualitative analysis of image quality

An image quality assessment with regards to the visualization of both lymph nodes and lymphatic vessels was performed independently by two radiologists to identify the optimal concentration and administration volume using a visual grading analysis based on the criteria of qualitative scores. The criteria used were 1) venous contamination, 2) dermal backflow, 3) lymph node congestion, and 4) score for the visualization of lymph nodes and lymphatic vessels. A detailed summary of the criteria for qualitative scores is presented in Table 4.

[Table 4]

| Description | Qualitative score | | | |
|---|---|---|---|---|
| | 0 | 1 | 2 | 3 |
| Venous contamination | Present | Absent | - | - |
| Dermal backflow | Present | Absent | - | - |
| Lymph node congestion | Present | Absent | - | - |
| Visualization | Poor | Moderate | Good | Excellent |

This scoring system (with 1 indicating none) was used such that higher scores reflected better image quality.

### 3-5. Statistical analysis

The interobserver agreements for qualitative assessment were evaluated using weighted kappa values. The strength of the interobserver agreement according to the weighted kappa value was assessed as follows: ≤0.2, poor; 0.21-0.40, fair; 0.41-0.60, moderate; 0.61-0.80, good; and 0.81-1.00, excellent [15,16]. the Kolmogorov-Smirnov test was used to confirm relaxation times following a normal distribution. The values of $T_1$, $T_2$, and $T_2$* relaxation time as a function of anatomical lesions were compared using a paired Student's *t*-test. Statistical analyses were performed using IBM SPSS Statistics for Windows/Macintosh, v. 26.0 (IBM Corp., Armonk, NY, USA). For all of the statistical analyses, a two-sided level of $p < 0.05$ was considered to indicate a statistically significant difference.

## [Results]

### 1. Dose finding study

The results of the qualitative score analysis of lymphatic vessels for the adaptive optimal dose-finding study at various dosing conditions of 0.028, 0.056, and 0.112 mg Fe/kg with different concentrations (7.5, 15, and 30 mM) are demonstrated in Table 5. In all tested conditions, venous contamination was not observed. The qualitative image analysis demonstrated that the doses of 0.028 mg Fe/kg with concentrations of 7.5, 15, and 30 mM showed a relatively low total score of 2.5, 4.5, and 3.5, respectively (FIG. 14a and Table 5). At a dose of 0.056 mg Fe/kg with concentrations of 15 mM and 30 mM, the total scores were 6 and 5, respectively. Since the higher total score was achieved at the concentration of 15 mM rather than 30 mM at same dose of 0.056 mg Fe/kg, a further higher dose of 0.112 mg Fe/kg at a concentration of 15 mM was tested, and a total score of 6 was achieved (FIG. 14b and Table 5). In the quantitative analysis, a dose condition of 0.056 and 0.112 mg Fe/kg at a concentration of 15 mM showed the best contrast enhancement of lymphatic vessels and nodes. Specifically, $SNR_{LN}$, $SNR_{LV}$, $CNR_{LN}$, and $CNR_{LV}$ showed the highest values of 295.62 ± 27.08, 233.14 ± 20.31, 216.22 ± 20.21, and 153.16 ± 13.61, respectively, at a dose of 0.112 mg Fe/kg with a concentration of 15 mM (FIG. 15). Based on these quantitative and qualitative analyses, the dose condition of 0.056 and 0.112 mg Fe/kg with a concentration of 15 mM were selected for the dose expansion study for the reproducibility assessment.

[Table 5]

| Order | Group | Beagle ID | Readers | Qualitative scoring | | | | |
|---|---|---|---|---|---|---|---|---|
| | | | | Venous contamination | Dermal backflow | Lymph node congestion | Visualization | Total |
| 1 | IN-V-001 7.5 mM 0.028 mg Fe/kg | #01 | 1 | 1.0 | 1.0 | 0.0 | 0.0 | 2.0 |
| | | | 2 | 1.0 | 1.0 | 1.0 | 0.0 | 3.0 |
| | | Average | | 1.0 | 1.0 | 0.5 | 0.0 | 2.5 |
| 2 | IN-V-001 15 mM 0.028 mg Fe/kg | #01 | 1 | 1.0 | 1.0 | 1.0 | 2.0 | 5.0 |
| | | | 2 | 1.0 | 1.0 | 1.0 | 1.0 | 4.0 |
| | | Average | | 1.0 | 1.0 | 1.0 | 1.5 | 4.5 |
| 3 | IN-V-001 30 mM 0.028 mg Fe/kg | #02 | 1 | 1.0 | 1.0 | 0.0 | 1.0 | 3.0 |
| | | | 2 | 1.0 | 1.0 | 1.0 | 1.0 | 4.0 |
| | | Average | | 1.0 | 1.0 | 1.0 | 1.0 | 3.5 |

(continued)

| Order | Group | Beagle ID | Readers | Qualitative scoring | | | | |
|---|---|---|---|---|---|---|---|---|
| | | | | Venous contamination | Dermal backflow | Lymph node congestion | Visualization | Total |
| 4 | IN-V-001 30 mM 0.056 mg Fe/kg | #03 | 1 | 1.0 | 1.0 | 1.0 | 2.0 | 5.0 |
| | | | 2 | 1.0 | 1.0 | 1.0 | 2.0 | 5.0 |
| | | Average | | 1.0 | 1.0 | 1.0 | 2.0 | 5.0 |
| 5 | IN-V-001 15 mM 0.056 mg Fe/kg | #02 | 1 | 1.0 | 1.0 | 1.0 | 3.0 | 6.0 |
| | | | 2 | 1.0 | 1.0 | 1.0 | 3.0 | 6.0 |
| | | Average | | 1.0 | 1.0 | 1.0 | 3.0 | 6.0 |
| 6 | IN-V-001 15 mM 0.112 mg Fe/kg | #02 | 1 | 1.0 | 1.0 | 1.0 | 3.0 | 6.0 |
| | | | 2 | 1.0 | 1.0 | 1.0 | 3.0 | 6.0 |
| | | Average | | 1.0 | 1.0 | 1.0 | 3.0 | 6.0 |

**2. Reproducibility study**

The results of the dose expansion study for the reproducibility evaluation are presented in Table 5. All acquired MRL imaging clearly demonstrated the visibility of lymphatic vessels and lymph nodes without venous contamination. In the qualitative analysis, the doses of 0.056 and 0.112 mg Fe/kg with a concentration of 15 mM received total scores of 5.33 and 5.66, respectively (Table 6). Overall, the qualitative analysis in both groups showed a good visualization for both lymphatic vessels and lymph nodes. No venous contamination and dermal backflow were observed for all tested conditions (FIG. 16). In the quantitative analysis, $SNR_{LN}$, $SNR_{LV}$, $CNR_{LN}$, and $CNR_{LV}$ exhibited the maximum values of 178.12 ± 32.84, 133.76 ± 30.33, 119.96 ± 43.40, and 83.94 ± 19.43 for the dose group of 0.056 mg Fe/kg and 257.34 ± 61.10, 195.44 ± 45.26, 199.37 ± 61.58, and 138.32 ± 48.03 for the dose group of 0.112 mg Fe/kg, respectively, at 30 min after the administration of INV-001 (FIG. 17). Furthermore, these values exhibited a gradual decrease until the 70-min mark, with a range of decrease rates between 7.3 and 22.8%, resulting in a continuous visualization of the lymphatic vessels and lymph nodes. Compared to the dose group of 0.056 mg Fe/kg, all tested results for the dose group of 0.112 mg Fe/kg showed a higher value in both lymphatic vessels and lymph nodes. These results indicate that INV-001 effectively enhanced lymphatic vessels and lymph nodes in a dose-dependent manner. Based on both quantitative and qualitative analyses, an administration dose of 0.056 and 0.112 mg Fe/kg with a concentration of 15 mM demonstrated good reproducibility in visualizing lymphatic vessels and lymph nodes without venous contamination. Therefore, it can be determined that an administration dose higher than 0.056 mg Fe/kg with a concentration of 15 mM INV-001 is sufficient for MRL in beagle dogs.

[Table 6]

| Group | Beagle ID | Readers | Qualitative scoring | | | | |
|---|---|---|---|---|---|---|---|
| | | | Venous contamina tion | Dermal backflow | Lymph node congestion | Visualization | Total |
| INV-001 | #01 | 1 | 1.0 | 1.0 | 0.0 | 1.0 | 3.0 |
| | | 2 | 1.0 | 1.0 | 1.0 | 2.0 | 5.0 |

(continued)

| Group | Beagle ID | Readers | Qualitative scoring | | | | |
|---|---|---|---|---|---|---|---|
| | | | Venous contamina tion | Dermal backflow | Lymph node congestion | Visualization | Total |
| 15 mM 0.056 mg Fe/kg | #02 | 1 | 1.0 | 1.0 | 1.0 | 3.0 | 6.0 |
| | | 2 | 1.0 | 1.0 | 1.0 | 3.0 | 6.0 |
| | #03 | 1 | 1.0 | 1.0 | 1.0 | 3.0 | 6.0 |
| | | 2 | 1.0 | 1.0 | 1.0 | 3.0 | 6.0 |
| Average | | | 1.0 | 1.0 | 1.0 | 2.5 | 5.33 |
| IN-V-001 15 mM 0.112 mg Fe/kg | #01 | 1 | 1.0 | 1.0 | 1.0 | 2.0 | 5.0 |
| | | 2 | 1.0 | 1.0 | 1.0 | 2.0 | 5.0 |
| | #02 | 1 | 1.0 | 1.0 | 1.0 | 3.0 | 6.0 |
| | | 2 | 1.0 | 1.0 | 1.0 | 3.0 | 6.0 |
| | #03 | 1 | 1.0 | 1.0 | 1.0 | 3.0 | 6.0 |
| | | 2 | 1.0 | 1.0 | 1.0 | 3.0 | 6.0 |
| Average | | | 1.0 | 1.0 | 1.0 | 2.66 | 5.66 |

**3. Excretion study**

To confirm the excretion of INV-001, $T_1$, $T_2$, and $T_2$* relaxation time of the liver and kidneys were analyzed at 0 (pre-injection) and 48 h after administration (Table 7). INV-001 was administered at a dose of 0.056 mg Fe/kg with a concentration of 15 mM. Compared to pre-injection (0 hour), there were no statistically significant differences after 48 h for $T_1$, $T_2$, and $T_2$* relaxation time measured at both the liver and kidneys ($p > 0.05$), except for $T_2$* relaxation time of the liver ($p < 0.05$) (FIG. 20). Additionally, the INV-001 did not remain in the injection site(FIG. 21). Therefore, it is considered that the INV-001 was effectively excreted from the body within 48 h.

[Table 7]

**Supplementary Table 1.** A detailed summary of the scan parameters for calculating relaxation time

| | **$T_1$ relaxation time** | **$T_2$ relaxation time** | **$T_2$* relaxation time** |
|---|---|---|---|
| FOV | 300 × 244 | 300 × 244 | 300 × 244 |
| Slice thickness/gap (mm) | 7 / 0.7 | 7 / 0.7 | 7 / 0.7 |
| TR (ms) | 20/40/60/80/120/200 | 2400 | 300 |
| TE (ms) | 6.6 | 13.8 / 27.6 / 41.4 / 55.2 / 69 / 82.8 / 96.6 / 110.4 | 2.46 / 4.92 / 7.38 / 9.84 / 12.3 / 14.76 / 17.22 |
| Average | 1 | 1 | 1 |
| No. of slices | 20 | 15 | 20 |
| Scan orientation | Axial | Axial | Axial |
| Acquisition matrix | 256 × 187 | 320 × 208 | 256 × 166 |

Note: FOV: field of view. TR: repetition time. TE: echo time. $T_1$, $T_2$, and $T_2$* relaxation time images were acquired using variable TR. turbo spin echo with multi-echo, and gradient with multi-echo sequence, respectively.

**Example 4**

**4-1. Synthesis of Nanostructures for Intra-articular Injection**

Dextran (180 μmol, average molecular weight 10,000 Da) was dissolved in 9 mL of distilled water, followed by the addition of 75 mmol of epichlorohydrin and 75 mmol of NaOH. Subsequently, 380 mmol of ethylenediamine was added, and the mixture was stirred at room temperature (RT) for 24 hours. After the succinylation reaction was carried out at RT for an additional 24 hours, the dextran core was purified by dialysis using a 15 kDa molecular weight cutoff (MWCO) filter. An

excess amount of ferric chloride hexahydrate solution was added to the dextran core solution. The pH was adjusted to 8 using 1 M NaOH, and the mixture was reacted at room temperature for 1 hour. After ultrafiltration purification, a nanostructure (code: INV-002) was obtained. The iron concentration at maximum formulation was 1.6 mg/mL, and the viscosity at this concentration was 7.1 cP (FIG. 8).

The nanostructures synthesized in Examples 2-1 and 4-1 were observed visually as transparent yellow to yellow-brown solutions. The iron content and dextran content were analyzed using inductively coupled plasma (ICP) and the phenol-sulfuric acid method, respectively, and the weight ratio was determined to be approximately 3:100. The number of crosslinker-derived functional groups was confirmed by o-phthalaldehyde assay and elemental analysis after the crosslinking reaction. Based on these analyses, the proportion of monosaccharide units bound to crosslinkers relative to the total monosaccharide units in the polysaccharide-crosslinked colloidal particles was controlled in the range of 2% to 60%. The crosslinker substitution ratio was between 10% and 50% of the available dextran functional groups, and among these, 20% to 50% were found to remain unreacted and exposed at the surface. Even after chelation with iron ions, o-phthalaldehyde assay results indicated the continued presence of crosslinker-derived functional groups, suggesting that while some crosslinkers were chelated with iron ions, others remained in a non-chelated, exposed form. According to dynamic light scattering (DLS) analysis, the synthesized nanostructures exhibited a uniform size distribution with a hydrodynamic diameter of less than 8 nm, which is below the renal filtration cutoff. The surface charge () was measured in the range of -20 mV to 0 mV. Gel permeation chromatography (GPC) analysis showed that the average molecular weight was approximately 15 kDa for INV-001 and approximately 30 kDa for INV-002. Considering that the nanostructures were synthesized from dextran precursors with average molecular weights of 5,000 Da and 10,000 Da, respectively, this suggests that 2 to 3 dextran chains were crosslinked to form each nanostructure.

### 4-2. T1 MRI Contrast Effect of the Intra-Articularly Administered Nanostructure INV-002

To evaluate whether INV-002 synthesized in Example 4-1 can be used as a T1 MRI contrast agent for magnetic resonance arthrography (MR arthrography), a test was conducted to determine whether the T1 MRI contrast effect is retained even after mixing with an iodinated X-ray contrast agent.

For the test, a mixture of INV-002 from Example 4-1 and Iopamidol, a representative iodinated X-ray contrast agent, was prepared. The INV-002-Iopamidol mixture exhibited a stronger T1 MRI contrast effect than INV-002 alone at the same concentration. For example, when INV-002 and Iopamidol were mixed in a 1:1 ratio, the resulting mixture showed approximately twice the T1 MRI contrast effect compared to INV-002 alone at the same iron concentration. These results demonstrate that INV-002 from Example 4-1 retains its strong T1 MRI contrast-enhancing properties even when mixed with an iodinated X-ray contrast agent, confirming its potential utility as an effective T1 MRI contrast agent for magnetic resonance arthrography.

### 4-3. Magnetic Resonance Arthrography Using the Intra-Articularly Administered Nanostructure INV-002

Male Sprague-Dawley rats aged 7 weeks or older were anesthetized using a mixture of oxygen and isoflurane gas. INV-002 synthesized in Example 4-1 was then administered into the knee joint, and T1-weighted MRI images were acquired using a fast spin echo sequence.

As shown in FIGS. 22A-22D, the knee joint cavity of animals injected with INV-002 appeared significantly brighter in the MRI images compared to the pre-injection state. This increase in signal intensity was also confirmed quantitatively, as shown in FIG. 22E. For example, the T1 signal intensity of the synovial fluid increased from 4,900 (before injection) to 21,000 (after injection). Subsequently, the contrast-to-noise ratios (CNRs) of various anatomical regions within the joint cavity- including the meniscus, joint capsule, cruciate ligament, bone, and fat - were analyzed (FIG. 22F). The CNR was calculated as the difference in average signal intensity between the synovial fluid and each anatomical structure, divided by the standard deviation of the background signal. After INV-002 injection, the CNRs for the meniscus, capsule, cruciate ligament, bone, and fat regions increased by 10-fold, 8.5-fold, 18-fold, 14-fold, 12-fold, and 4-fold, respectively, compared to pre-injection levels.

The contrast effect of INV-002 was not only stronger than that of the gadolinium-based contrast agent (e.g., Dotarem) but also lasted longer. For quantitative analysis of contrast enhancement, the signal-to-noise ratio (SNR) was measured at two intra-articular locations (indicated by orange arrows), and the average SNR was plotted over time. Both INV-002 and Dotarem exhibited peak SNR values at 0.25 hours post-injection, with INV-002 showing approximately twice the SNR of Dotarem at that time point (FIGS. 23A and 23B). While the SNR of Dotarem dropped to baseline within 0.5 hours post-injection, INV-002 maintained a significant SNR for up to 6 hours, with contrast enhancement no longer observed after 9 hours (FIG. 23C).

These results indicate that INV-002 from Example 4 exhibits effective intra-articular contrast enhancement, enabling clearer visualization of intra-articular anatomical structures. Furthermore, the longer-lasting contrast effect of INV-002 compared to Dotarem is expected to offer practical advantages for high-resolution imaging, repeated scanning,

or re-imaging in case of initial scan failure (FIG. 23).

### 4-4. Excretion of the Intra-Articularly Administered Nanostructure INV-002

As shown in the results of Example 4-3, no contrast enhancement was observed in the knee joint cavity of rats starting from 9 hours post-injection of INV-002, indicating that INV-002 was absorbed and eliminated from the joint cavity within 9 hours. To more accurately analyze the excretion pathway, INV-002 was administered into the knee joint cavity of Sprague-Dawley rats aged 7 weeks or older, and urine was collected thereafter. Urine samples were collected at 0-24 hours, 24-48 hours, and 48-72 hours post-injection, and the iron content in each sample was quantified using inductively coupled plasma (ICP) analysis. A control group received a saline injection, and urine was collected and analyzed in the same manner and at the same time points.

As shown in FIG. 24A, the iron content in the urine of both male and female rats injected with INV-002 was statistically significantly higher than that in the saline-injected control group. In particular, the total amount of iron detected in urine was approximately 0.018 mg, which is within the margin of error compared to the initially administered dose, indicating that most of the INV-002 injected into the joint cavity was excreted via urine within 24 hours. As shown in FIG. 24B, the urine samples collected after INV-002 administration appeared visibly darker brown in color compared to the saline-injected control group, attributable to the inherent color of INV-002.

To confirm the complete elimination of INV-002 from the joint cavity, histological analysis was performed using Perl's Prussian blue iron staining, a method capable of detecting trace amounts of iron. Joint tissues from the INV-002-injected animals were harvested, fixed in 10% neutral buffered formalin, subjected to Perl's Prussian blue iron staining, and examined under a digital microscope. As shown in FIG. 25, no iron accumulation was observed in either the INV-002-injected group (FIG. 25A) or the saline-injected control group (FIG. 25B).

These results indicate that INV-002, when administered into the joint cavity, is excreted via urine without accumulation in the joint tissue.

### 4-5. Contrast Enhancement and Clearance of the Nanostructure INV-002 Administered into the Intrathecal Space

Following the results of Example 4-3, which confirmed that INV-002 administered into the joint cavity was eliminated from the cavity, a subsequent experiment was conducted to determine whether INV-002 would also exhibit contrast enhancement and be cleared when administered into the intrathecal space. Sprague-Dawley rats aged 7 weeks or older were anesthetized using a mixture of oxygen and isoflurane, and INV-002 prepared according to Example 4-1 was administered into the intrathecal space. T1-weighted MRI images were acquired using a fast spin echo sequence.

As shown in FIG. 26, the intrathecal space of the animals administered with INV-002 appeared as a bright signal in the T1-weighted MRI images. Compared to the pre-injection state, the brightness of the intrathecal space increased immediately after injection, reaching a peak at 30 and 60 minutes post-injection. By 90 minutes post-injection, the brightness began to diminish, and by 120 minutes, it had returned to a level comparable to the pre-injection state.

These findings indicate that the nanostructure INV-002 administered into the intrathecal space is gradually cleared from the space over time.

### 4-6. Single-Dose Safety Assessment of the Intra-Articularly Administered Nanostructure INV-002

To evaluate the local intra-articular toxicity of INV-002, a single-dose injection of INV-002 was administered into the joint cavities of Sprague-Dawley rats and Beagle dogs, followed by a two-week observation period. The concentration of INV-002 used in the local toxicity test was 1.6 mg Fe/mL, which corresponds to the highest concentration achievable as shown in FIG. 8 and is approximately 11 times higher than the anticipated clinical formulation concentration of 0.14 mg Fe/mL. The maximum injectable volume for the joint cavity was used-0.1 mL for rats and 1 mL for Beagle dogs-resulting in doses of 0.16 mg Fe/head and 1.6 mg Fe/head, respectively.

No abnormal symptoms were observed, and as shown in FIG. 27, there were no differences in body weight trends between the INV-002-treated group and the untreated control group. Accordingly, the NOAEL (No Observed Adverse Effect Level) was determined to be 0.160 mg Fe/head for rats and 1.60 mg Fe/head for Beagle dogs, which represent 23-fold and 6-fold the anticipated clinical dose, respectively, thereby demonstrating excellent safety.

### 4-7. Single-Dose Blood Chemistry and Histopathological Evaluation of the Intra-Articularly Administered Nanostructure INV-002

Since INV-002 of Example 4-1 was found to be absorbed into the circulatory system and excreted via the renal elimination pathway, blood chemistry analysis was conducted to evaluate liver function (alanine transaminase, ALT;

aspartate transaminase, AST; alkaline phosphatase, ALP; gamma-glutamyltransferase, GGT) and kidney function (blood urea nitrogen, BUN; creatinine, CR). For blood chemistry analysis, blood was collected from the abdominal aorta, and serum was separated via centrifugation. The levels of ALT, AST, ALP, GGT, BUN, and CR were analyzed using an automated biochemical analyzer.

For histopathological evaluation, organs were fixed in 10% neutral buffered formalin and processed for hematoxylin and eosin (H&E) staining according to the manufacturer's protocol. Tissue sections were counterstained with eosin for 1 minute and analyzed under a digital microscope.

As shown in FIG. 28, all parameters in the blood chemistry analysis were within the normal range, and no pathological abnormalities or lesions were observed in the histopathological results, indicating that INV-002 possesses excellent in vivo biocompatibility.

No abnormal changes in cellular structures (e.g., collapse, distortion, or dilation) were observed.

None of the organs exhibited hemorrhage, inflammation, or necrosis, indicating the non-toxicity of INV-002. Blood chemistry results confirmed normal renal and hepatic function in the rats injected with INV-002.

## Example 5: Shoulder MR Arthrography Using an Iron-based positive T1 MRI Contrast Agent NEMO-103

### 5-1. Preparation of the Dextran-Crosslinked T1 MRI Contrast Agent NEMO-103

Dextran-crosslinked nanoparticles were synthesized by mixing an aqueous solution of 20 mM dextran T10 with sodium hydroxide solution and epichlorohydrin, followed by the addition of ethylenediamine. The resulting dextran-crosslinked nanoparticles were purified using a 10 kDa molecular weight cutoff (MWCO) filter. The terminal amine groups of the dextran-crosslinked nanoparticles were then substituted with carboxyl groups via reaction with succinic anhydride. After further purification using a 10 kDa MWCO filter, the carboxylated dextran-crosslinked nanoparticles were reacted with ferric chloride solution for 1 hour and subsequently purified with a 10 kDa MWCO filter to yield the dextran-crosslinked T1 MRI contrast agent NEMO-103 (FIG. 29a).

### 5-2. *Phantom study*

In a phantom study using a 3T MRI scanner, NEMO-103 exhibited bright T1 signal intensity on T1-weighted images, comparable to that of Dotarem® (FIGS. 29b and 29c). The measured $r_1$ and $r_2$ relaxivity values of NEMO-103 were 2.0 $mM^{-1}s^{-1}$ and 2.3 $mM^{-1}s^{-1}$, respectively (FIGS. 29d and 29e). The $r_2/r_1$ ratio, which is an important parameter for T1 MRI contrast agents, was calculated to be 1.15. Given that an ideal T1 contrast agent exhibits an $r_2/r_1$ ratio close to 1, the observed ratio of 1.15 indicates that NEMO-103 is a highly suitable T1 MRI contrast agent. For comparison, the measured $r_1$ and $r_2$ values of Dotarem® were 4.7 $mM^{-1}s^{-1}$ and 5.2 $mM^{-1}s^{-1}$, respectively, resulting in an $r_2/r_1$ ratio of 1.11.

### 5-3. Image Quality Assessment

FIGS. 30 and 31 illustrate the phase design and image quality assessment method of a clinical comparative study between NEMO-103, a dextran-crosslinked T1 MRI contrast agent, and Dotarem®, a representative clinical gadolinium-based contrast agent (GBCA) for T1 MRI. Phase I refers to MR arthrography (MRA) images acquired within 30 minutes after contrast agent administration. Phase II refers to MRA images acquired between 30 and 60 minutes post-administration, and Phase III refers to MRA images acquired between 110 and 130 minutes post-administration. In Comparison 1, no quantitative or qualitative differences were observed between NEMO-103-based MRA images acquired during Phase I and Phase II (FIG. 32). In Comparison 2, when comparing NEMO-103-based MRA images from Phase I with GBCA-based MRA images, no difference was observed in contrast-to-noise ratio (CNR); however, statistically significant differences were found in capsular distension ($p < 0.05$, FIG. 33). Specifically, NEMO-103-based MRA images showed higher scores in overall image quality. Although no significant difference in posterior capsular distension was observed between the two groups, NEMO-103-based MRA images showed superior distension in both the inferior capsule and the axillary pouch. In Comparison 3, which compared NEMO-103-based MRA images from Phase II with GBCA-based MRA images, more prominent differences were observed (FIG. 34). The CNR was significantly higher in the NEMO-103 group than in the GBCA group. In addition, more pronounced differences in inferior capsular and axillary pouch distension were observed. As a result, NEMO-103-based MRA images achieved superior outcomes in both inferior capsular and axillary pouch distension.

### 5-4. Visual Turing Test (VTT)

In the VTT for Comparison 1, radiologists were instructed to distinguish between NEMO-103-based MRA images obtained in Phase I and Phase II. The combined accuracy of the 8 testers was 46.8% (146/312), showing no statistically

significant difference compared to random guessing (50.0%, 156/312; $p = 0.423$). In the VTT for Comparison 2, radiologists were instructed to differentiate between NEMO-103-based and GBCA-based MRA images acquired in Phase I. The combined accuracy of the 32 testers averaged 53.3% (624/1170), with no statistically significant difference compared to random guessing (50.0%, 585/1170; $p = 0.107$).

[Discussion]

Amid growing safety concerns surrounding gadolinium-based contrast agents (GBCAs), iron (Fe)-based alternatives have emerged as a subject of active investigation. The present example evaluates the efficacy of NEMO-103 in comparison with a GBCA by assessing contrast-to-noise ratio (CNR) and MR arthrography (MRA) image quality. In direct intra-articular shoulder MRA, NEMO-103 (0.035 mg Fe/kg) and GBCA (0.037 mg Gd/kg) were each administered via direct joint injection, and the resulting CNR and visual Turing test (VTT) outcomes at 30 minutes post-injection (Comparison 2) demonstrated nearly equivalent image quality. Both contrast agents effectively delineated key anatomical structures, including tissue contrast, sharpness, rotator cuff tendons, labral structures, and adjacent cartilage. Furthermore, NEMO-103 maintained an enhanced CNR even at 120 minutes post-injection.

According to this example, the MRA image quality at 30 minutes (Phase I) and 60 minutes (Phase II) post-injection could not be distinguished in the visual Turing test, suggesting the potential extension of the imaging time window following injection. This may be advantageous for improving the operational efficiency of MR imaging facilities in clinical settings. According to current reports, particulate contrast agents with a hydrodynamic diameter of 3 nm are excreted from the joint cavity solely via lymphatic vessels due to their larger size and are not cleared through the venous system. Given that NEMO-103 has a larger hydrodynamic size (approximately 4.0 nm), it can only be excreted via lymphatic drainage, leading to prolonged retention within the joint cavity. In contrast, GBCAs (<1 nm) are rapidly cleared through both lymphatic and venous routes, thus limiting the time window available for image acquisition. However, in the MRA images taken 24 hours after administration, complete clearance of NEMO-103 from the joint cavity was confirmed, and no residual contrast agent was detected.

On the other hand, there was no statistically significant difference in posterior capsular distension between the MRA images based on NEMO-103 and those based on GBCA. This is presumed to be attributable to the patient's positioning during shoulder MRA. In the supine position, joint fluid tends to shift posteriorly, resulting in relatively less residual fluid in anatomical structures such as the undersurface of the subscapularis tendon and the axillary pouch. Over time, as the contrast agent is resorbed, this difference becomes more pronounced in the case of GBCA. Consequently, GBCA exhibited a relatively higher incidence of axillary pouch obliteration compared to NEMO-103. Moreover, the anterior aspect of the glenohumeral joint includes critical structures such as the subscapularis tendon and the anteroinferior glenoid labrum. Since multiple structures must be evaluated in this region, resorption of joint fluid and positional shifts caused by the supine posture may affect the conspicuity of lesions in this anterior compartment.

Unlike the long-established use of GBCAs, the novel contrast agent NEMO-103 requires safety evaluation. According to the clinical study report, treatment-emergent adverse events (TEAEs) were observed in 3 out of 32 subjects (9.4%) who underwent NEMO-103-based MRA, including two cases of localized injection-site reactions (muscle pain) and one case of hypertension. These adverse events were considered "unrelated to the investigational drug" in terms of causality, and all subjects recovered without medical intervention. Furthermore, no drug-related adverse events (ADRs), serious adverse events (SAEs), or severe adverse events occurred, and no subject discontinued participation due to these events. Evaluation of residual NEMO-103 in the liver and spleen at 24 hours post-injection confirmed complete clearance in all 30 subjects (100%) who underwent delayed imaging. The relative signal intensity (rSI) differences between the pre-contrast abdominal MRI and the 24-hour delayed MRI were all positive (liver: $13.2 \pm 4.0$; spleen: $5.5 \pm 0.9$), indicating elimination of the contrast agent. Two subjects did not undergo 24-hour delayed imaging that included liver and spleen evaluation.

Another advantage of NEMO-103 lies in its potential to eliminate reliance on conventional off-label use. Traditional GBCA-based MR arthrography requires a laborious dilution process of approximately 1:200, which increases the risk of contamination and infection, while also hindering standardization of contrast agent concentration. In contrast, NEMO-103 is supplied as a vial formulation pre-diluted to a concentration suitable for MR arthrography, thereby offering relative freedom from issues such as infection, contamination, and heterogeneity in contrast concentration.

As a retrospective study involving a limited number of patients, the present investigation has several limitations. First, since the subjects were not patients who underwent arthroscopic surgery, the diagnostic accuracy of NEMO-103-based shoulder MRA and GBCA-based shoulder MRA for individual lesions could not be evaluated with reference to surgical findings. However, by emphasizing the role of the contrast agent within the joint cavity-such as enhancement of contrast between surrounding structures and distension of the joint space-meaningful differences over time between MRA images using NEMO-103 and those using GBCA were observed. In addition, through a visual Turing test (VTT) involving a large number of testers, NEMO-103 demonstrated image quality comparable to that of GBCA from various perspectives. Future clinical trials may be designed to involve patients requiring surgical intervention, using operative records as the gold

standard to evaluate the diagnostic accuracy of both contrast agents. Second, this study did not assess both contrast agents in the same subject. Individual variability may exist in joint cavity distension and contrast agent resorption. However, performing two MRAs using different contrast agents within a short time interval in the same patient presents a substantial challenge in study design. To overcome this limitation, the time interval between contrast agent injection and image acquisition was carefully reviewed, and phase matching was applied for comparative analysis. Third, it is noteworthy that the GBCA group was significantly older than the NEMO-103 group. Although not definitively proven, potential age-related differences in contrast agent resorption rates may exist. Nevertheless, considering that synovial fluid volume tends to increase with more severe degeneration and symptoms such as pain, it is plausible that the GBCA group, consisting of actual patients, had a greater synovial fluid volume than the relatively younger, volunteer-based NEMO-103 group.

In conclusion, NEMO-103-based MR arthrography of the shoulder is, at minimum, interchangeable with GBCA-based MR arthrography in terms of contrast-to-noise ratio (CNR), joint distension, image quality, and VTT performance. Moreover, it demonstrated enhanced resistance to contrast agent resorption over time, suggesting a potential extension of the feasible imaging time window.

## Example 6

### 6-1. Synthesis of C-DNP-3 Using 3 kDa Dextran

Dextran (molecular weight 3 kDa, 1 g) was dissolved in 4.2 mL of distilled water. To this solution, 8.3 mL of NaOH solution was added, followed by the addition of 3.3 mL of epichlorohydrin under stirring. Subsequently, 7 mL of diethylenetriamine (DETA) was added, and the reaction mixture was stirred for an additional 24 hours to yield C-DNP-3, a 3 kDa dextran-based crosslinked nanoparticle. The resulting product was purified by ultrafiltration. The hydrodynamic size, measured by dynamic light scattering (DLS), was 3 nm.

### 6-2. Synthesis of C-DNP-5 Using 5 kDa Dextran

Except for replacing 3 kDa dextran with 5 kDa dextran, the synthesis was carried out under the same procedure as in Example 6-1, yielding C-DNP-5, a 5 kDa dextran-based crosslinked nanoparticle. The product was purified by ultrafiltration. The hydrodynamic size measured by DLS was 4 nm.

### 6-3. Synthesis of C-DNP-10 Using 10 kDa Dextran

Except for replacing 3 kDa dextran with 10 kDa dextran, the synthesis was carried out under the same procedure as in Example 6-1, yielding C-DNP-10, a 10 kDa dextran-based crosslinked nanoparticle. The product was purified by ultrafiltration. The hydrodynamic size measured by DLS was 5 nm.

### 6-4. Synthesis of C-CMDNP-10 Using 10 kDa Carboxymethyl Dextran (CM-Dextran)

Except for replacing 3 kDa dextran with 10 kDa CM-dextran, the synthesis was carried out under the same procedure as in Example 6-1, yielding C-CMDNP-10, a 10 kDa CM-dextran-based crosslinked nanoparticle. The product was purified by ultrafiltration. The hydrodynamic size measured by DLS was 5 nm.

### 6-5. Substitution of Surface Functional Groups on C-DNP or C-CMDNP

#### 6-5-1. Amine Group

The surface functional group of C-DNP or C-CMDNP synthesized in Examples 6-1 through 6-4 is an amine group.

#### 6-5-2. Carboxyl Group

To 10 mL of C-DNP or C-CMDNP prepared in Examples 6-1 through 6-4, 30 mg of succinyl anhydride (SA) was added. The reaction mixture was stirred for 12 hours. The final product was purified by ultrafiltration.

#### 6-5-3. Thiol Group

To 10 mL of C-DNP or C-CMDNP prepared in Examples 6-1 through 6-4, N-succinimidyl S-acetylthioacetate was added. The reaction mixture was stirred for 12 hours. The final product was purified by ultrafiltration.

### 6-5-4. Hydroxyl Group

To 10 mL of C-DNP or C-CMDNP synthesized in Examples 6-1 through 6-4, nitrous acid was added. The reaction mixture was stirred for 12 hours. The final product was purified by ultrafiltration.

### 6-6. Surface Charge Modulation of C-DNP and C-CMDNP

In 10 mL of C-DNP synthesized in Examples 6-1 through 6-3, when 30 mg of succinyl anhydride (SA) was added according to the procedure of Example 6-5-2, the measured surface charge was -3 mV. When 50 mg of SA was added, the surface charge was -20 mV. In the absence of added SA, the surface charge was +5 mV. Experiments conducted using C-DNP-3, C-DNP-5, and C-DNP-10 showed consistent results under the same substitution conditions, with respect to the amount of SA added and the resulting surface charge. In the case of 10 mL of C-CMDNP-10 synthesized in Example 6-4, which initially exhibited a surface charge of +5 mV, the addition of 30 mg of SA according to Example 6-5-2 yielded a surface charge of -4 mV. Representative results showing the change in surface charge of C-DNP-10 depending on the amount of SA added are presented in FIG. 35. These results demonstrate that increasing the amount of added SA leads to a more negative surface charge, thereby enabling tunable modulation of the surface potential of the colloidal particles.

### 6-7. Surface Coating of Inorganic Nanoparticles Using C-DNP/C-CMDNP

10 mg of 4 nm iron oxide nanoparticles (see FIGS. 36a and 36b) were dispersed in tetramethylammonium hydroxide (TMAOH) and stirred. The supernatant was removed by magnetic decantation, followed by two washes with 10 mL each of hexane and acetone. The washed iron oxide nanoparticles were then mixed with 100 mg of C-DNP-3, C-DNP-5, C-DNP-10, and C-CMDNP-10, each substituted with carboxyl groups according to Example 6-5-2, and stirred for 12 hours. The resulting C-DNP/C-CMDNP-coated iron oxide nanoparticles (IONP@C-DNP-3, IONP@C-DNP-5, IONP@C-DNP-10, IONP@C-CMDNP-10) were purified via ultrafiltration.

### 6-8. Hydrodynamic Size of Inorganic Nanoparticles Coated with C-DNP/C-CMDNP

The hydrodynamic sizes of IONP@C-DNP-3, IONP@C-DNP-5, and IONP@C-DNP-10 prepared in Example 6-7 were 7 nm, 9 nm, and 11 nm, respectively (see FIG. 36c). Because the carboxymethyl (CM) group is small (angstrom scale) and does not significantly affect the overall particle size (nanometer scale), the hydrodynamic size of IONP@C-CMDNP-10 prepared in Example 6-7 was also 11 nm (see FIG. 36d).

### 6-9. Surface Charge of Nanoparticles Coated with C-DNP Having Various Surface Charges

Using the C-DNP-3, C-DNP-5, and C-DNP-10 prepared in Example 6-7 with surface charges adjusted to -20 mV, -3 mV, and +5 mV at pH 7, the 4 nm iron oxide nanoparticles were surface-coated in accordance with Example 6-7. The surface charges of the coated nanoparticles were -20 mV when coated with -20 mV C-DNP, -3 mV when coated with -3 mV C-DNP, and +5 mV when coated with +5 mV C-DNP. This result indicates that the surface of the iron oxide nanoparticles was uniformly coated with C-DNP, as the resulting surface charges matched those of the C-DNP regardless of the original surface charge of the nanoparticles.

Representative data showing the surface charges of iron oxide nanoparticles coated with -20 mV, -3 mV, and +5 mV C-DNP-10 are provided in FIG. 37. Thus, the surface charge of coated nanoparticles can be controlled using C-DNPs with tunable surface charges.

### 6-10. Evaluation of Colloidal Stability of Iron Oxide Nanoparticles Coated with C-DNP and C-CMDNP

Solutions containing salts (0, 0.15, 0.5, and 1.0 M NaCl) and pH values (5, 7, and 8), mimicking physiological conditions, were prepared. To each solution, 0.1 mg of iron oxide nanoparticles coated with C-DNP-10 or C-CMDNP-10 prepared in Example 6-7 (IONP@C-DNP-10, IONP@C-CMDNP-10) was added. Colloidal stability was monitored over 28 days. As shown in FIG. 38a, IONP@C-DNP-10 remained dispersed without aggregation or precipitation under all tested physiological conditions. In addition, as shown in FIG. 38b, no changes in hydrodynamic size were observed over the monitoring period, indicating excellent colloidal stability. Similarly, IONP@C-CMDNP-10 also showed excellent colloidal stability without precipitation, as illustrated in FIG. 39a.

### 6-11. Surface Coating of Inorganic Nanoparticles Using Non-crosslinked Native Dextran

10 mg of 4 nm iron oxide nanoparticles were dispersed in tetramethylammonium hydroxide (TMAOH) and stirred.

The supernatant was removed by magnetic decantation, and the nanoparticles were washed twice with 10 mL of hexane and acetone, respectively. The washed iron oxide nanoparticles were mixed with 50, 100, 500, 1000, or 2000 mg of non-crosslinked native dextran (molecular weight: 10 kDa) and stirred for 12 hours. The dextran-coated nanoparticles were purified by ultrafiltration. The hydrodynamic sizes of the dextran-coated iron oxide nanoparticles were 160 nm, 85 nm, 49 nm, 26 nm, and 18 nm, respectively. As shown in FIG. 39b, the minimum hydrodynamic size of iron oxide nanoparticles achievable using native dextran (10 kDa) was 18 nm, requiring a minimum of 2000 mg of dextran. This is 20 times the amount of C-DNP-10 (100 mg) required for coating. In other words, when using non-crosslinked native dextran, 20 times more material is needed compared to C-DNP-10 to achieve colloidal stability of iron oxide nanoparticles. Furthermore, as shown in FIG. 39a, iron oxide nanoparticles coated with 100 mg of C-DNP-10 did not show precipitation during colloidal stability tests, whereas those coated with 100 mg of non-crosslinked native dextran showed precipitation within 7 days under physiological conditions. Thus, C-DNP enables coating with 1/20 the amount of dextran while ensuring superior colloidal stability.

This effect is attributed to the compact spherical structure of the dextran-crosslinked nanoparticles of the present invention, the strong coordination bonding between the crosslinker-derived functional groups (e.g., carboxyl or amine groups) and the iron oxide nanoparticle surface, and their excellent colloidal stability.

### 6-12. Surface Coating of Inorganic Nanoparticles Using Non-Crosslinked CM Dextran

10 mg of 4 nm iron oxide nanoparticles were added to tetramethylammonium hydroxide (TMAOH) and stirred. The supernatant was removed by magnetic decantation, and the nanoparticles were washed twice with 10 mL of hexane and acetone, respectively. The washed iron oxide nanoparticles were mixed with 2000 mg of non-crosslinked carboxymethyl dextran (CM dextran, molecular weight: 10 kDa) and stirred for 12 hours. The CM dextran-coated nanoparticles were purified by ultrafiltration. The hydrodynamic size of the CM dextran-coated iron oxide nanoparticles was 18 nm. Additionally, as shown in FIG. 39a, iron oxide nanoparticles coated with 100 mg of C-CMDNP-10 did not show precipitation during colloidal stability tests, whereas those coated with 100 mg of non-crosslinked CM dextran showed precipitation within 7 days under physiological conditions.

### 6-13. Anti-opsonization Effect of C-DNP-coated Iron Oxide Nanoparticles

Magnetic nanoparticles coated with C-DNP-5 adjusted to surface charges of -20, - 3, and +5 mV, as prepared in Example 6-11, were mixed with fetal bovine serum (FBS) and incubated for 10 minutes. The mixtures were loaded onto 1% agarose gel and subjected to electrophoresis, and the migration of nanoparticles was visualized (FIG. 40). FIG. 40 compares the anti-opsonization effect of serum protein binding by evaluating band migration and broadening based on the surface charge of C-DNP-5-coated magnetic nanoparticles. Magnetic nanoparticles coated with C-DNP-5 with a surface charge of +5 mV migrated in the direction of the negative electrode during electrophoresis (FIG. 40a). However, when mixed with serum proteins, nonspecific adsorption increased the hydrodynamic size and caused surface charge randomization, leading to band broadening and altered migration direction (FIG. 40a). Nanoparticles coated with C-DNP-5 with a surface charge of -3 mV migrated slightly toward the positive electrode (FIG. 40b), and when mixed with serum proteins, no band broadening was observed, indicating that protein binding was inhibited and migration direction remained the same (FIG. 40b). Nanoparticles coated with C-DNP-5 with a surface charge of -20 mV showed significant migration toward the positive electrode (FIG. 40c), and mixing with serum proteins caused nonspecific binding, increasing the hydrodynamic size and resulting in band broadening (FIG. 40c).

Thus, as demonstrated in FIG. 40, the electrophoretic profiles of C-DNP-coated iron oxide nanoparticles confirm their anti-opsonization effect depending on surface charge modulation.

### 6-14. Pharmacokinetic Analysis of C-DNP-coated Iron Oxide Nanoparticles by Tuning Particle Size and Surface Charge

As illustrated in FIG. 41, 4 nm iron oxide nanoparticles were coated with various C-DNPs and administered to animals, followed by time-dependent observation of MRI signal changes. Since 4 nm iron oxide exhibits both T1 and T2 contrast enhancement modes in MRI, these dual imaging modes were utilized for pharmacokinetic profiling.

An iron oxide nanoparticle coated with C-DNP-3 having a surface charge of -3 mV and hydrodynamic size of 3 nm exhibited a total hydrodynamic size of 7 nm. Because this hydrodynamic size is less than or equal to 8 nm and the surface charge is close to neutral (0 mV), the nanoparticle is not subject to phagocytosis by macrophages and can be excreted renally when administered intravenously. Accordingly, renal excretion of the C-DNP-3-coated iron oxide nanoparticle was confirmed in mice using T1-weighted MRI imaging (FIG. 41a). An iron oxide nanoparticle coated with C-DNP-10 having a surface charge of -3 mV and hydrodynamic size of 5 nm exhibited a total hydrodynamic size of 11 nm. Since its hydrodynamic size exceeds 8 nm and its surface charge is close to neutral, it is not readily phagocytosed and remains

in the bloodstream for an extended period, making it suitable for prolonged vascular imaging (FIG. 41b). Therefore, even 30 minutes after intravenous injection in mice, cardiovascular structures such as the heart, aorta, and carotid arteries were clearly visualized by T1-weighted MRI. An iron oxide nanoparticle coated with C-DNP-10 having a surface charge of -30 mV and hydrodynamic size of 5 nm also exhibited a total hydrodynamic size of 11 nm. Given its size greater than 8 nm and a highly negative surface charge of -30 mV, the nanoparticle is readily phagocytosed by Kupffer cells in the liver after intravenous administration in mice, resulting in hepatic accumulation. This liver-specific accumulation was confirmed using T2-weighted MRI imaging (FIG. 41c).

**Example 7**

This example investigates whether crosslinking various polysaccharides including dextran (see FIG. 42) can form polysaccharide-crosslinked colloidal particles usable as T1 MRI contrast agents.

**7-1. Synthesis of Nanostructures Using Dextran T-5**

A total of 180 μmol of dextran T-5 (FIG. 42 (a-i), average molecular weight: 5,000 Da) was dissolved in 9 mL of distilled water, followed by the addition of epichlorohydrin and NaOH. Subsequently, 380 mmol diethylenetriamine was added, and the mixture was stirred at room temperature (RT) for 24 hours. The resulting product was purified using a 5 kDa molecular weight cut-off (MWCO) filter.

**7-2. Synthesis of Nanostructures Using Maltodextrin**

The procedure was identical to that described in Example 7-1, except that maltodextrin (FIG. 42 (a-ii), average molecular weight: 990 Da) was used in place of dextran T-5.

**7-3. Synthesis of Nanostructures Using α-Cyclodextrin**

The procedure was identical to that described in Example 7-1, except that α-cyclodextrin (FIG. 42 (b-i), average molecular weight: 970 Da) was used in place of dextran T-5.

**7-4. Synthesis of Nanostructures Using β-Cyclodextrin**

The procedure was identical to that described in Example 7-1, except that β-cyclodextrin (FIG. 42 (b-ii), average molecular weight: 1,100 Da) was used in place of dextran T-5.

**7-5. Synthesis of Nanostructures Using Inulin**

The procedure was identical to that described in Example 7-1, except that inulin (FIG. 42 (c), average molecular weight: 2,500 Da) was used in place of dextran T-5.

To confirm the crosslinking of the nanostructures synthesized in Examples 7-1 to 7-5, each sample was prepared at an equal concentration, placed in a cuvette, and analyzed using a UV-Vis spectrophotometer in the 200-400 nm wavelength range. While typical polymers do not exhibit absorbance in the visible range, nanoparticles formed by crosslinking scatter transmitted light and thereby exhibit an absorption phenomenon at shorter wavelengths. As shown in FIG. 43, the polymers before crosslinking showed no absorbance in the observed range (FIG. 43, orange line), whereas the crosslinked nanostructures exhibited absorption in the short-wavelength range (200-250 nm), confirming the formation of nanostructures via crosslinking (FIG. 43, black line).

The nanostructures synthesized in Examples 7-1 through 7-5 contained surface amine groups introduced via diethylenetriamine. To quantify the number of surface amine groups per nanostructure, an o-phthalaldehyde assay was performed. The results showed that dextran T-5 contained 12.7 amine groups, maltodextrin 4.5, α-cyclodextrin 8.1, β-cyclodextrin 9.0, and inulin 8.0. This indicates that the synthesis method consistently introduces amine groups onto the surface of all nanostructures.

**7-6. Introduction of Carboxyl Groups onto Nanostructures**

To each of the nanostructures prepared in Examples 7-1 to 7-5, 25 mg of succinic anhydride was added and the mixture was stirred for 24 hours at room temperature to induce a succinylation reaction. The product was purified using a 5 kDa MWCO filter.

The number of residual amine groups in the materials from Example 7-6 was measured using the o-phthalaldehyde assay. The number of residual amine groups per nanostructure was as follows: dextran T-5, 0.7; maltodextrin, 0.2; α-cyclodextrin, 0.2; β-cyclodextrin, 0.2; and inulin, 0.4. A value of less than or equal to 1 indicates that essentially all amine groups were converted into carboxyl groups. Therefore, it was confirmed that the amine groups on the nanostructures derived from various polymers were successfully replaced with carboxyl groups in this reaction.

### 7-7. Iron Loading onto Nanostructures

To the carboxylated nanostructures of Example 7-6, 45 μL of ferric chloride hexahydrate solution was added. The pH was adjusted to 8 using 2.5 M NaOH, and the mixture was reacted at room temperature for 1 hour. The product was purified using a 5 kDa MWCO filter to yield the iron-loaded nanostructures.

The iron content and polymer content of the materials from Example 7-7 were analyzed. Surface-bound iron was measured using inductively coupled plasma (ICP) analysis, and the content of each polymer was determined by the phenol-sulfuric acid method. The amount of iron bound per 1 mg of polymer was as follows: dextran T-5, 0.03 mg; maltodextrin, 0.026 mg; α-cyclodextrin, 0.033 mg; β-cyclodextrin, 0.026 mg; and inulin, 0.029 mg. These results confirmed that similar amounts of iron were introduced across different polymer-based nanostructures under the same reaction conditions.

### 7-8. Measurement of Hydrodynamic Diameter and Surface Charge of Iron-Loaded Nanostructures

The hydrodynamic diameter of the iron-loaded nanostructures obtained in Example 7-7 was analyzed using dynamic light scattering (DLS). The results indicated that dextran T-5 exhibited a diameter of 3.6 nm, maltodextrin 6.8 nm, α-cyclodextrin 2.9 nm, β-cyclodextrin 2.8 nm, and inulin 3.8 nm, confirming that the synthesized nanostructures possess similar hydrodynamic sizes. Surface charge measurements showed values of -3.01 mV for dextran T-5, - 6.62 mV for maltodextrin, -9.06 mV for α-cyclodextrin, -7.32 mV for β-cyclodextrin, and -2.83 mV for inulin. These results confirm that the synthesized iron-loaded nanostructures exhibit comparable surface charge and hydrodynamic diameter.

### 7-9. Viscosity Comparison of Nanostructures

The nanostructures from Example 7-7 were prepared at a concentration of 5 mg/mL, and viscosity measurements were performed at 25°C. The viscosity values of the crosslinked polymers were 1.09 mPa·s for dextran T-5, 1.43 mPa·s for maltodextrin, 1.26 mPa·s for α-cyclodextrin, 1.30 mPa·s for β-cyclodextrin, and 1.14 mPa·s for inulin, indicating similar viscosity profiles among the nanostructures.

### 7-10. T1 MRI Contrast Performance Evaluation of Nanostructures

To evaluate the T1 MRI performance of the materials from Example 7-7, both the spin-spin relaxivity coefficient ($r_2$) and spin-lattice relaxivity coefficient ($r_1$) were measured, and the $r_2/r_1$ ratio was calculated. The $r_2/r_1$ ratio is a critical indicator in determining whether a contrast agent is suitable for T1 or T2 MRI. As shown in Fig. 44, 3.0 Tesla MRI analysis revealed the following: dextran T-5 exhibited an $r_1$ of 1.90 and $r_2$ of 2.27 ($r_2/r_1$ = 1.19); maltodextrin exhibited $r_1$ = 4.60, $r_2$ = 5.19 ($r_2/r_1$ = 1.13); α-cyclodextrin exhibited $r_1$ = 5.26, $r_2$ = 5.78 ($r_2/r_1$ = 1.10); β-cyclodextrin exhibited $r_1$ = 5.62, $r_2$ = 6.24 ($r_2/r_1$ = 1.11); and inulin exhibited $r_1$ = 4.33, $r_2$ = 4.73 ($r_2/r_1$ = 1.09). These results indicate that all synthesized nanostructures possess $r_2/r_1$ ratios close to 1, thereby confirming their suitability as T1 MRI contrast agents.

### 7-11. In Vivo Contrast Efficacy Evaluation of Iron-Loaded Nanostructures

The nanostructures from Example 7-7 were administered intravenously to mice, and T1-weighted images were acquired using 9.4 Tesla MRI. Male Balb/c mice (5 weeks or older) were anesthetized and injected via the tail vein with the nanostructures. T1-weighted MRI images were obtained at various time points, and signal-to-noise ratio (SNR) was analyzed. As shown in FIG. 45, all iron-loaded nanostructures from Example 7-7 exhibited bright vascular signals (e.g., in the jugular vein) within approximately 3-5 minutes post-injection.

### 7-12. Renal Excretion of Iron-Loaded Nanostructures

Following intravenous administration of the nanostructures from Example 7-7, T1-weighted MRI scans were performed for up to 1 hour using a 9.4 Tesla MRI to evaluate renal clearance. As shown in FIG. 46, T1 signals began to appear in the bladder within 30 minutes for most iron-loaded nanostructures. These observations indicate that the administered agents passed through the kidneys and were excreted into the bladder, confirming renal clearance.

**Claims**

1. A formulation for non-intravenous administration, comprising nanoparticles having a hydrodynamic diameter of 2 to 20 nm and a surface charge ranging from -20 mV to 0 mV,

   the nanoparticles being adjusted in size and charge by hydration of hydrophilic functional groups exposed on the surface thereof, such that the nanoparticles are not permeated into or drained into blood capillaries at the administration site but are selectively drained into terminal lymphatic vessels,
   wherein the nanoparticles:

   (i) are themselves therapeutic agents; and/or
   (ii) serve as carriers for other therapeutic agents,

   and wherein the nanoparticles are engineered to provide a drug modality that, upon administration into a tissue site rather than via intravenous injection, is selectively drained into lymphatic vessels, thereby avoiding drainage into blood capillaries and thus prolonging residence time at the administration site.

2. The formulation of claim 1, wherein the nanoparticles are:

   (a) polysaccharide-crosslinked colloidal particles formed by intra- and/or inter-molecular crosslinking of 1 to 3 branched polysaccharides or 2 to 30 cyclic polysaccharides at hydroxyl groups of constituent monosaccharide building blocks using a crosslinker, such that the particles are resistant to enzymatic hydrolysis in vivo; or
   (b) entities surface-coated with the polysaccharide-crosslinked colloidal particles.

3. The formulation of claim 1, wherein the nanoparticles extend the residence time at the administration site, such that interstitial space expansion, anatomical space distension, and/or lymph node enlargement is induced.

4. The formulation of claim 1, wherein the nanoparticles comprise at least one component selected from the group consisting of iron, manganese, and gadolinium.

5. The formulation of claim 1, wherein the nanoparticles are engineered to be squeezable by hydration of hydrophilic functional groups exposed on the surface thereof, such that dermal backflow or lymph node congestion is not induced in lymphatic vessels at a lymphedema site.

6. The formulation of claim 1, wherein the nanoparticles are excreted from the body without residual accumulation at the administration site within one week.

7. The formulation of claim 1, wherein the non-intravenous administration is by intradermal injection, subcutaneous injection, or intralymphatic injection.

8. The formulation of claim 1, wherein the nanoparticles are excreted via the liver or kidneys.

9. The formulation of claim 1, wherein the formulation provides a contrast agent for use in medical imaging techniques that visualize the lymphatic system and its functions.

10. The formulation of claim 1, wherein the nanoparticles serve as a contrast agent for effective imaging of disease diagnosis or biological phenomena at the molecular and cellular levels.

11. The formulation of claim 1, wherein the lymphatic vessels into which the nanoparticles are drained have a diameter of 0.1 mm or greater.

12. The formulation of claim 9, wherein the formulation is used to evaluate lymphatic flow, drainage, or congestion.

13. The formulation of claim 9, wherein the medical imaging technique is selected from the group consisting of magnetic resonance lymphangiography, ultrasound lymphangiography, computed tomography (CT) lymphangiography, positron emission tomography (PET) lymphangiography, and lymphoscintigraphy.

14. The formulation of any one of claims 1 to 13, wherein the nanoparticles are contrast agents exhibiting T1-MRI contrast

effects.

**15.** A contrast agent composition engineered for non-intravenous administration, comprising nanoparticles having a hydrodynamic diameter of 2 to 20 nm and a surface charge of -20 mV to 0 mV, wherein the nanoparticles are configured such that they are substantially excluded from entering or being drained into blood capillaries at the administration site and are selectively drained into lymphatic vessels, such that, upon administration to tissue other than via intravenous injection, the composition enables selective imaging of peripheral and central lymphatic vessels without venous contamination.

**16.** The contrast agent composition of claim 15, wherein the nanoparticles comprise:

(a) polysaccharide-crosslinked colloidal particles formed by intra- and/or inter-molecular crosslinking of 1 to 3 branched polysaccharides or 2 to 30 cyclic polysaccharides at hydroxyl groups of the constituent monosaccharide building blocks using a crosslinker, such that the nanoparticles are resistant to hydrolysis by in vivo enzymes, and wherein metal ions selected from the group consisting of iron, manganese, and gadolinium are coordinated to functional groups derived from the crosslinker on the nanoparticle surface; or
(b) magnetic nanoparticles surface-coated with the polysaccharide-crosslinked colloidal particles,

wherein the nanoparticles are selectively drained into lymphatic vessels when administered to tissue other than via intravenous injection, thereby avoiding drainage into blood capillaries and thus prolonging residence time at the administration site.

**17.** The contrast agent composition of claim 15, wherein the nanoparticles comprise magnetic substances exhibiting T1-weighted MRI contrast enhancement.

**18.** The contrast agent composition of claim 15, wherein the composition provides imaging of lymphatic vessels and/or lymph nodes.

**19.** The contrast agent composition of claim 15, wherein the composition provides spatial resolution sufficient to visualize individual lymphatic vessels regardless of depth or subcutaneous tissue condition.

**20.** The contrast agent composition of claim 15, wherein the lymphatic vessels can be visualized via T1-weighted MRI for up to 1 hour after intradermal or subcutaneous administration of the nanoparticles.

**21.** The contrast agent composition of claim 15, wherein the composition is used in planning venous-lymphatic anastomosis procedures.

**22.** A method of providing diagnostic information regarding lymphatic diseases without venous contamination, comprising administering the contrast agent composition of any one of claims 15 to 21.

**23.** The method of claim 22, wherein enhancement of venous blood vessels due to drainage of the contrast agent from lymphatic vessels into the venous circulation is excluded, thereby eliminating vascular imaging noise.

**24.** The method of claim 22, wherein the acquired imaging data comprises an image of lymphatic vessel obstruction.

**25.** The method of claim 22, wherein the provided information supports early diagnosis or prevention of lymphedema.

**26.** A formulation for non-intravenous administration, comprising polysaccharide-crosslinked colloidal particles having:

• a hydrodynamic diameter of 2 to 20 nm, preferably 10 nm or less, more preferably 8 nm or less, and most preferably 6 nm or less; and
• a surface charge ranging from -20 mV to 0 mV,
wherein the polysaccharide-crosslinked colloidal particles are formed by intra- and/or inter-molecular crosslinking of 1 to 3 branched polysaccharides or 2 to 30 cyclic polysaccharides at hydroxyl groups of constituent monosaccharide building blocks using a crosslinker, such that the particles are resistant to enzymatic hydrolysis in vivo;
and wherein the formulation is engineered to provide a drug modality that is selectively drained into lymphatic vessels when administered to tissue or a body cavity other than via intravenous injection, thereby avoiding

drainage into blood capillaries and thus prolonging residence time at the administration site to 60 minutes or more.

FIG. 1

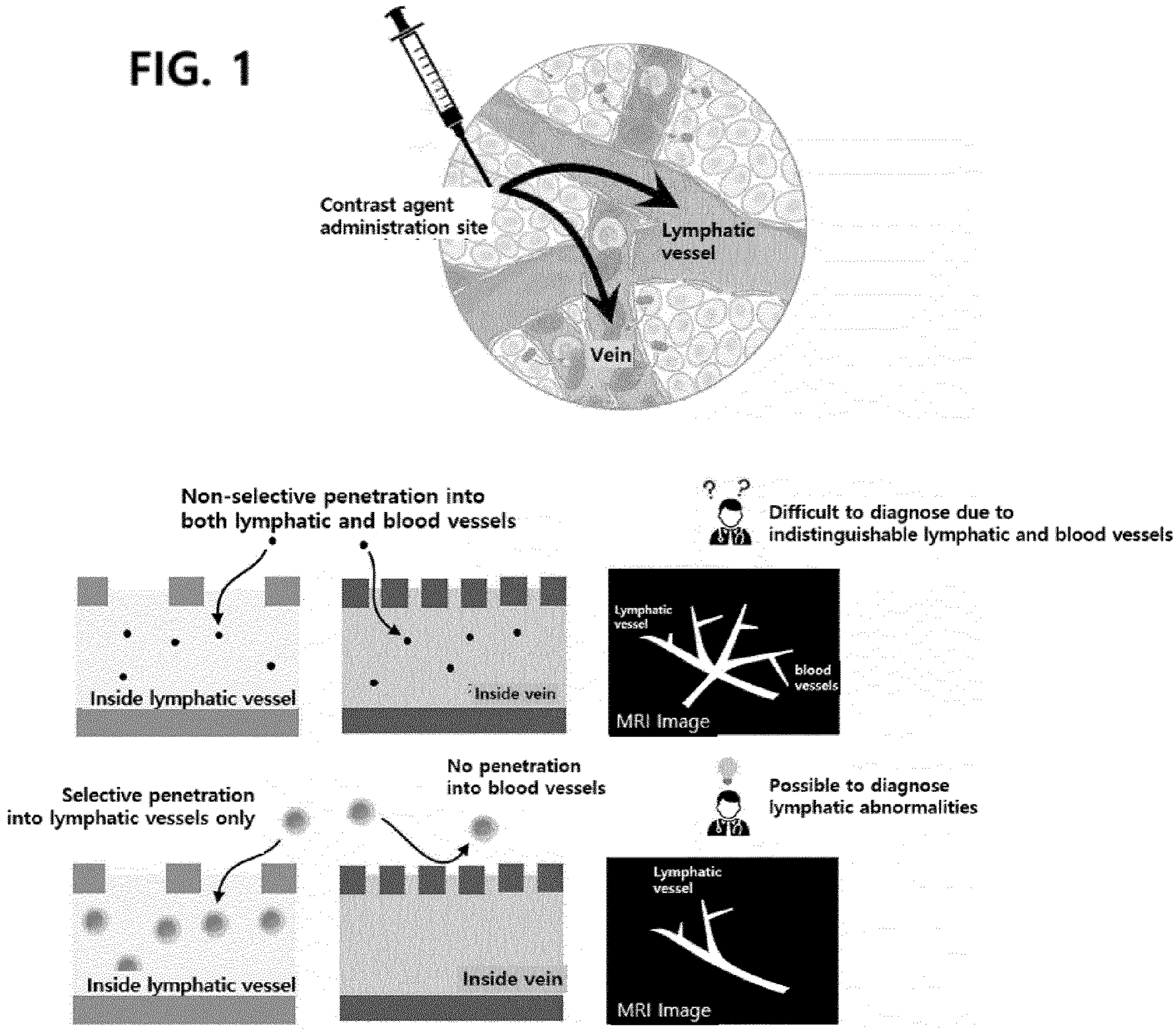
Contrast agent administration site
Lymphatic vessel
Vein
Non-selective penetration into both lymphatic and blood vessels
Difficult to diagnose due to indistinguishable lymphatic and blood vessels
Inside lymphatic vessel
Inside vein
Lymphatic vessel
blood vessels
MRI Image
Selective penetration into lymphatic vessels only
No penetration into blood vessels
Possible to diagnose lymphatic abnormalities
Inside lymphatic vessel
Inside vein
Lymphatic vessel
MRI Image

FIG. 2

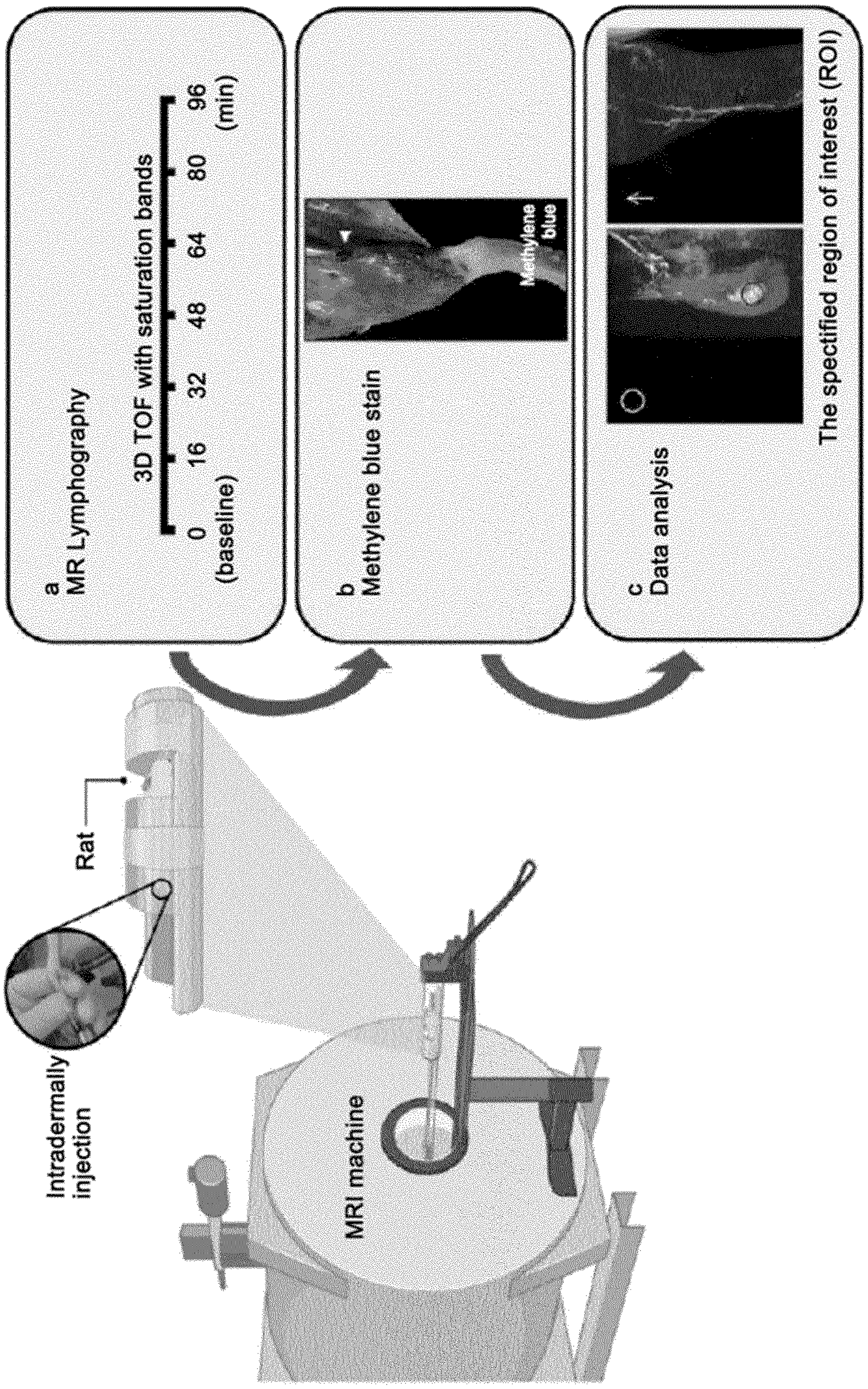
Intradermally injection
Rat
MRI machine
a
MR Lymphography
3D TOF with saturation bands
0 (baseline)
16
32
48
64
80
96 (min)
b
Methylene blue stain
Methylene blue
c
Data analysis
The spectified region of interest (ROI)

FIG. 3

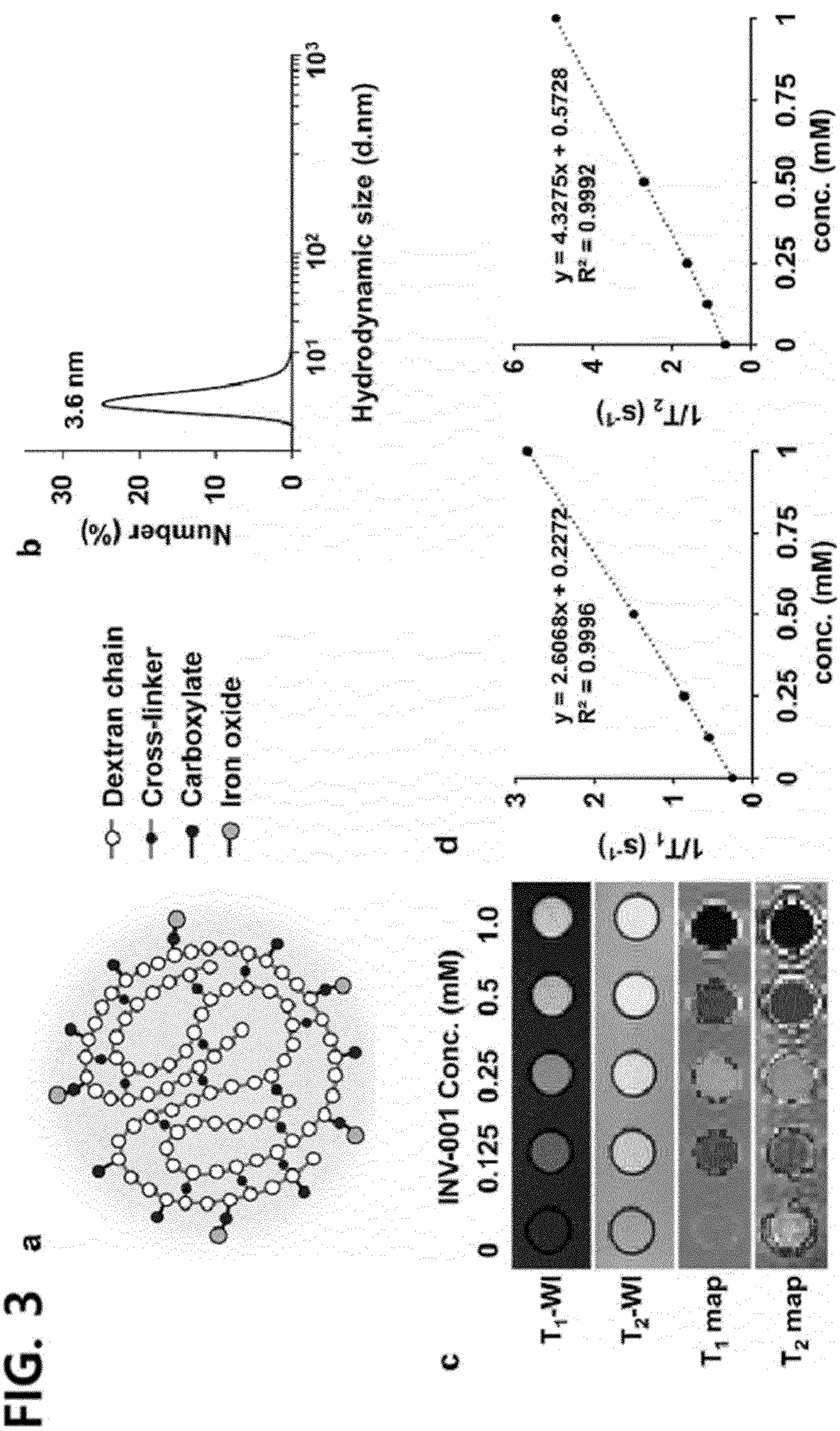
a
Dextran chain
Cross-linker
Carboxylate
Iron oxide
b
Number (%)
30
20
10
0
3.6 nm
$10^1$
$10^2$
$10^3$
Hydrodynamic size (d.nm)
c
INV-001 Conc. (mM)
0
0.125
0.25
0.5
1.0
$T_1$-WI
$T_2$-WI
$T_1$ map
$T_2$ map
d
$1/T_1$ ($s^{-1}$)
y = 2.6068x + 0.2272
$R^2$ = 0.9996
conc. (mM)
$1/T_2$ ($s^{-1}$)
y = 4.3275x + 0.5728
$R^2$ = 0.9992
conc. (mM)
0
0.25
0.50
0.75
1

FIG. 4

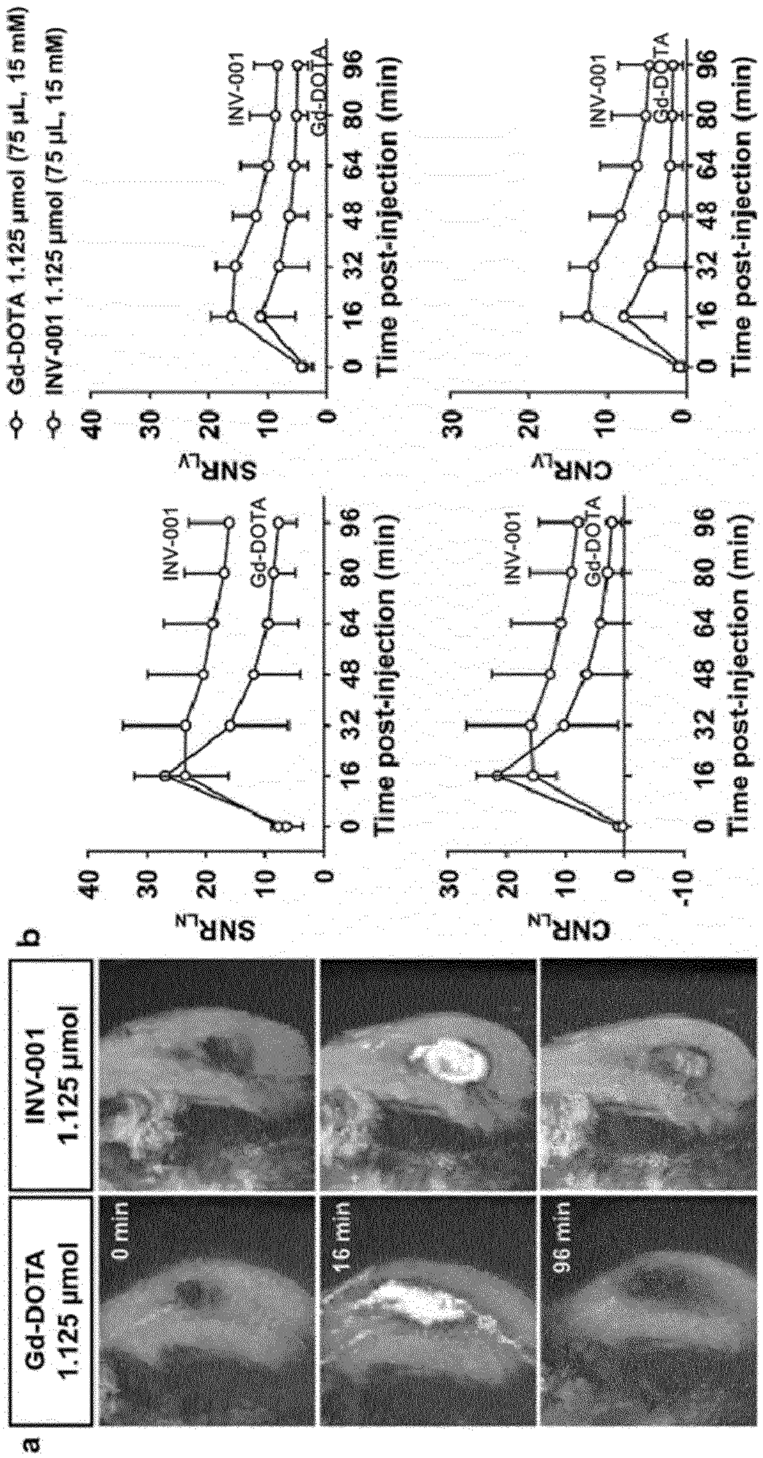
a
Gd-DOTA
1.125 µmol
INV-001
1.125 µmol
0 min
16 min
96 min
b
Gd-DOTA 1.125 µmol (75 µL, 15 mM)
INV-001 1.125 µmol (75 µL, 15 mM)
SNR$_{LN}$
CNR$_{LN}$
SNR$_{LV}$
CNR$_{LV}$
INV-001
Gd-DOTA
Time post-injection (min)
0 16 32 48 64 80 96

FIG. 5

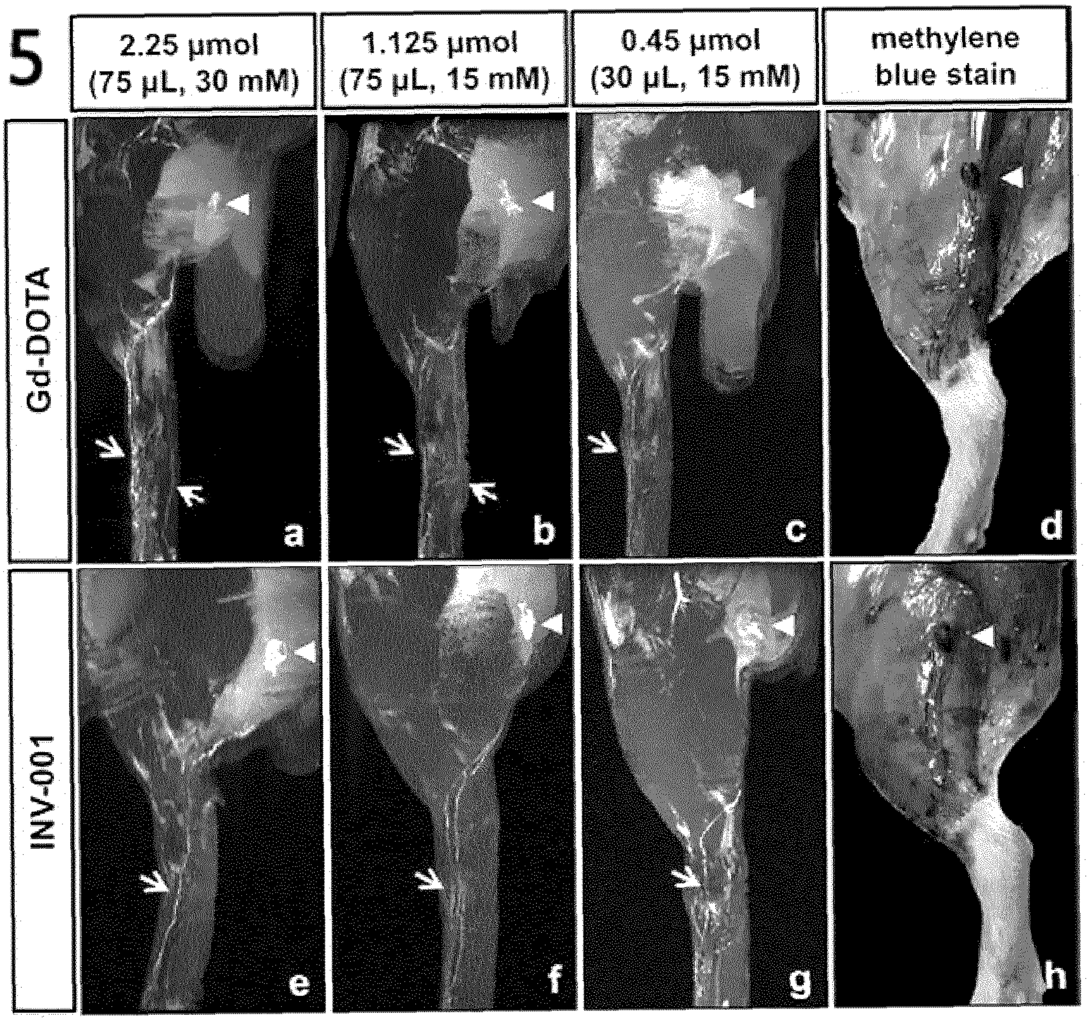
2.25 µmol
(75 µL, 30 mM)
1.125 µmol
(75 µL, 15 mM)
0.45 µmol
(30 µL, 15 mM)
methylene
blue stain
Gd-DOTA
INV-001
a
b
c
d
e
f
g
h

FIG. 6

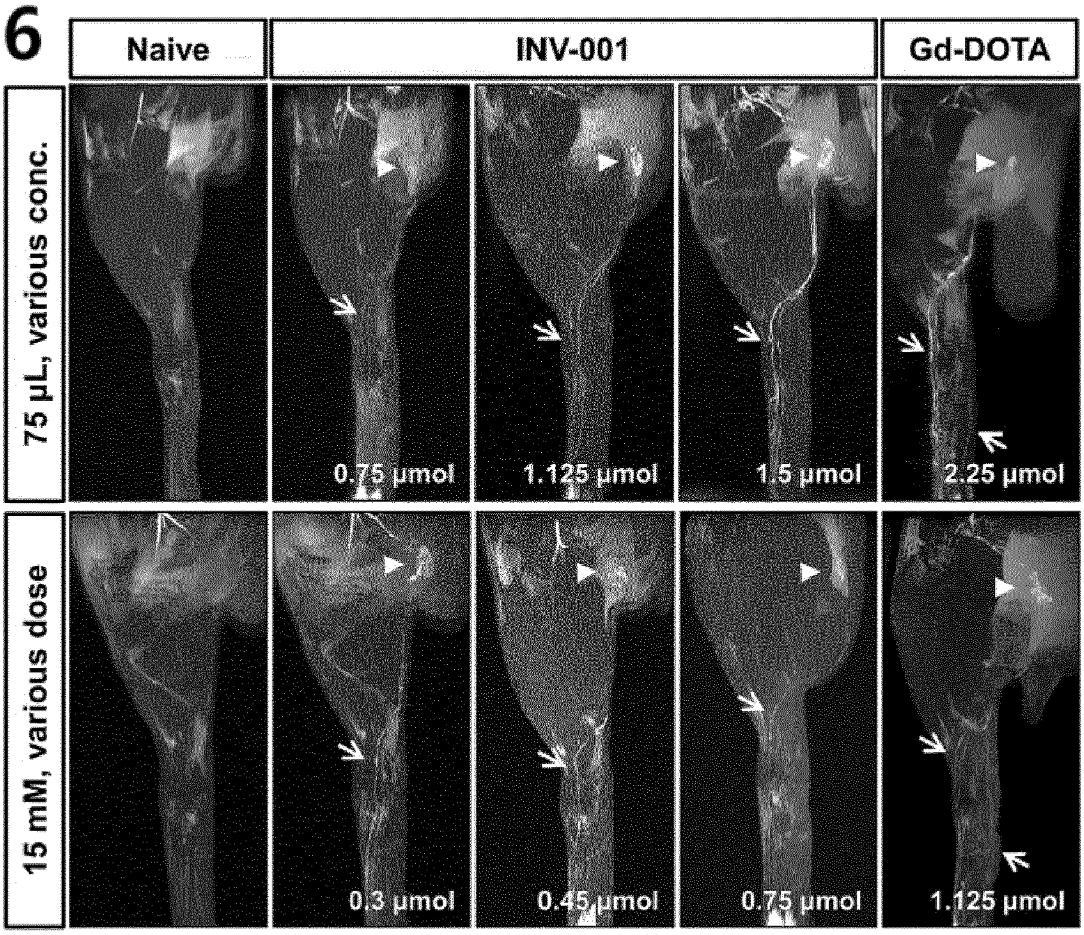
Naive
INV-001
Gd-DOTA
75 μL, various conc.
15 mM, various dose
0.75 μmol
1.125 μmol
1.5 μmol
2.25 μmol
0.3 μmol
0.45 μmol
0.75 μmol
1.125 μmol

FIG. 7

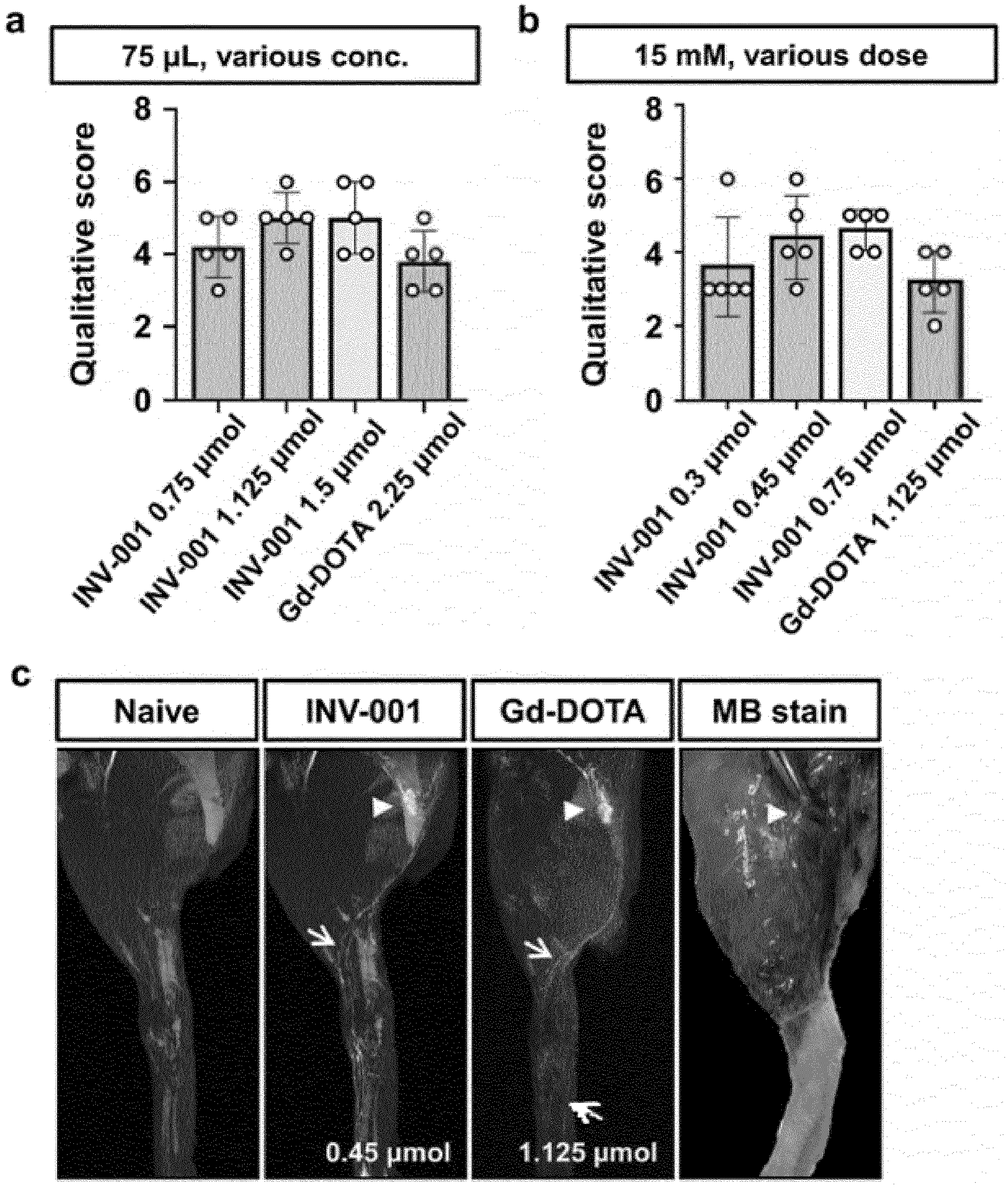
a
75 µL, various conc.
Qualitative score
8
6
4
2
0
INV-001 0.75 µmol
INV-001 1.125 µmol
INV-001 1.5 µmol
Gd-DOTA 2.25 µmol
b
15 mM, various dose
Qualitative score
8
6
4
2
0
INV-001 0.3 µmol
INV-001 0.45 µmol
INV-001 0.75 µmol
Gd-DOTA 1.125 µmol
c
Naive
INV-001
Gd-DOTA
MB stain
0.45 µmol
1.125 µmol

FIG. 8

| | INV-001 | INV-002 |
|---|---|---|
| Maximum iron concentration upon concentration | 2.6 Fe mg/ml | 1.6 Fe mg/ml |
| Viscosity at the same concentration | 5.3 cP | 5.3 cP |

FIG. 9

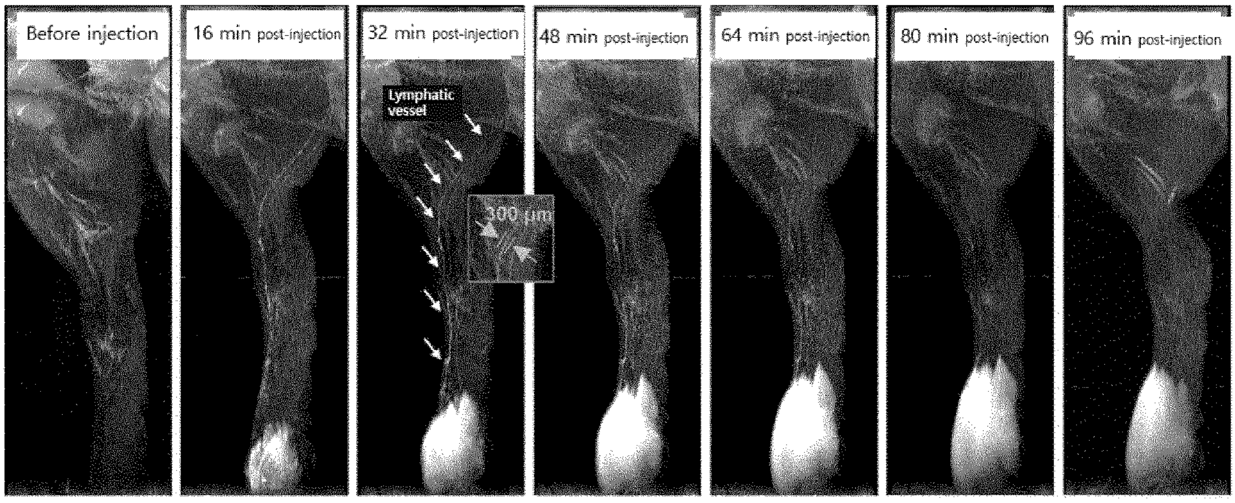


FIG. 10

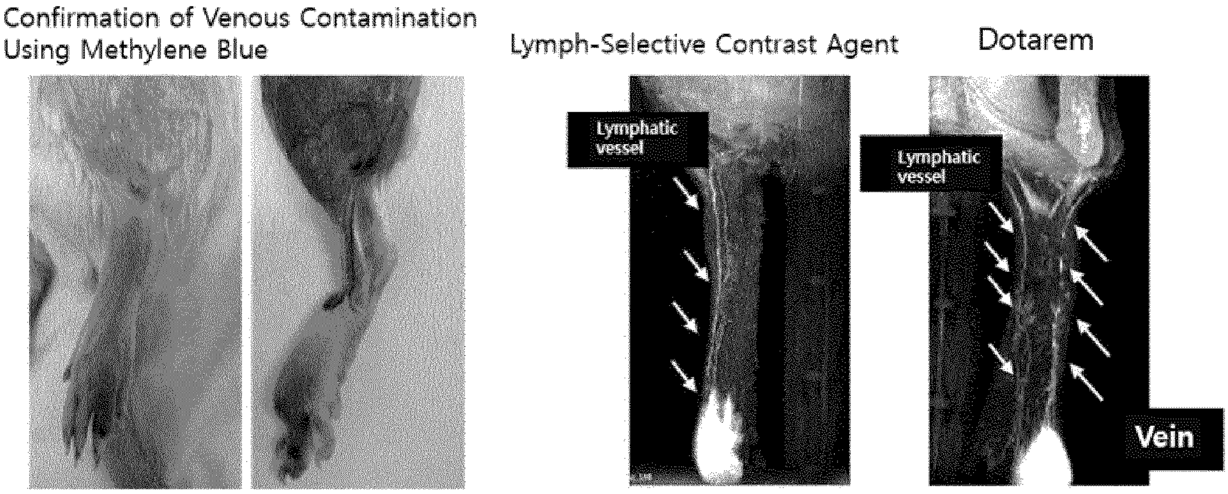

FIG. 11

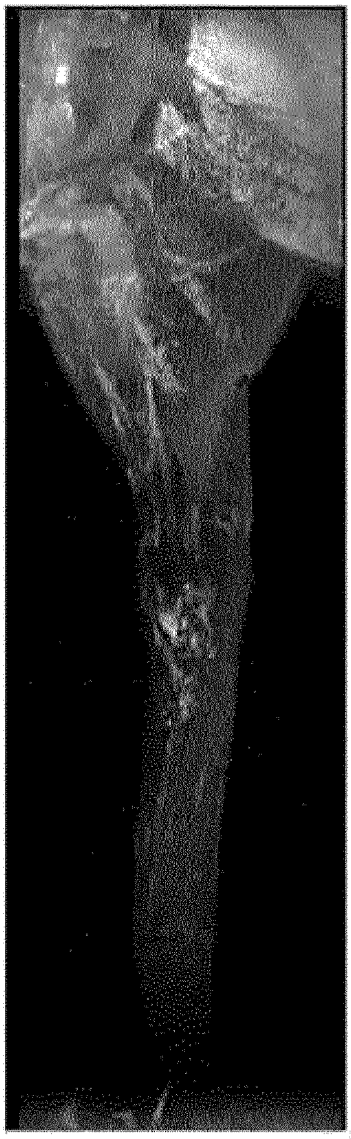

FIG. 12

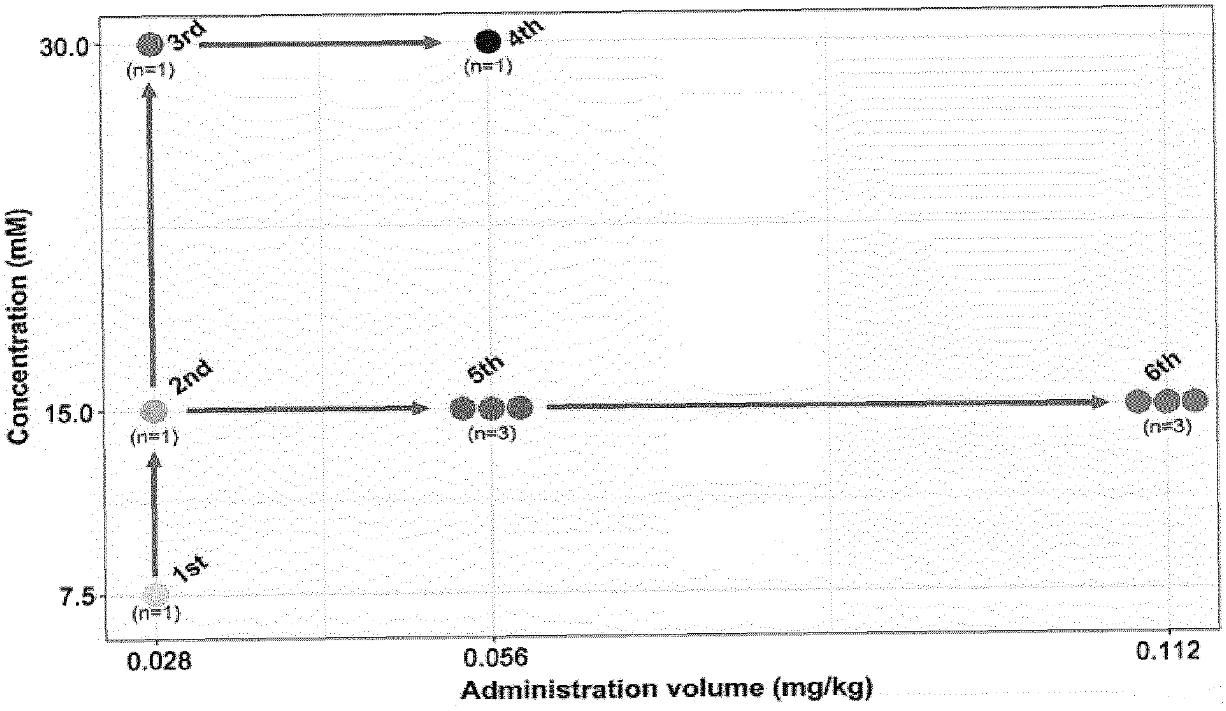
30.0
15.0
7.5
Concentration (mM)
0.028
0.056
0.112
Administration volume (mg/kg)
1st
(n=1)
2nd
(n=1)
3rd
(n=1)
4th
(n=1)
5th
(n=3)
6th
(n=3)

FIG. 13

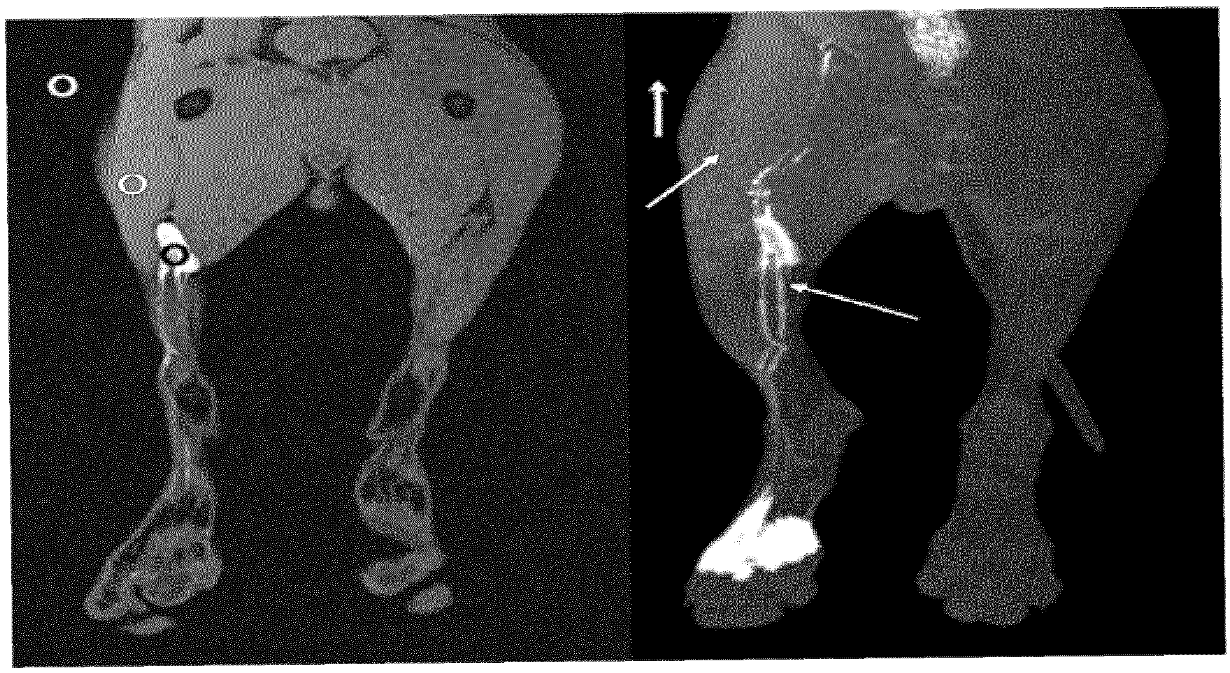

FIG. 14 a

A

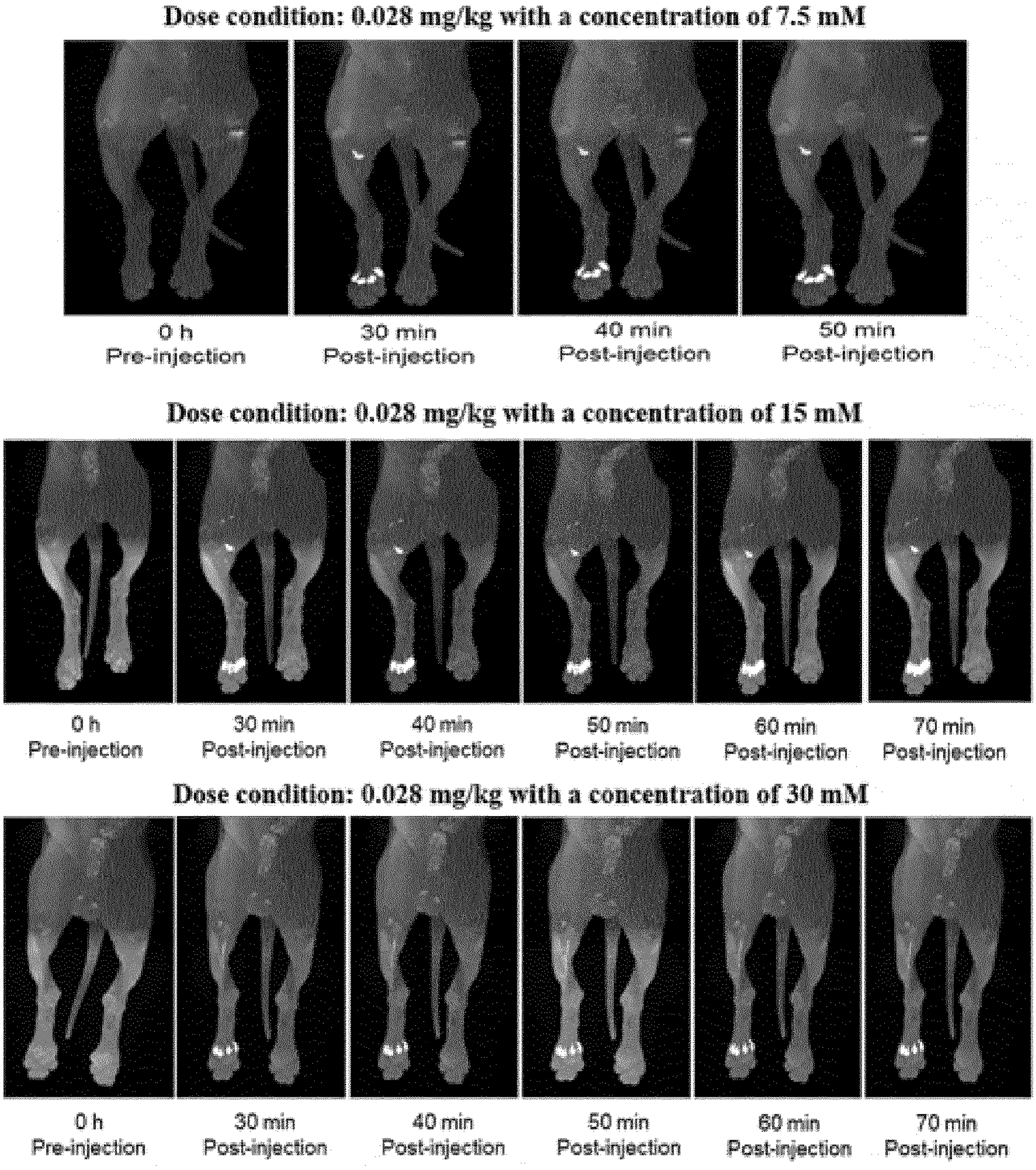
Dose condition: 0.028 mg/kg with a concentration of 7.5 mM
0 h
Pre-injection
30 min
Post-injection
40 min
Post-injection
50 min
Post-injection
Dose condition: 0.028 mg/kg with a concentration of 15 mM
0 h
Pre-injection
30 min
Post-injection
40 min
Post-injection
50 min
Post-injection
60 min
Post-injection
70 min
Post-injection
Dose condition: 0.028 mg/kg with a concentration of 30 mM
0 h
Pre-injection
30 min
Post-injection
40 min
Post-injection
50 min
Post-injection
60 min
Post-injection
70 min
Post-injection

# FIG. 14b

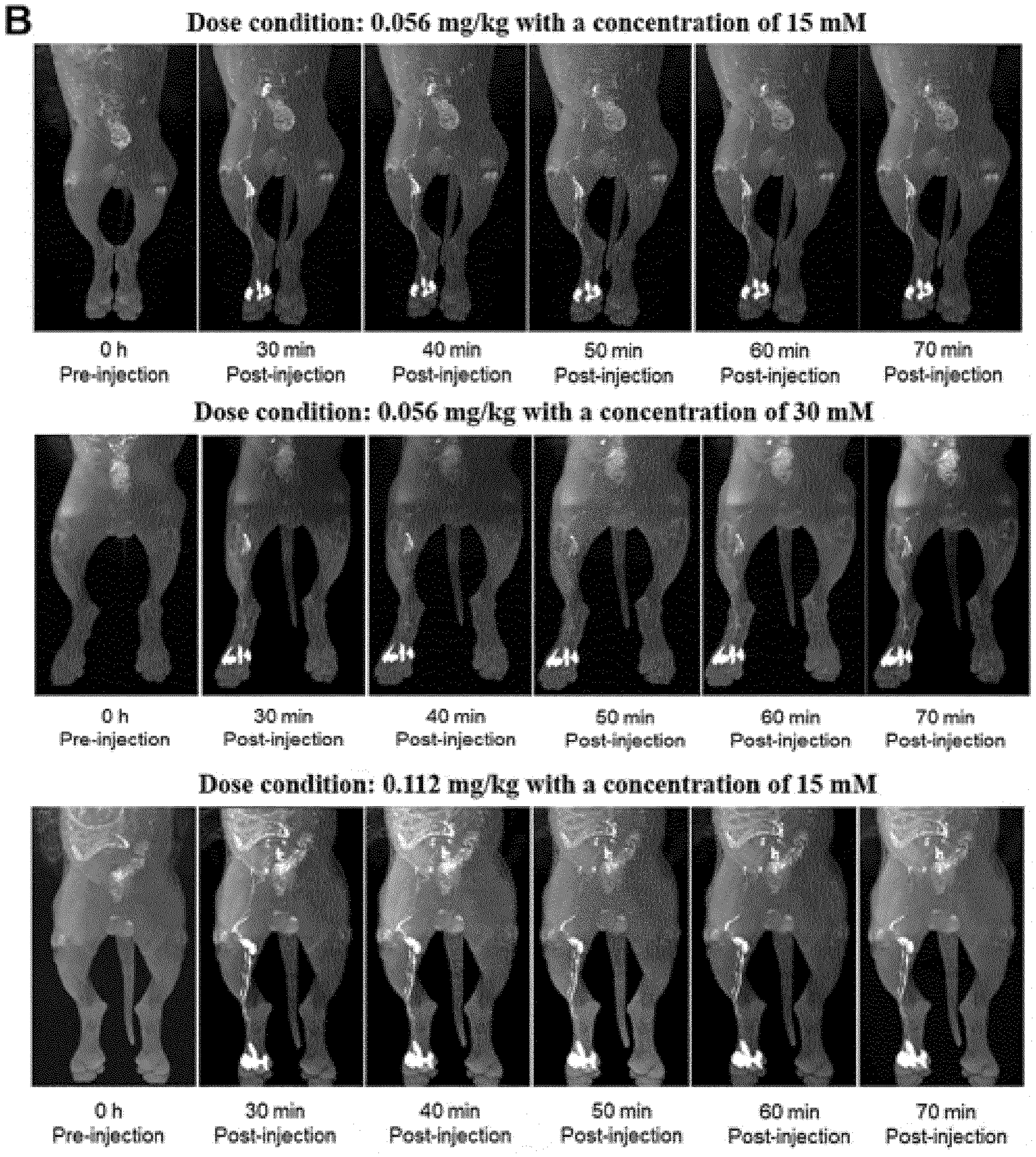
B
Dose condition: 0.056 mg/kg with a concentration of 15 mM
0 h
Pre-injection
30 min
Post-injection
40 min
Post-injection
50 min
Post-injection
60 min
Post-injection
70 min
Post-injection
Dose condition: 0.056 mg/kg with a concentration of 30 mM
0 h
Pre-injection
30 min
Post-injection
40 min
Post-injection
50 min
Post-injection
60 min
Post-injection
70 min
Post-injection
Dose condition: 0.112 mg/kg with a concentration of 15 mM
0 h
Pre-injection
30 min
Post-injection
40 min
Post-injection
50 min
Post-injection
60 min
Post-injection
70 min
Post-injection

FIG. 15

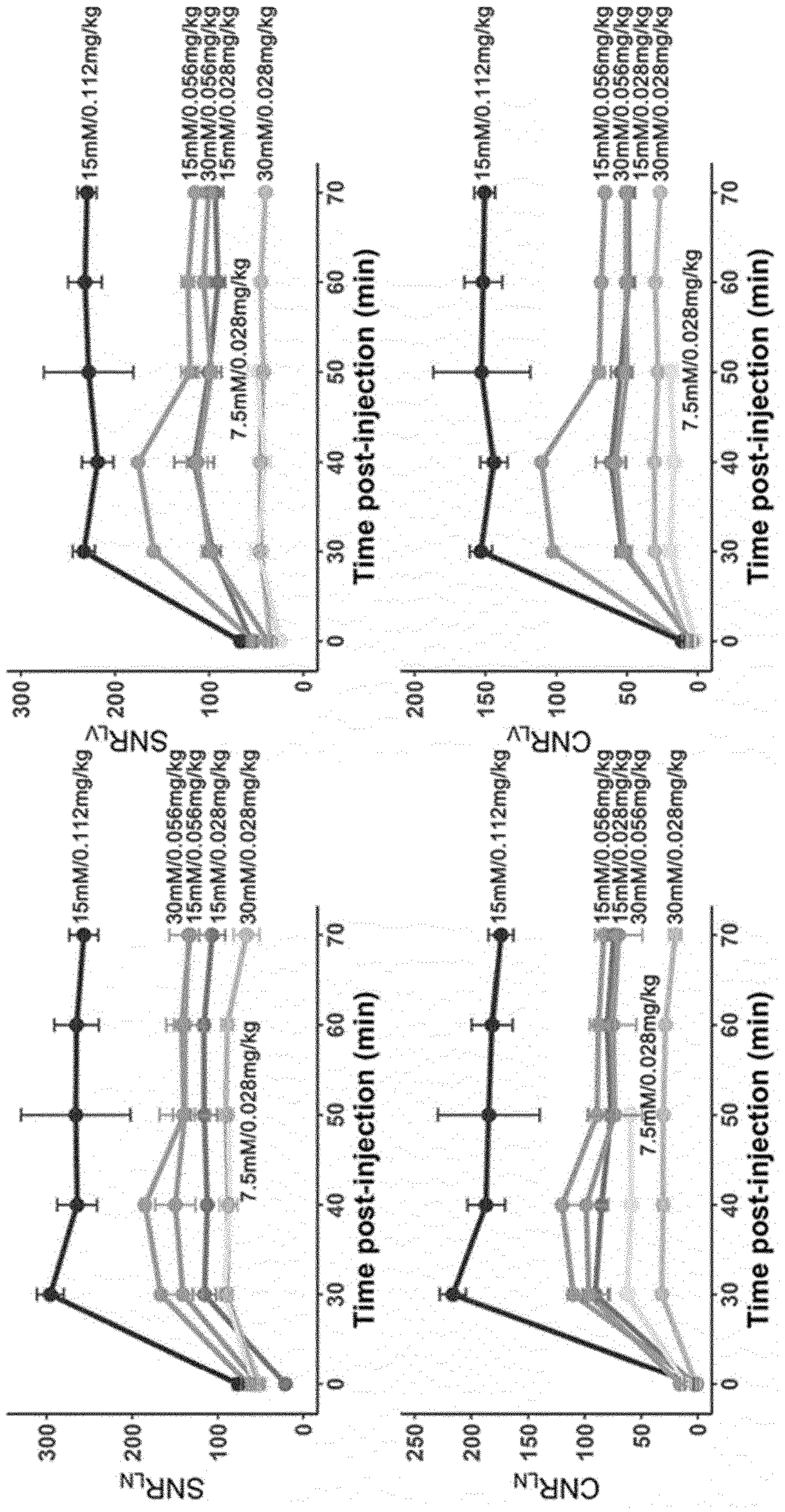
SNR_LN
SNR_LV
CNR_LN
CNR_LV
Time post-injection (min)
15mM/0.112mg/kg
30mM/0.056mg/kg
15mM/0.056mg/kg
15mM/0.028mg/kg
30mM/0.028mg/kg
7.5mM/0.028mg/kg

FIG. 16

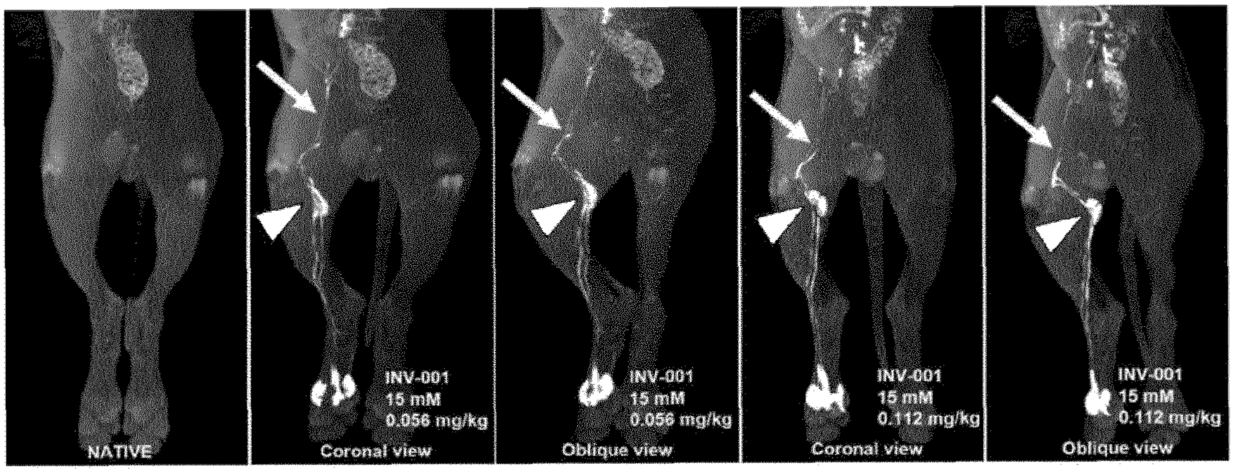
NATIVE
INV-001
15 mM
0.056 mg/kg
Coronal view
INV-001
15 mM
0.056 mg/kg
Oblique view
INV-001
15 mM
0.112 mg/kg
Coronal view
INV-001
15 mM
0.112 mg/kg
Oblique view

FIG. 17

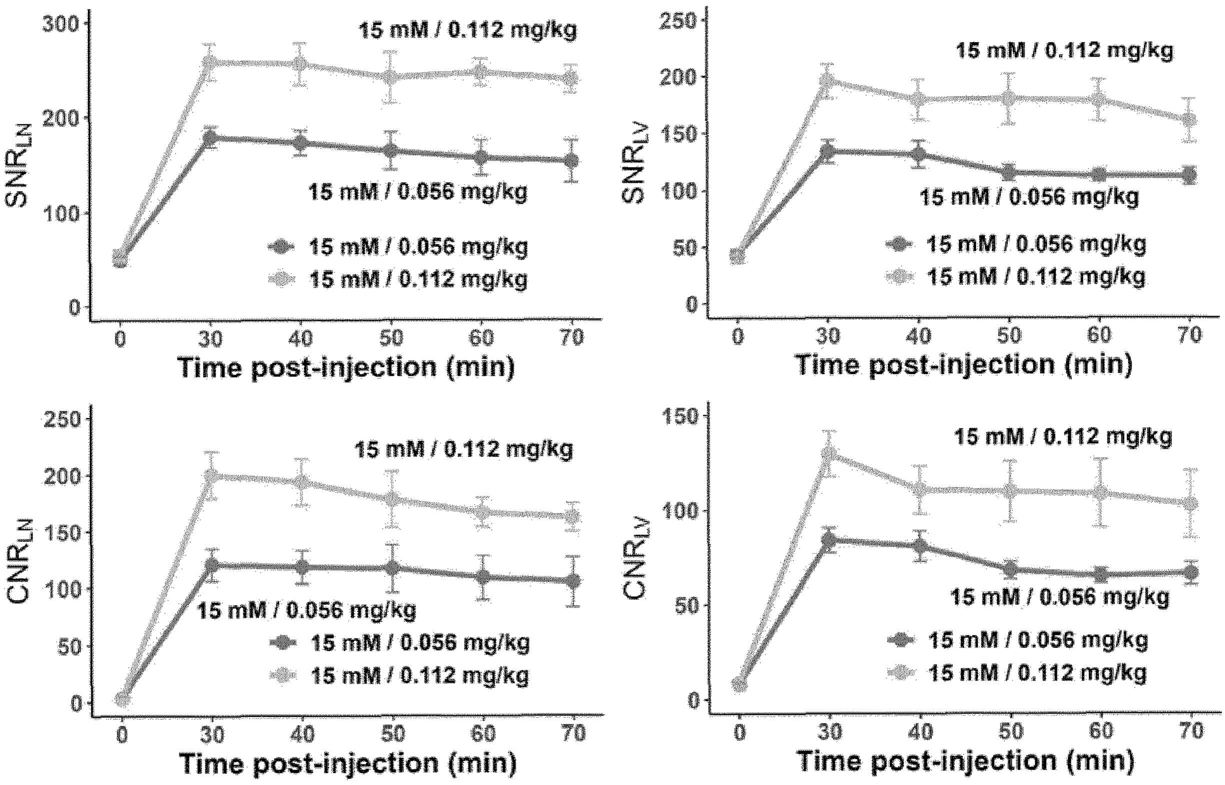
$SNR_{LN}$
15 mM / 0.112 mg/kg
15 mM / 0.056 mg/kg
Time post-injection (min)
$SNR_{LV}$
15 mM / 0.112 mg/kg
15 mM / 0.056 mg/kg
Time post-injection (min)
$CNR_{LN}$
15 mM / 0.112 mg/kg
15 mM / 0.056 mg/kg
Time post-injection (min)
$CNR_{LV}$
15 mM / 0.112 mg/kg
15 mM / 0.056 mg/kg
Time post-injection (min)

FIG. 18

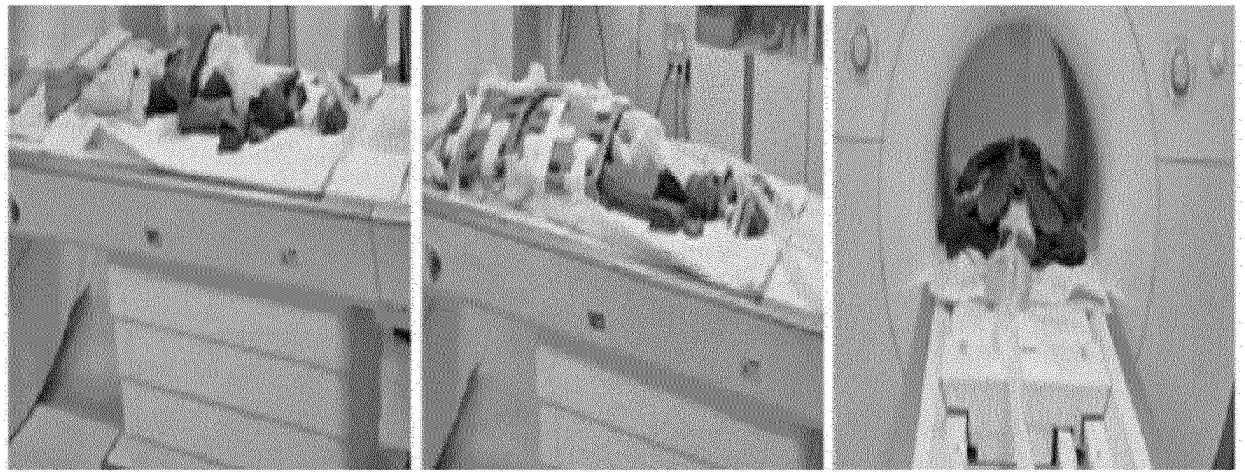

FIG. 19

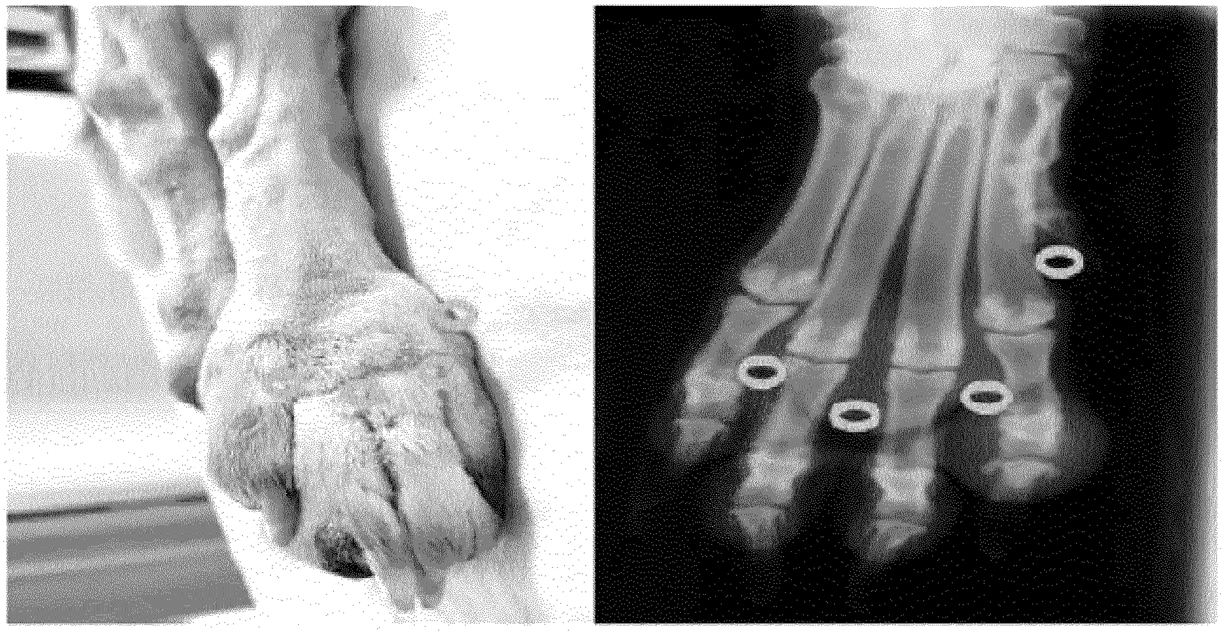

# FIG. 20

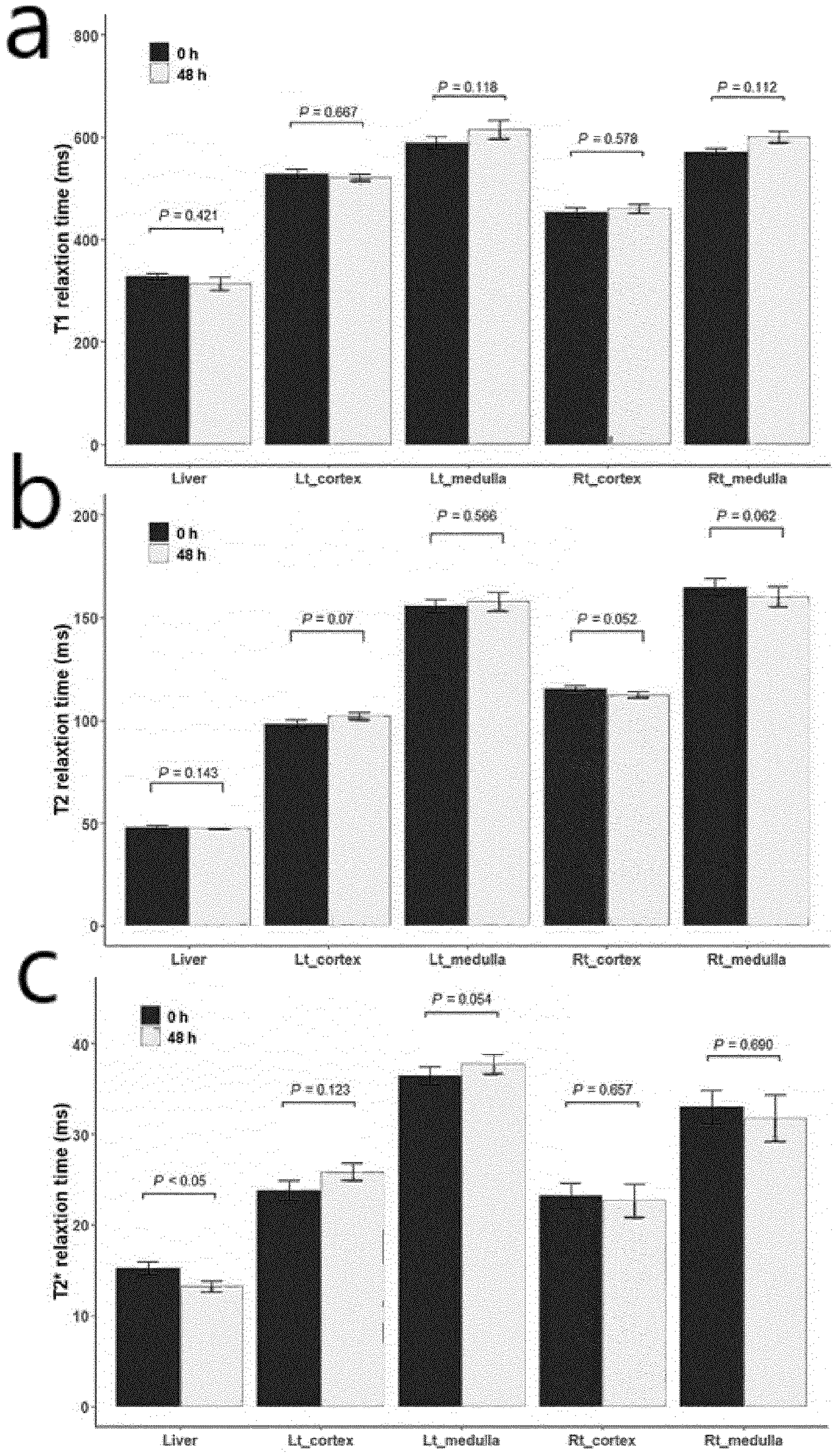
a
0 h
48 h
T1 relaxation time (ms)
800
600
400
200
0
P = 0.421
P = 0.667
P = 0.118
P = 0.578
P = 0.112
Liver
Lt_cortex
Lt_medulla
Rt_cortex
Rt_medulla
b
0 h
48 h
T2 relaxation time (ms)
200
150
100
50
0
P = 0.143
P = 0.07
P = 0.566
P = 0.052
P = 0.062
Liver
Lt_cortex
Lt_medulla
Rt_cortex
Rt_medulla
c
0 h
48 h
T2* relaxation time (ms)
40
30
20
10
0
P < 0.05
P = 0.123
P = 0.054
P = 0.657
P = 0.690
Liver
Lt_cortex
Lt_medulla
Rt_cortex
Rt_medulla

FIG. 21

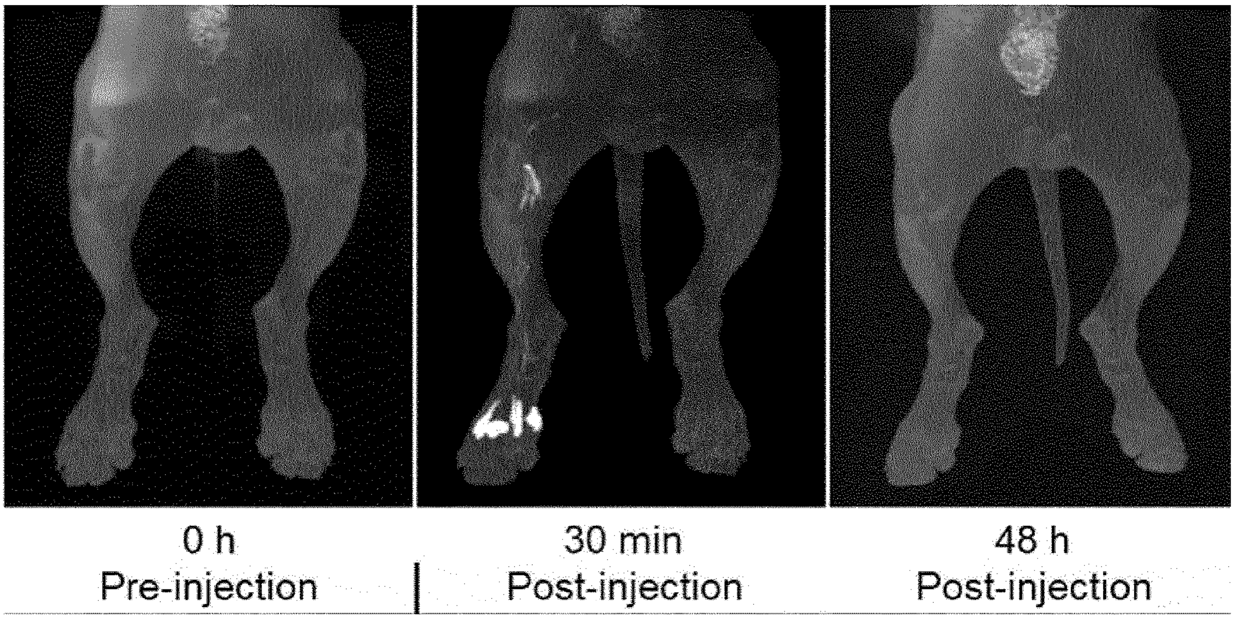

FIG. 22

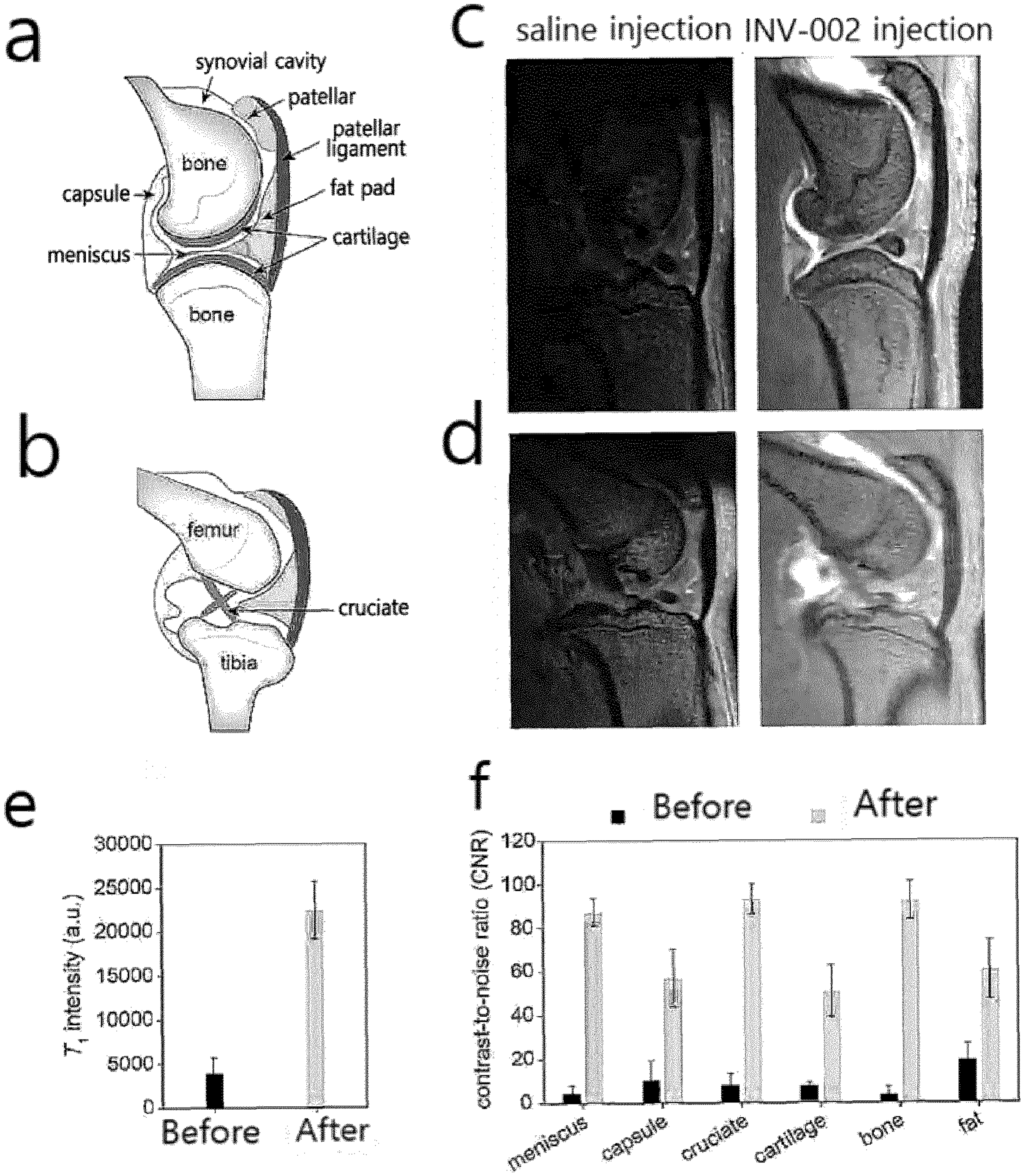
a
synovial cavity
patellar
patellar ligament
bone
capsule
fat pad
cartilage
meniscus
bone
b
femur
cruciate
tibia
c
saline injection
INV-002 injection
d
e
30000
25000
20000
15000
10000
5000
0
$T_1$ intensity (a.u.)
Before
After
f
Before
After
contrast-to-noise ratio (CNR)
120
100
80
60
40
20
0
meniscus
capsule
cruciate
cartilage
bone
fat

FIG. 23

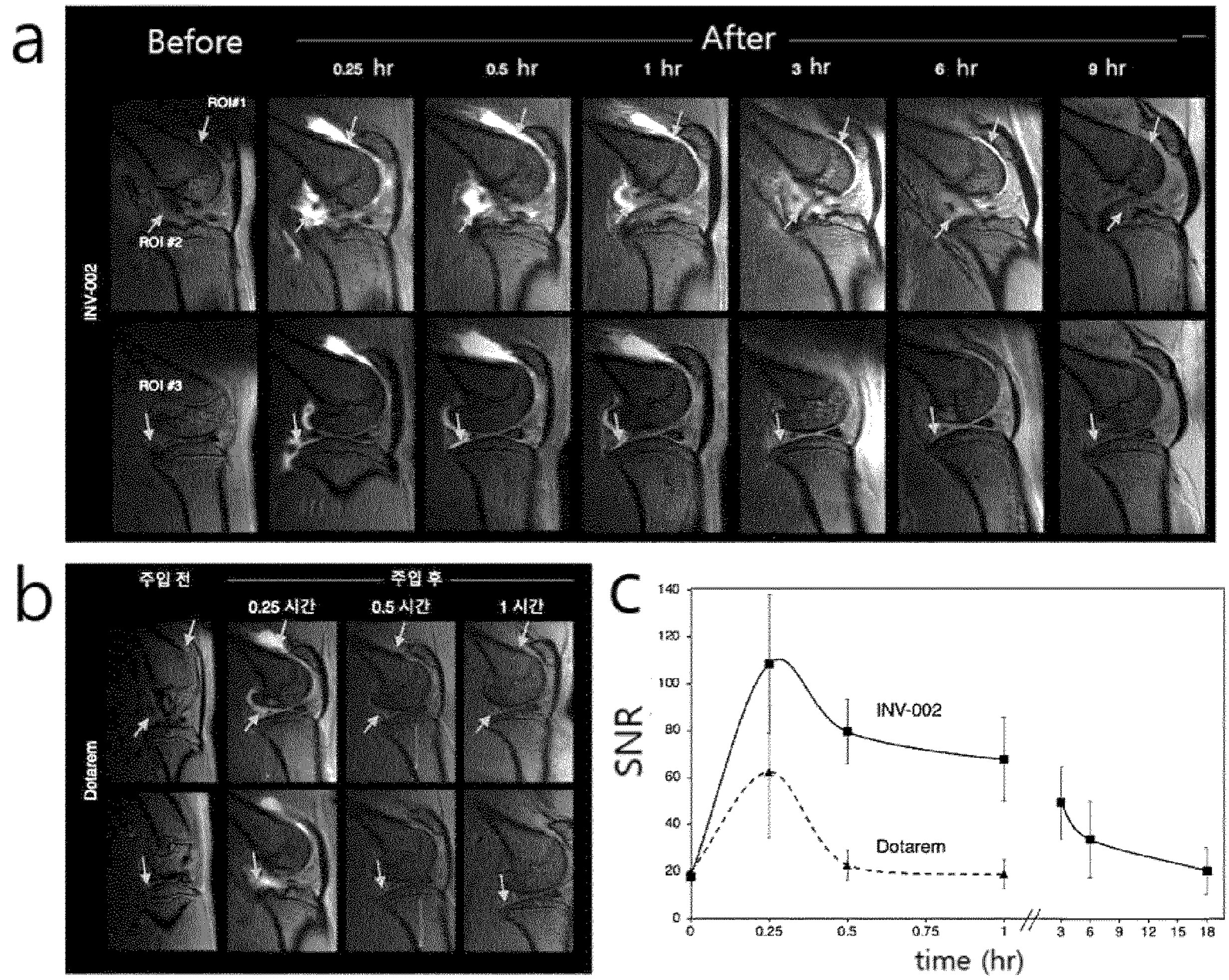
a
Before
After
0.25 hr
0.5 hr
1 hr
3 hr
6 hr
9 hr
ROI#1
ROI #2
ROI #3
INV-002
b
주입 전
주입 후
0.25 시간
0.5 시간
1 시간
Dotarem
c
SNR
INV-002
Dotarem
time (hr)
0
20
40
60
80
100
120
140
0.25
0.5
0.75
1
3
6
9
12
15
18

FIG. 24

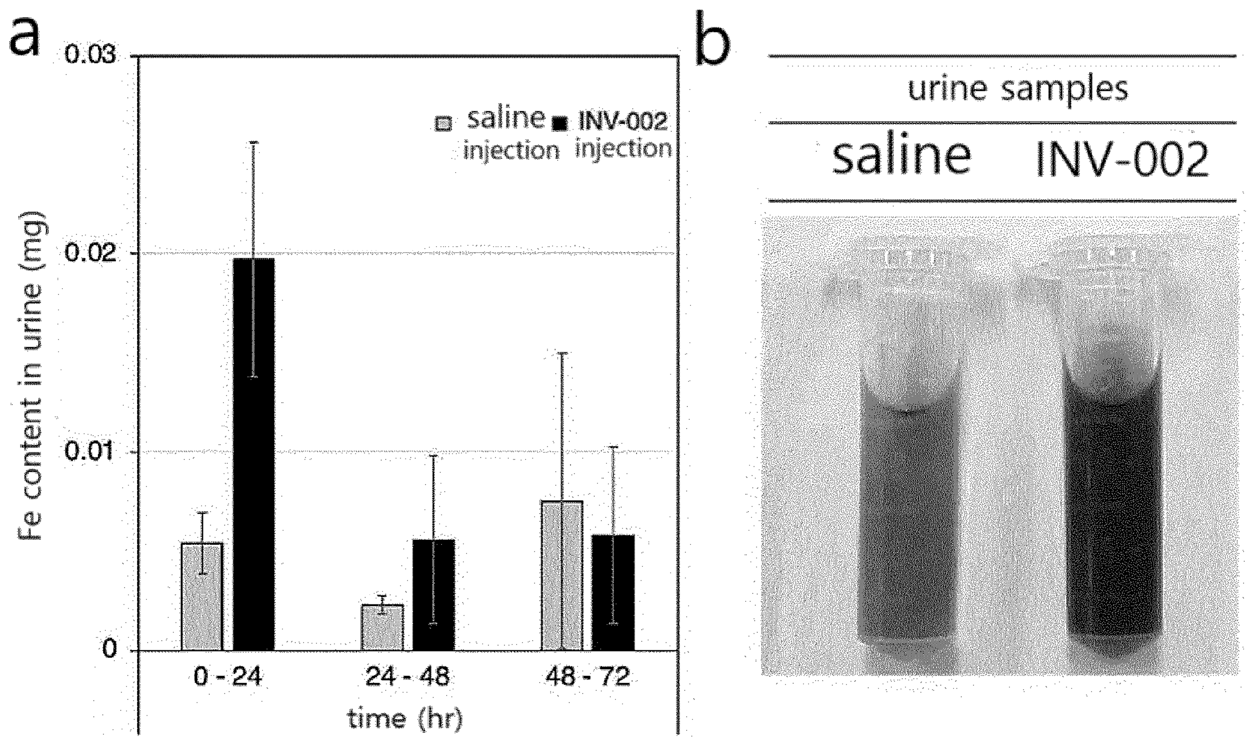
a
0.03
0.02
0.01
0
Fe content in urine (mg)
saline injection
INV-002 injection
0 - 24
24 - 48
48 - 72
time (hr)
b
urine samples
saline
INV-002

FIG. 25

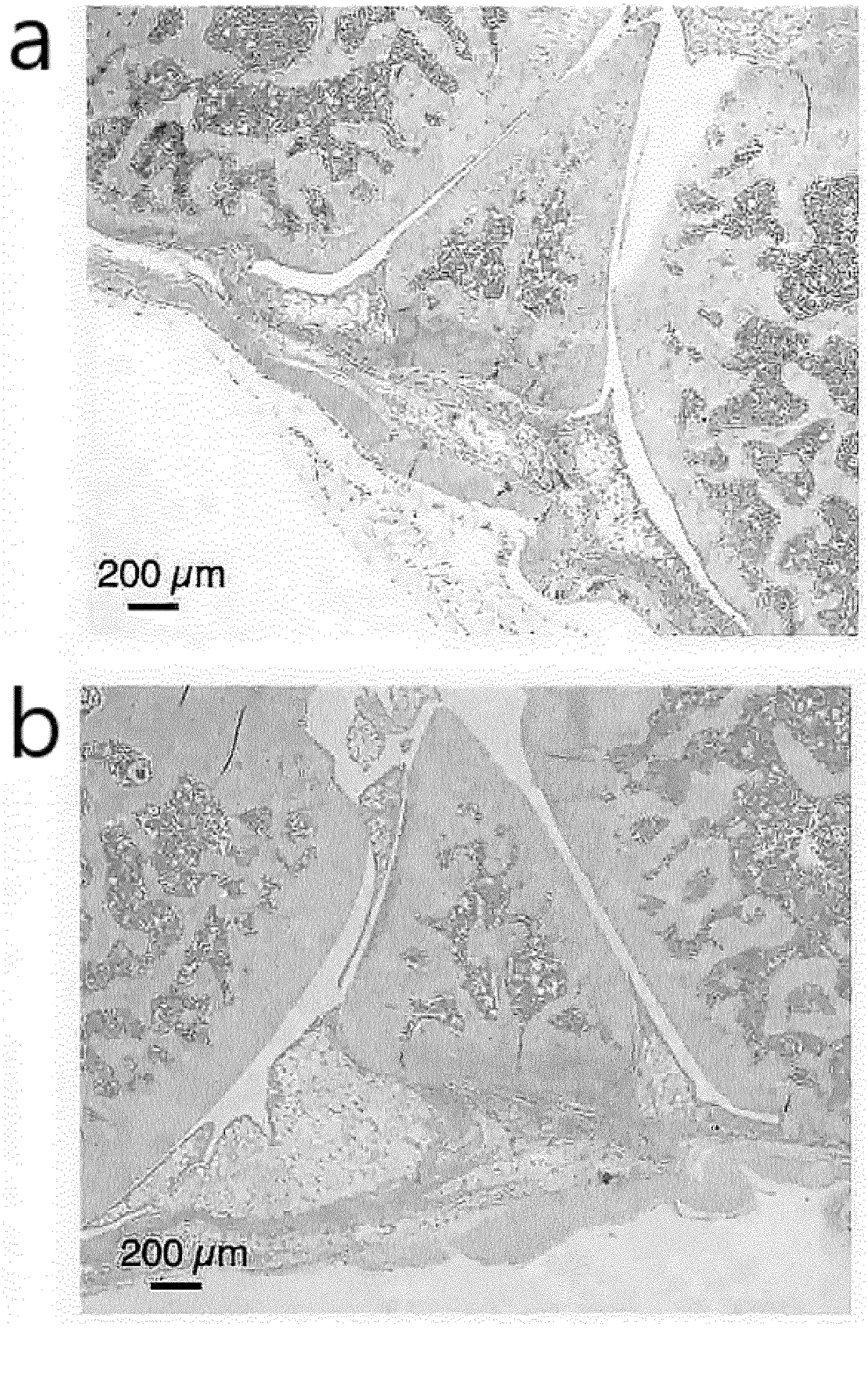

FIG. 26

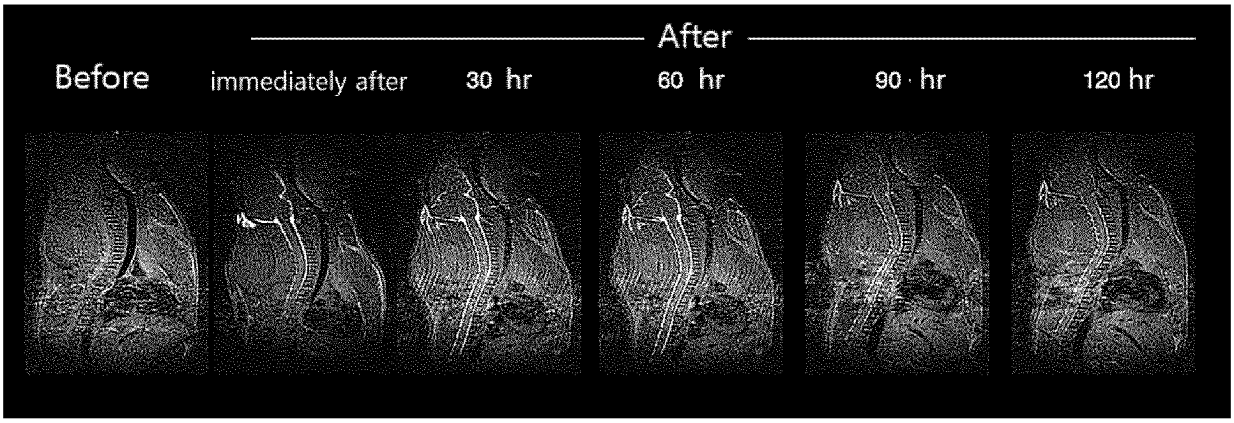
After
Before
immediately after
30 hr
60 hr
90 hr
120 hr

FIG. 27

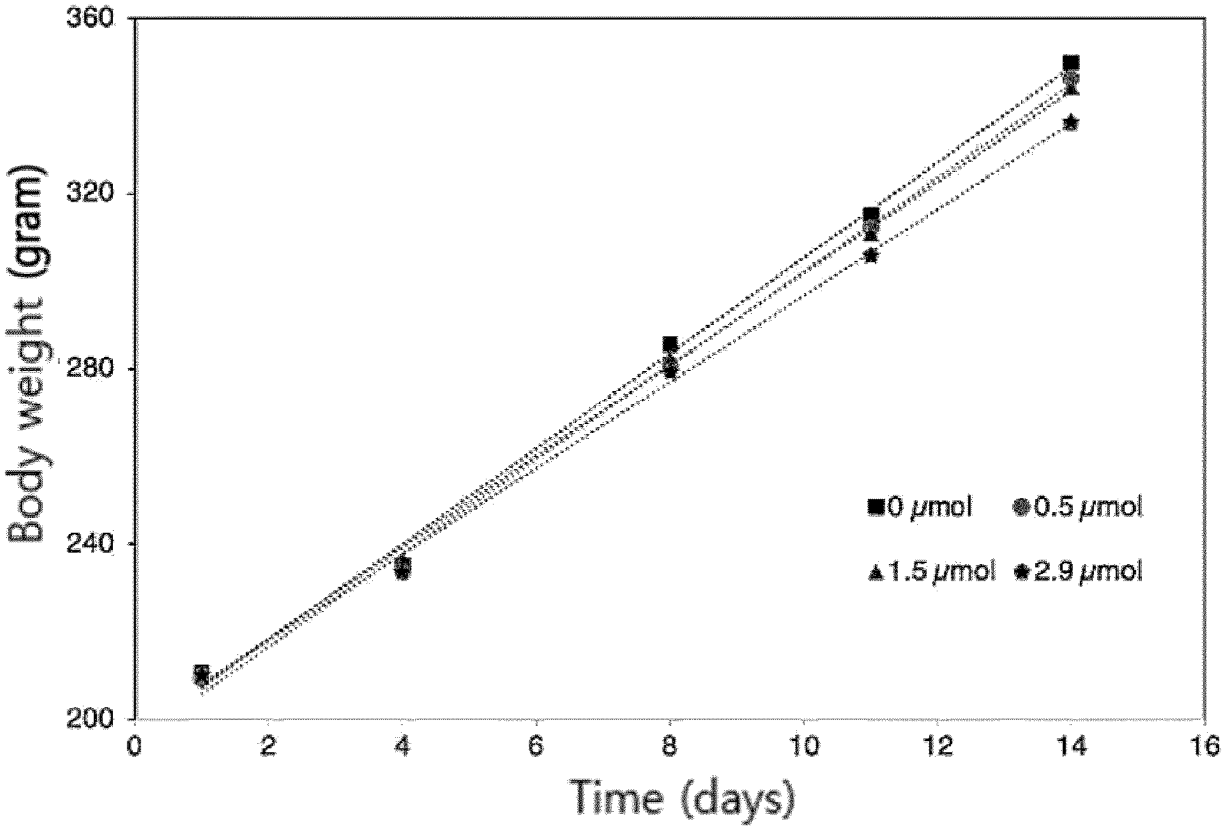
360
320
280
240
200
Body weight (gram)
0
2
4
6
8
10
12
14
16
Time (days)
0 μmol
0.5 μmol
1.5 μmol
2.9 μmol

FIG. 28

| **Dose** (Fe mg/head) | **ALT** (U/L) | **AST** (U/L) | **ALP** (U/L) | **GGT** (U/L) | **BUN** (mg/dL) | **Creatinine** (mg/dL) |
|---|---|---|---|---|---|---|
| 0 | 32.9 ± 2.9 | 68.1 ± 5.5 | 892.0 ± 149.2 | 0.34 ± 0.08 | 11.4 ± 0.7 | 0.42 ± 0.04 |
| 0.16 | 31.0 ± 1.8 | 72.1 ± 8.6 | 794.2 ± 185.7 | 0.26 ± 0.07 | 12.4 ± 1.5 | 0.45 ± 0.03 |

# FIG. 29

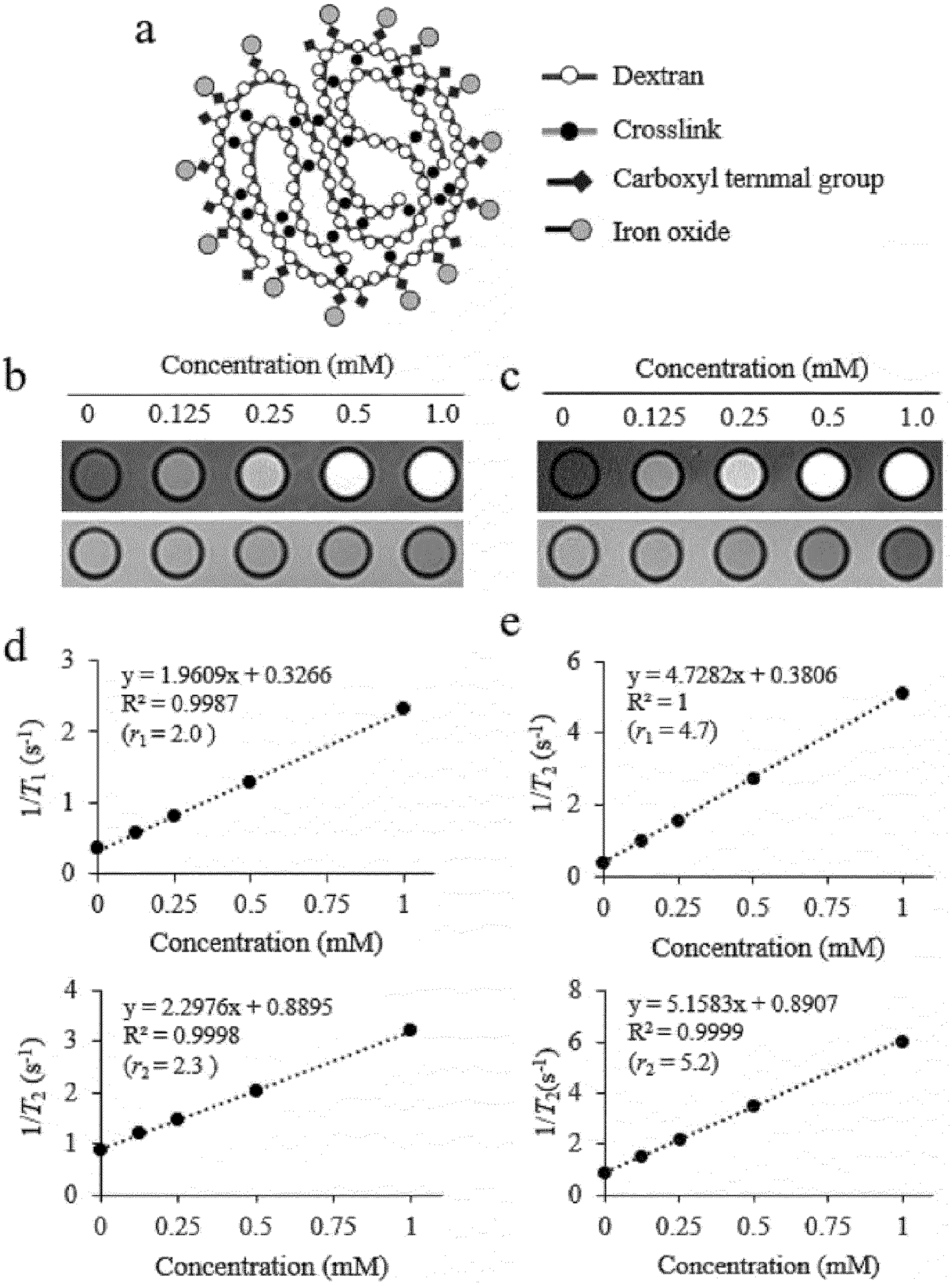
a
Dextran
Crosslink
Carboxyl termmal group
Iron oxide
b
Concentration (mM)
0
0.125
0.25
0.5
1.0
c
Concentration (mM)
0
0.125
0.25
0.5
1.0
d
y = 1.9609x + 0.3266
R² = 0.9987
($r_1$ = 2.0 )
1/$T_1$ ($s^{-1}$)
Concentration (mM)
y = 2.2976x + 0.8895
R² = 0.9998
($r_2$ = 2.3 )
1/$T_2$ ($s^{-1}$)
Concentration (mM)
e
y = 4.7282x + 0.3806
R² = 1
($r_1$ = 4.7)
1/$T_2$ ($s^{-1}$)
Concentration (mM)
y = 5.1583x + 0.8907
R² = 0.9999
($r_2$ = 5.2)
1/$T_2$($s^{-1}$)
Concentration (mM)

FIG. 30

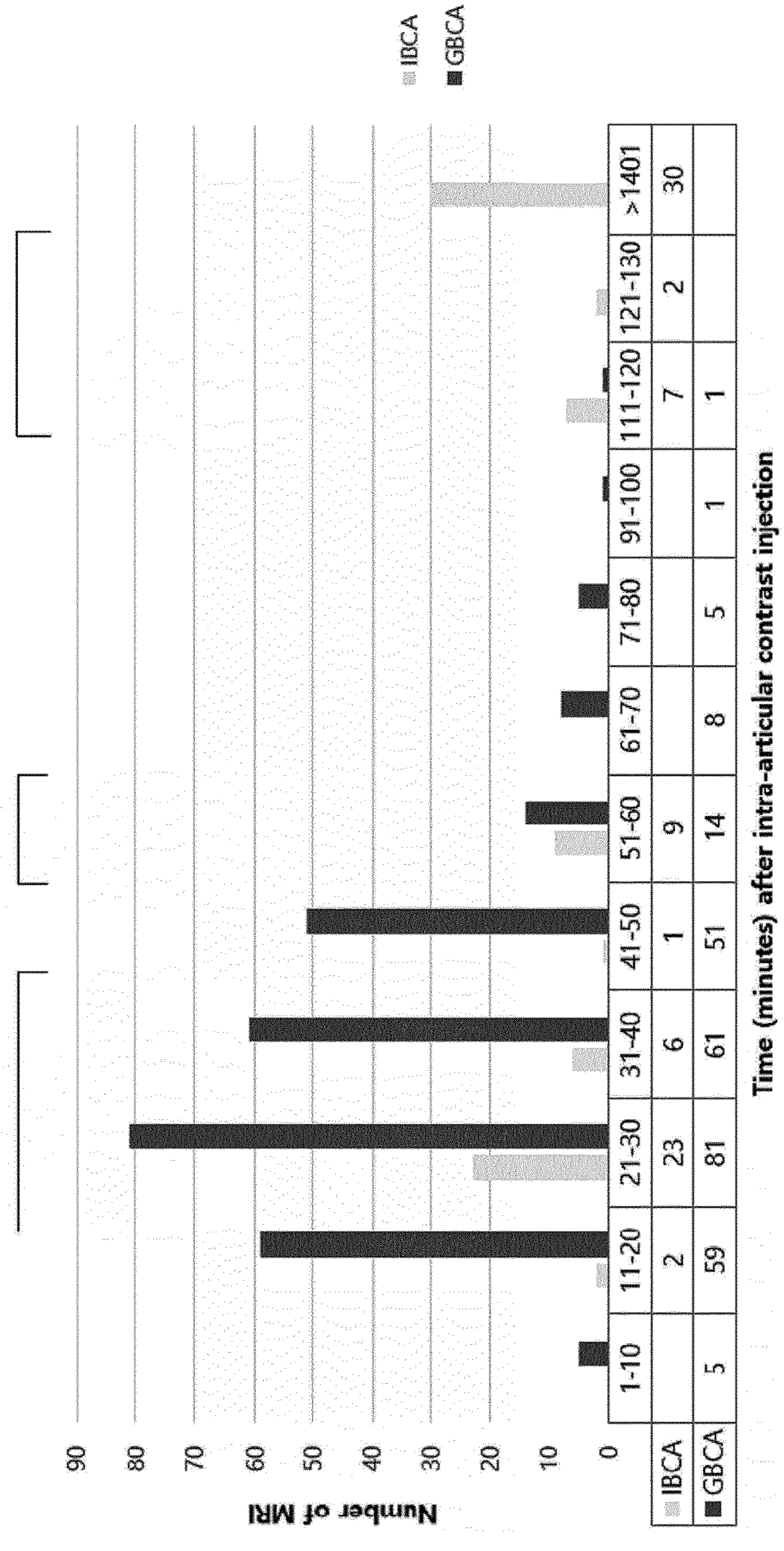
Phase I
Phase II
Phase III
Number of MRI
90
80
70
60
50
40
30
20
10
0
IBCA
GBCA
1-10
11-20
21-30
31-40
41-50
51-60
61-70
71-80
91-100
111-120
121-130
>1401
IBCA: 2, 23, 6, 1, 9, 7, 2, 30
GBCA: 5, 59, 81, 61, 51, 14, 8, 5, 1, 1
Time (minutes) after intra-articular contrast injection

FIG. 31

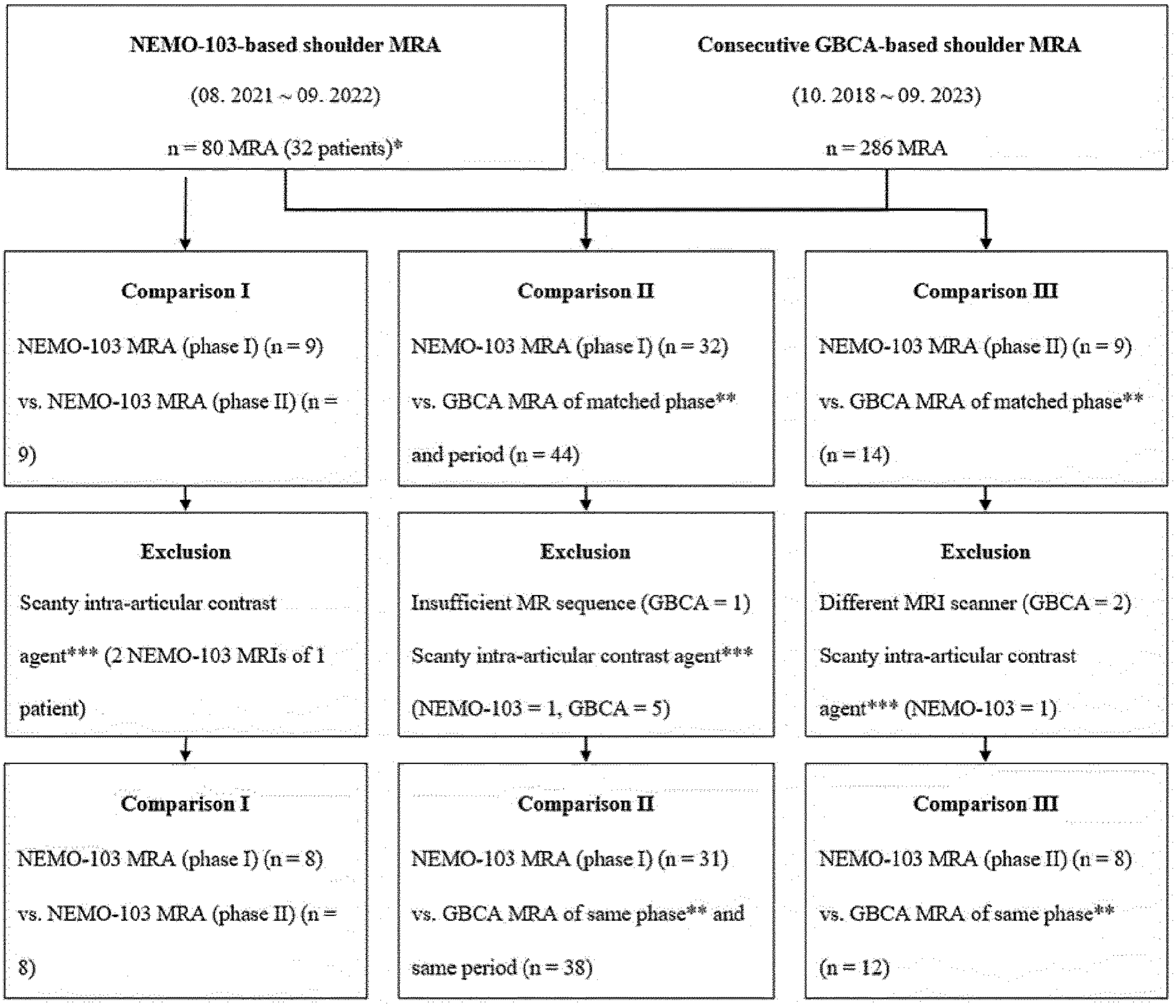
NEMO-103-based shoulder MRA
(08. 2021 ~ 09. 2022)
n = 80 MRA (32 patients)*
Consecutive GBCA-based shoulder MRA
(10. 2018 ~ 09. 2023)
n = 286 MRA
Comparison I
NEMO-103 MRA (phase I) (n = 9) vs. NEMO-103 MRA (phase II) (n = 9)
Comparison II
NEMO-103 MRA (phase I) (n = 32) vs. GBCA MRA of matched phase** and period (n = 44)
Comparison III
NEMO-103 MRA (phase II) (n = 9) vs. GBCA MRA of matched phase** (n = 14)
Exclusion
Scanty intra-articular contrast agent*** (2 NEMO-103 MRIs of 1 patient)
Exclusion
Insufficient MR sequence (GBCA = 1)
Scanty intra-articular contrast agent*** (NEMO-103 = 1, GBCA = 5)
Exclusion
Different MRI scanner (GBCA = 2)
Scanty intra-articular contrast agent*** (NEMO-103 = 1)
Comparison I
NEMO-103 MRA (phase I) (n = 8) vs. NEMO-103 MRA (phase II) (n = 8)
Comparison II
NEMO-103 MRA (phase I) (n = 31) vs. GBCA MRA of same phase** and same period (n = 38)
Comparison III
NEMO-103 MRA (phase II) (n = 8) vs. GBCA MRA of same phase** (n = 12)

FIG. 32

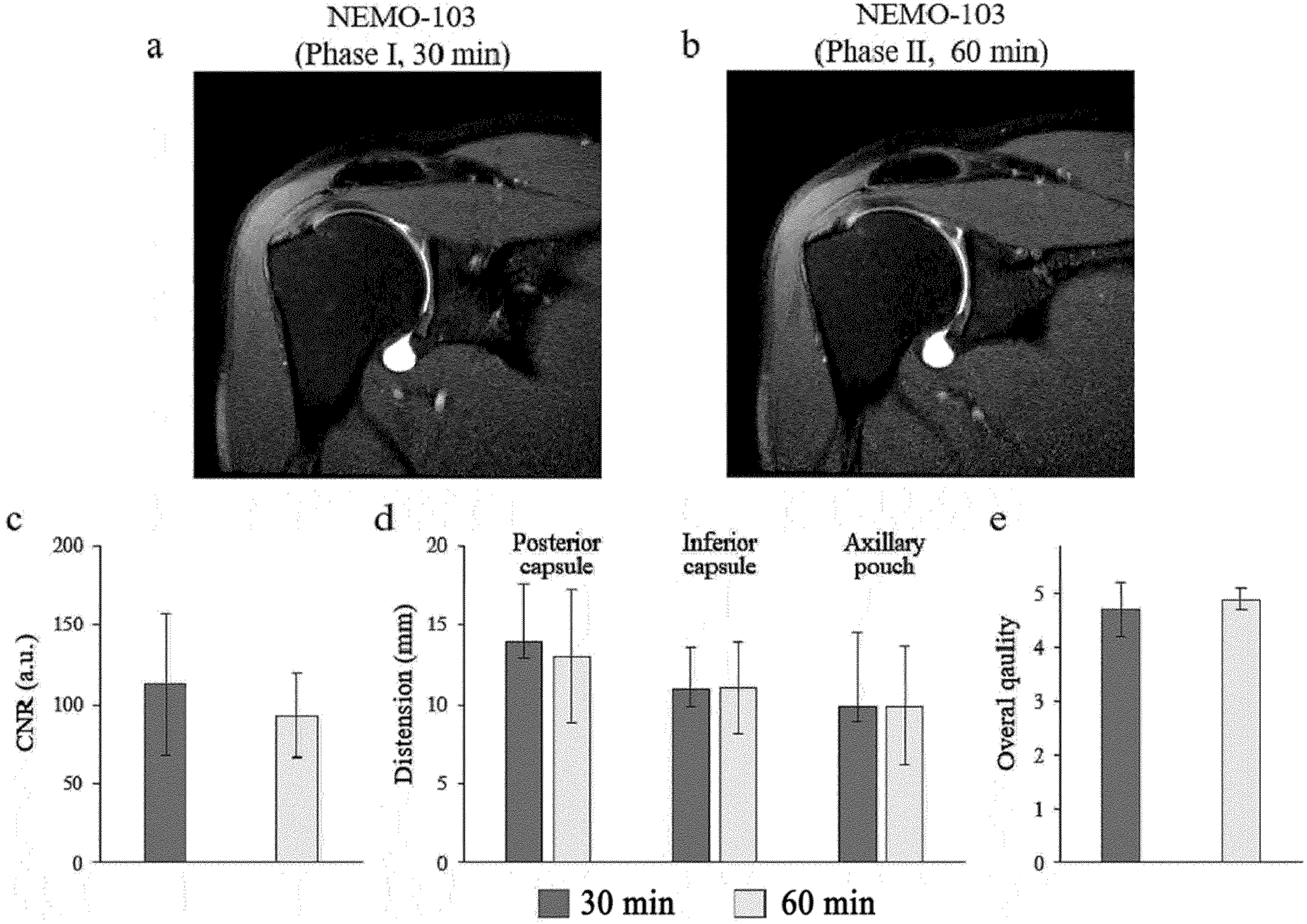
a
NEMO-103
(Phase I, 30 min)
b
NEMO-103
(Phase II, 60 min)
c
CNR (a.u.)
200
150
100
50
0
d
Distension (mm)
20
15
10
5
0
Posterior capsule
Inferior capsule
Axillary pouch
e
Overal qaulity
5
4
3
2
1
0
30 min
60 min

FIG. 33

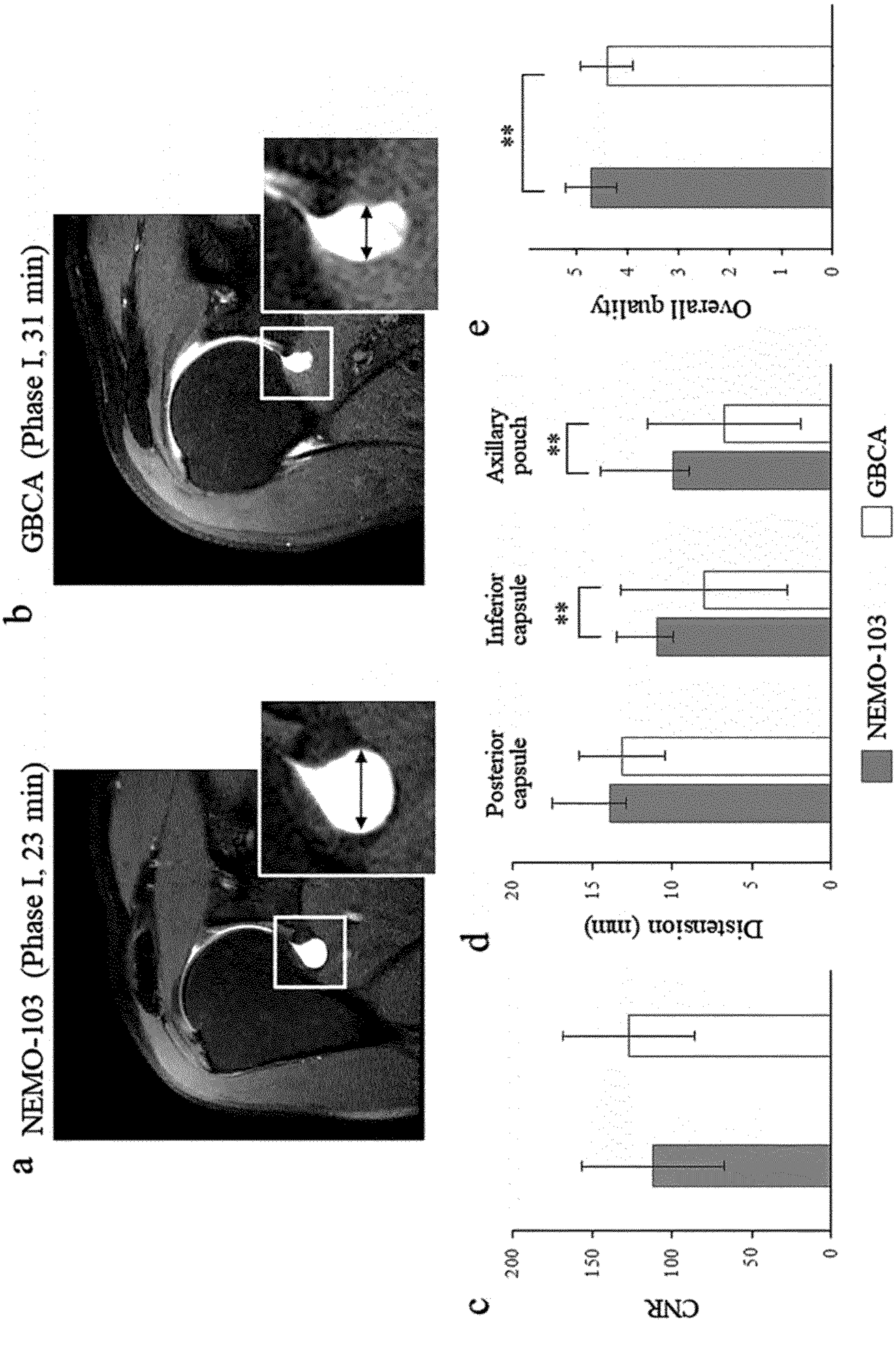
a
NEMO-103 (Phase I, 23 min)
b
GBCA (Phase I, 31 min)
c
CNR
0
50
100
150
200
d
Distension (mm)
0
5
10
15
20
Posterior capsule
Inferior capsule
Axillary pouch
**
**
e
Overall quality
0
1
2
3
4
5
**
NEMO-103
GBCA

FIG. 34

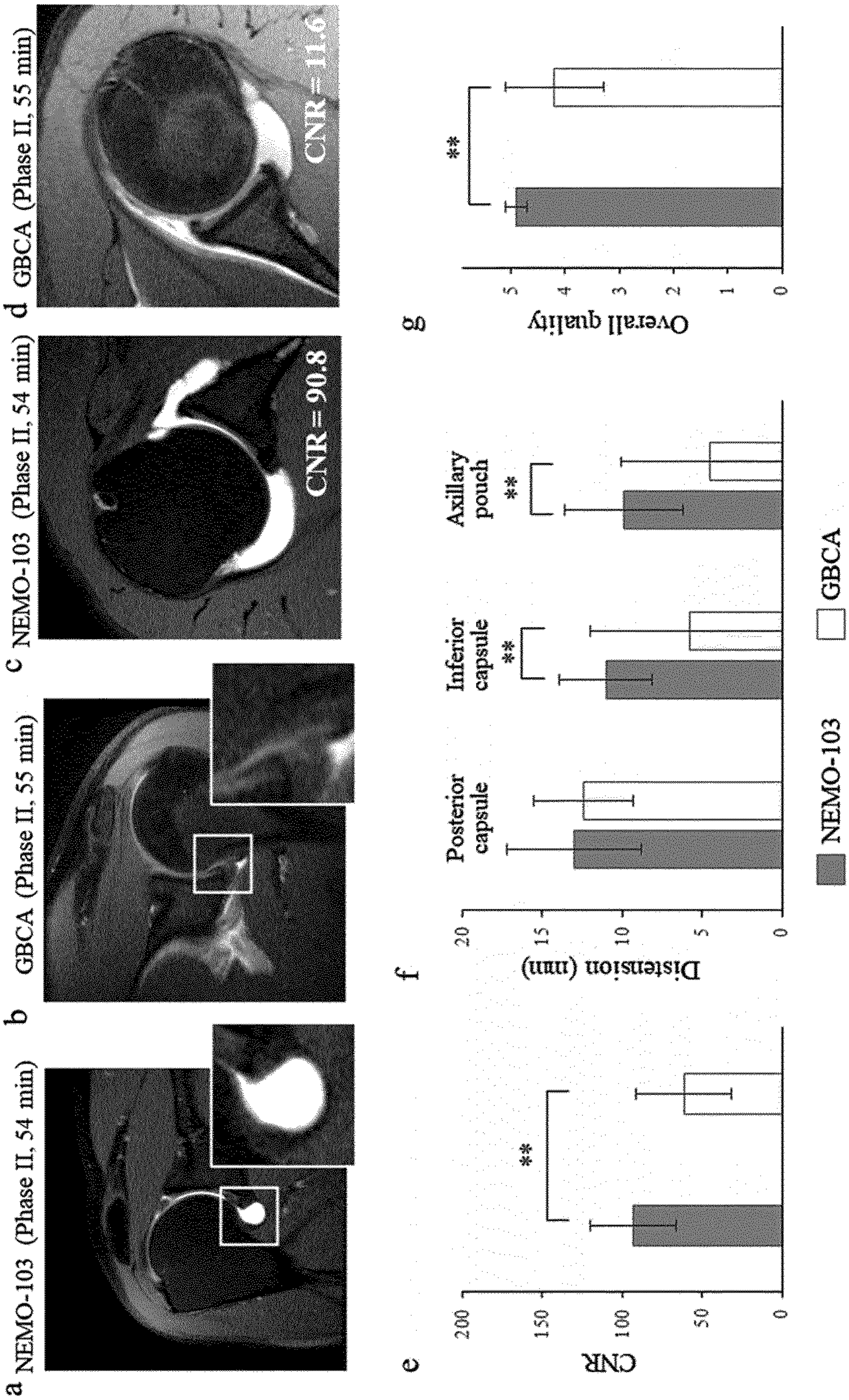
a NEMO-103 (Phase II, 54 min)
b GBCA (Phase II, 55 min)
c NEMO-103 (Phase II, 54 min)
CNR = 90.8
d GBCA (Phase II, 55 min)
CNR = 11.6
e
CNR
0
50
100
150
200
**
f
Distension (mm)
0
5
10
15
20
Posterior capsule
Inferior capsule
Axillary pouch
**
**
g
Overall quality
0
1
2
3
4
5
**
NEMO-103
GBCA

FIG. 35

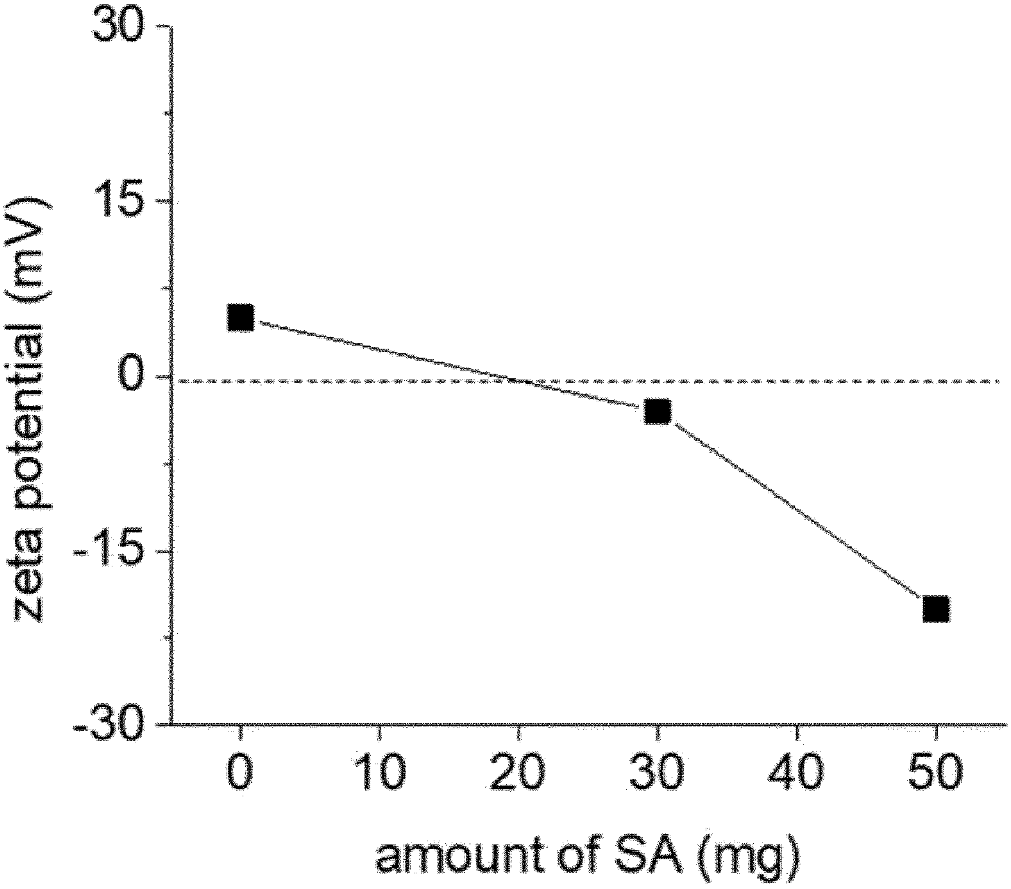

FIG. 36

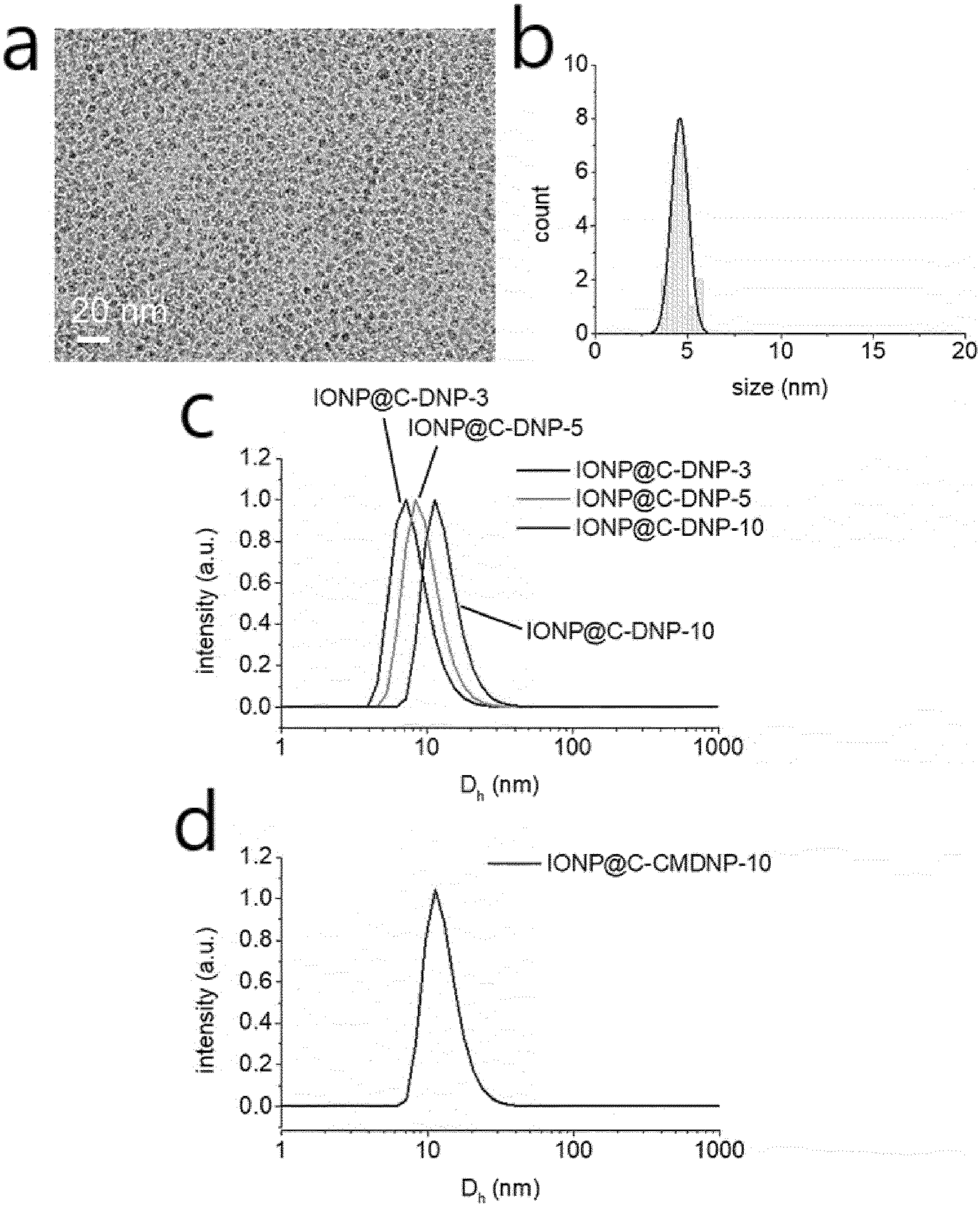
a
20 nm
b
count
size (nm)
c
IONP@C-DNP-3
IONP@C-DNP-5
IONP@C-DNP-10
intensity (a.u.)
$D_h$ (nm)
d
IONP@C-CMDNP-10
intensity (a.u.)
$D_h$ (nm)

FIG. 37

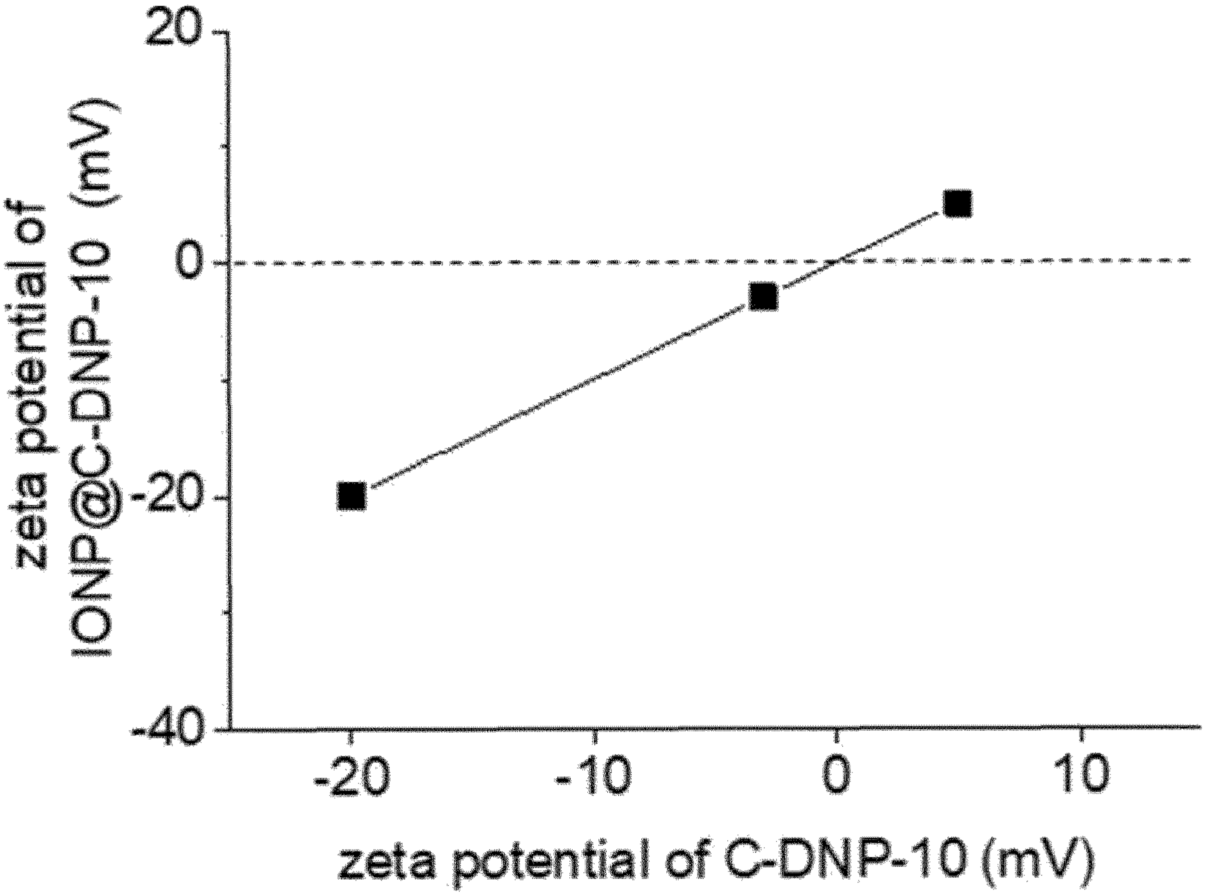
20
0
-20
-40
-20
-10
0
10
zeta potential of IONP@C-DNP-10 (mV)
zeta potential of C-DNP-10 (mV)

FIG. 38

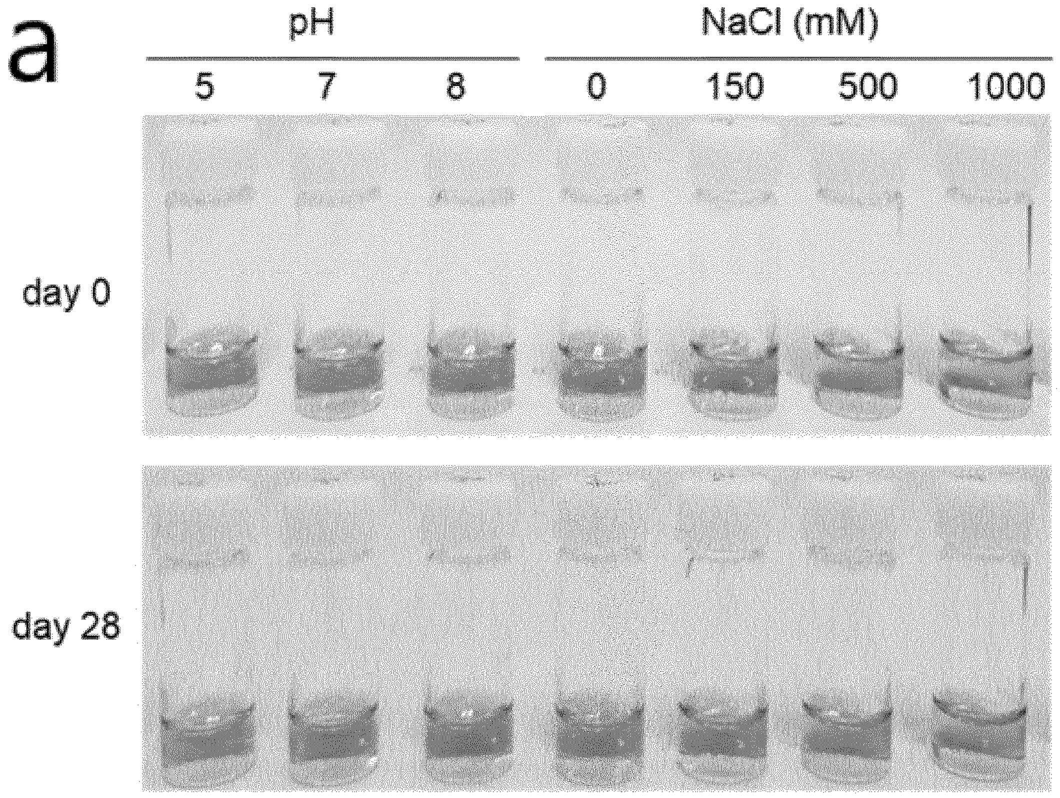
a
pH
5
7
8
NaCl (mM)
0
150
500
1000
day 0
day 28

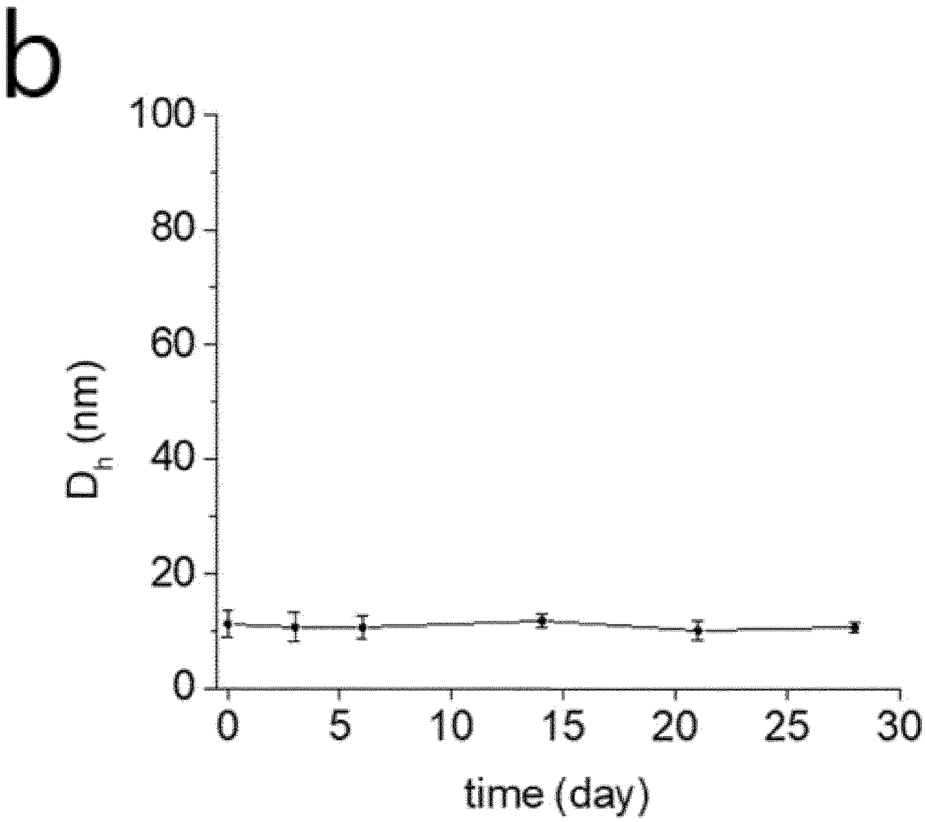
b
100
80
60
40
20
0
$D_h$ (nm)
0
5
10
15
20
25
30
time (day)

FIG. 39

a

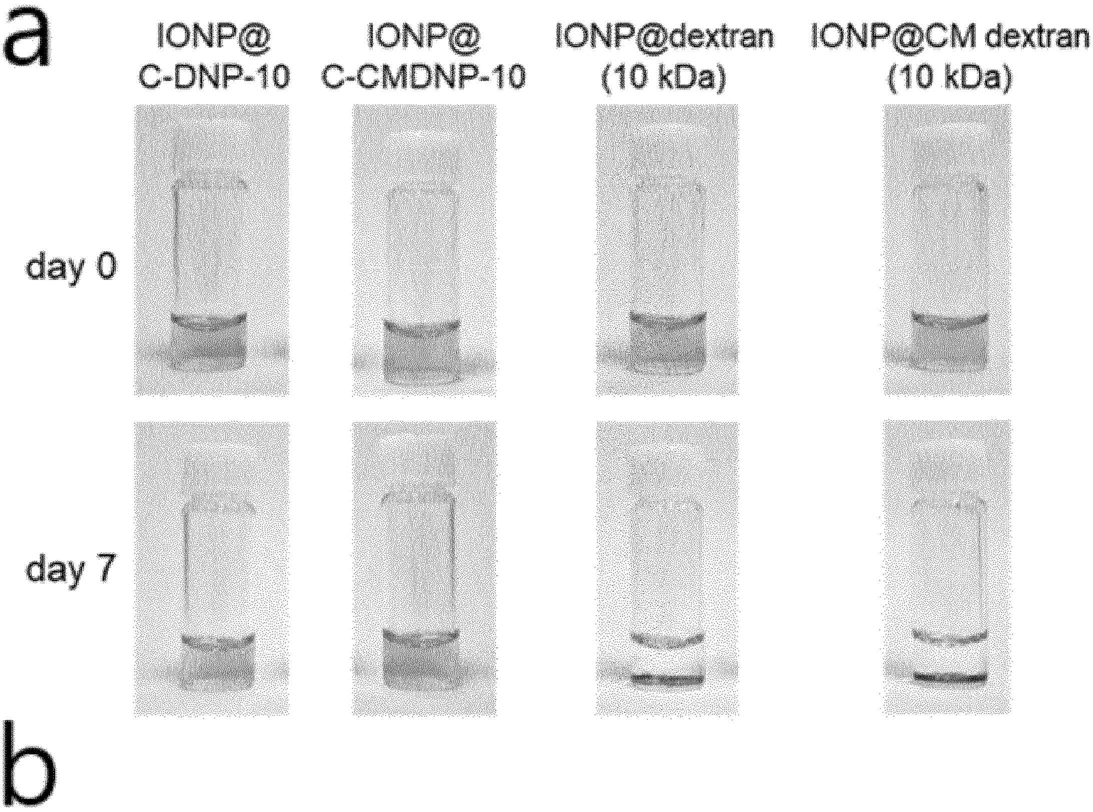
IONP@
C-DNP-10
IONP@
C-CMDNP-10
IONP@dextran
(10 kDa)
IONP@CM dextran
(10 kDa)
day 0
day 7

b

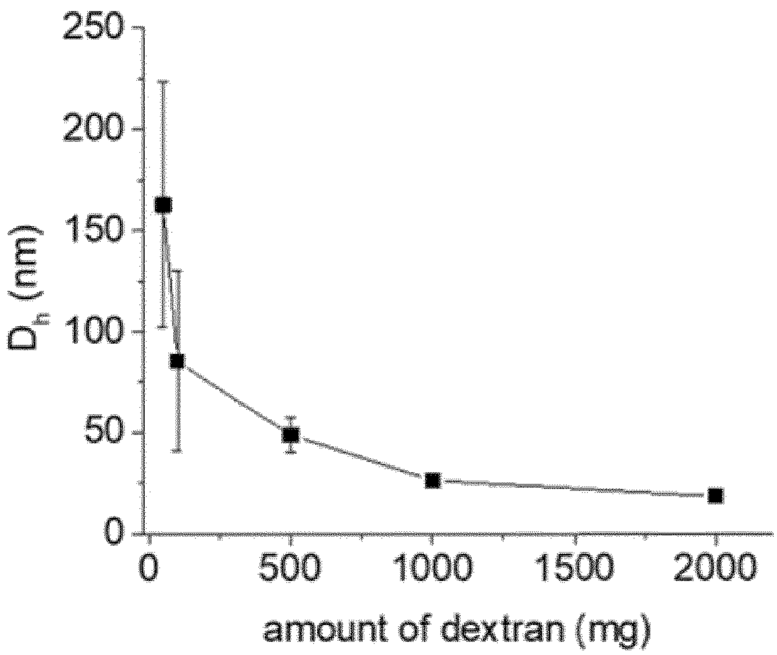
250
200
150
100
50
0
$D_h$ (nm)
0
500
1000
1500
2000
amount of dextran (mg)

FIG. 40

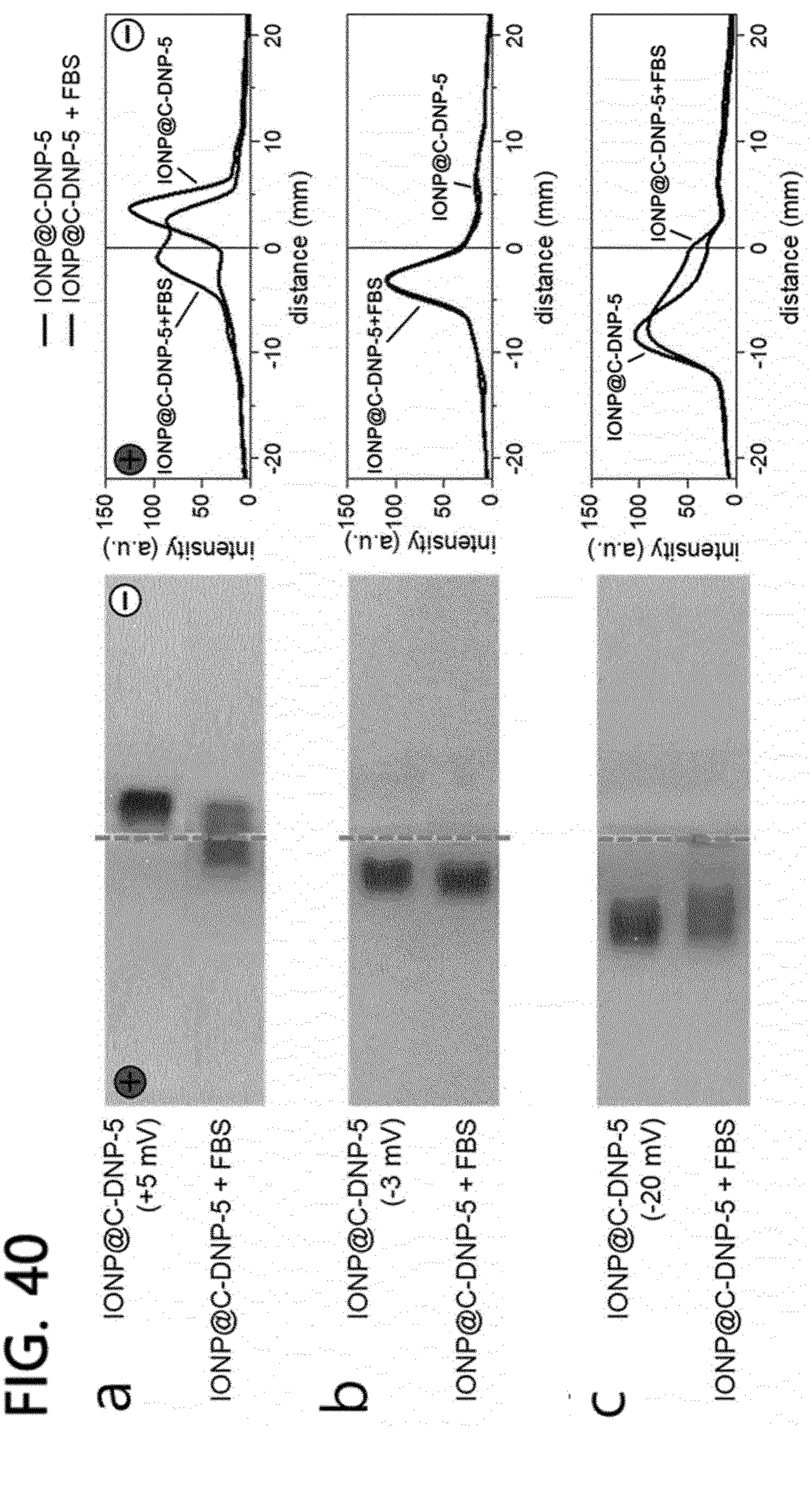
a
IONP@C-DNP-5 (+5 mV)
IONP@C-DNP-5 + FBS
b
IONP@C-DNP-5 (-3 mV)
IONP@C-DNP-5 + FBS
c
IONP@C-DNP-5 (-20 mV)
IONP@C-DNP-5 + FBS
IONP@C-DNP-5
IONP@C-DNP-5 + FBS
IONP@C-DNP-5+FBS
intensity (a.u.)
distance (mm)
150
100
50
0
-20
-10
0
10
20

FIG. 41

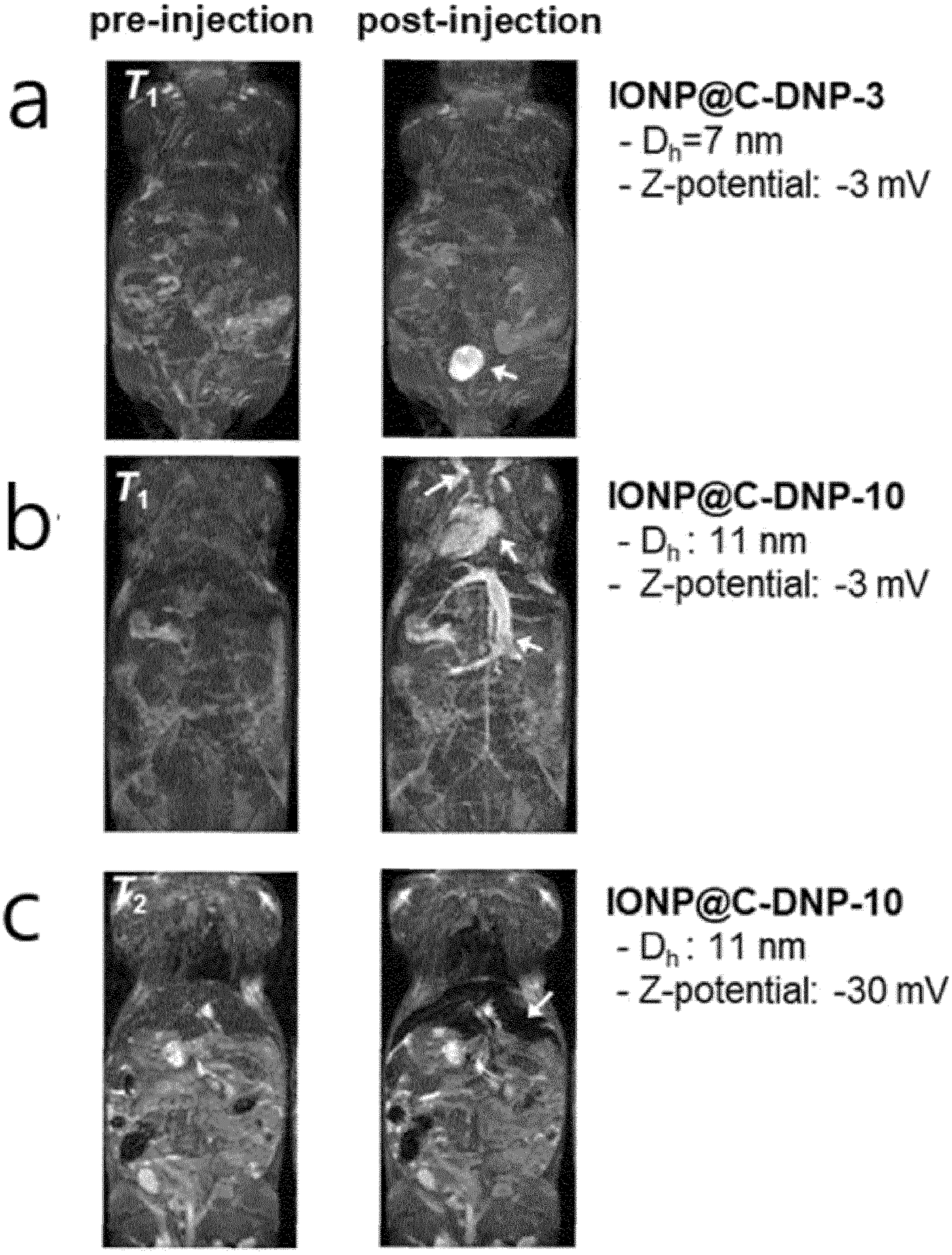
pre-injection
post-injection
a
$T_1$
IONP@C-DNP-3
- $D_h$=7 nm
- Z-potential: -3 mV
b
$T_1$
IONP@C-DNP-10
- $D_h$: 11 nm
- Z-potential: -3 mV
c
$T_2$
IONP@C-DNP-10
- $D_h$: 11 nm
- Z-potential: -30 mV

# FIG. 42

a-i

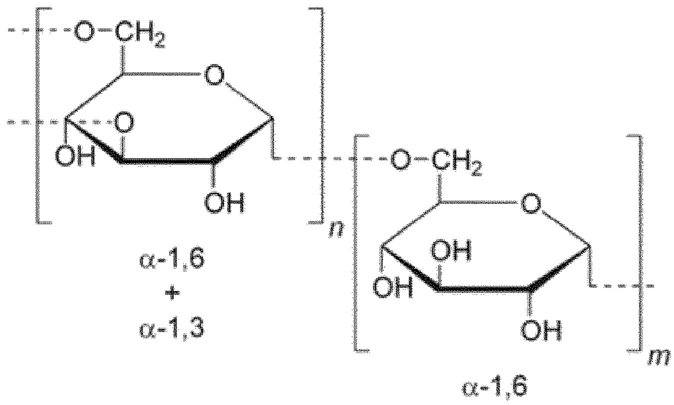


a-ii

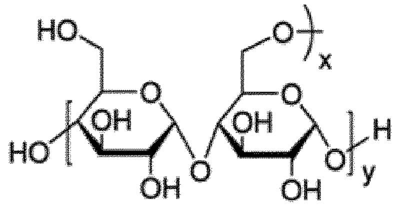

Maltodextrin

b-i

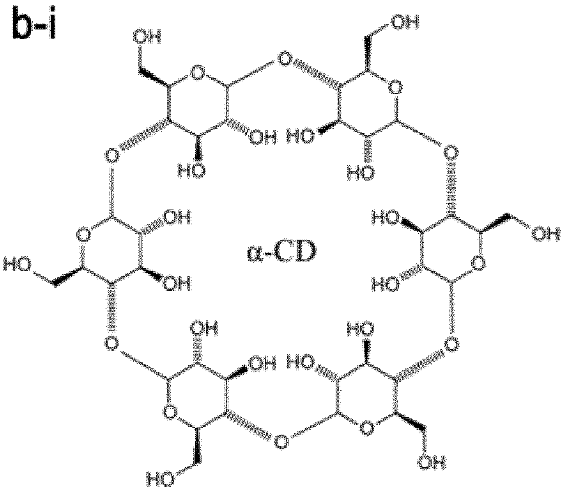


α-cyclodextrin

b-ii

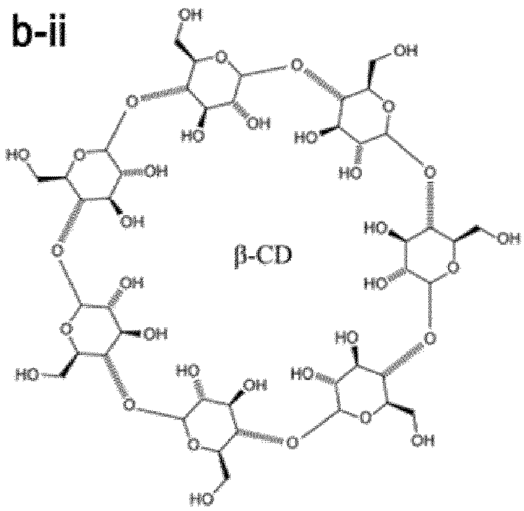


β-cyclodextrin

c

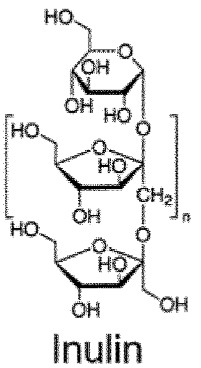

Inulin

FIG. 43

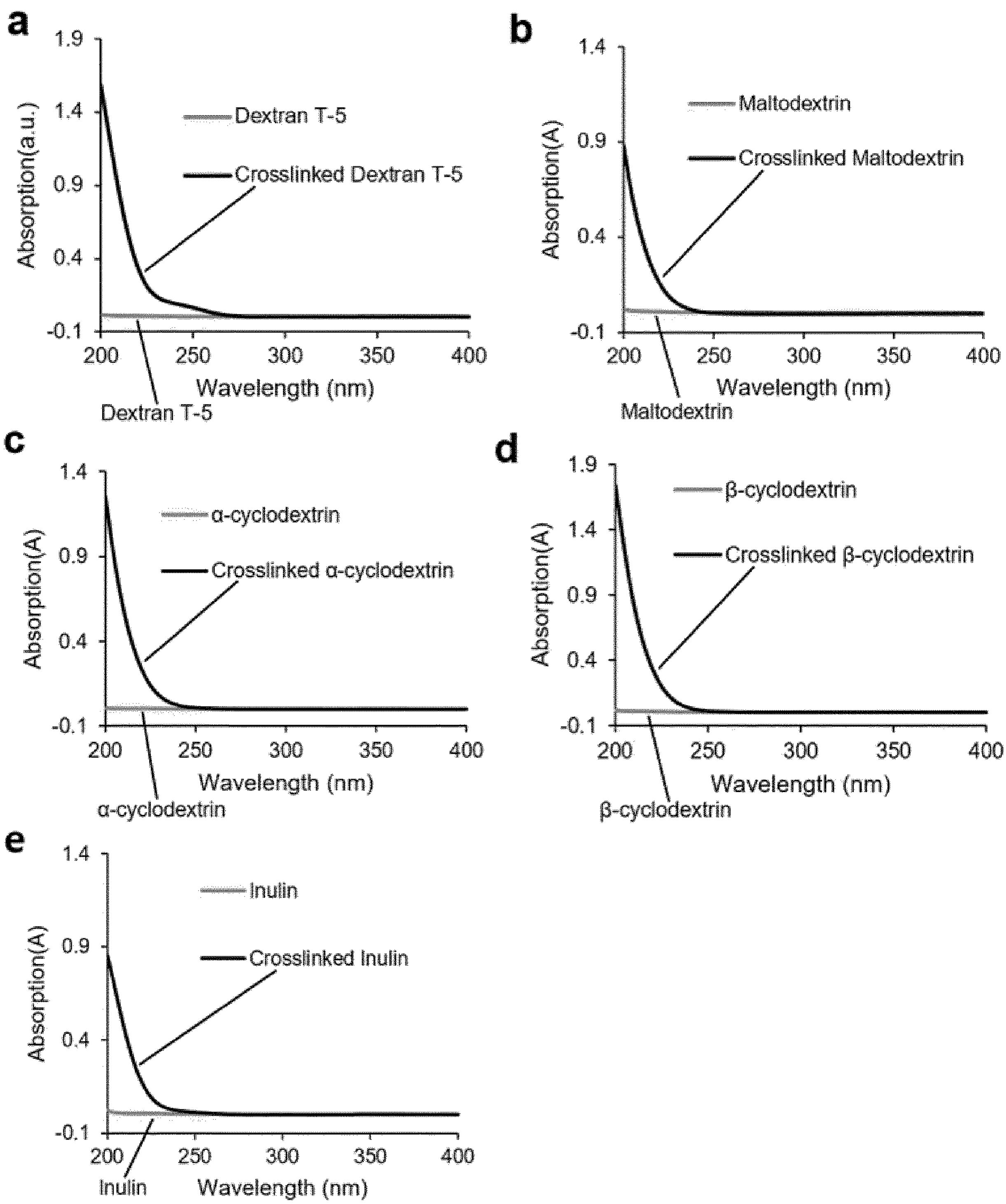
a
1.9
1.4
0.9
0.4
-0.1
Absorption(a.u.)
Dextran T-5
Crosslinked Dextran T-5
200
250
300
350
400
Wavelength (nm)
Dextran T-5
b
1.4
0.9
0.4
-0.1
Absorption(A)
Maltodextrin
Crosslinked Maltodextrin
200
250
300
350
400
Wavelength (nm)
Maltodextrin
c
1.4
0.9
0.4
-0.1
Absorption(A)
α-cyclodextrin
Crosslinked α-cyclodextrin
200
250
300
350
400
Wavelength (nm)
α-cyclodextrin
d
1.9
1.4
0.9
0.4
-0.1
Absorption(A)
β-cyclodextrin
Crosslinked β-cyclodextrin
200
250
300
350
400
Wavelength (nm)
β-cyclodextrin
e
1.4
0.9
0.4
-0.1
Absorption(A)
Inulin
Crosslinked Inulin
200
250
300
350
400
Inulin
Wavelength (nm)

FIG. 44

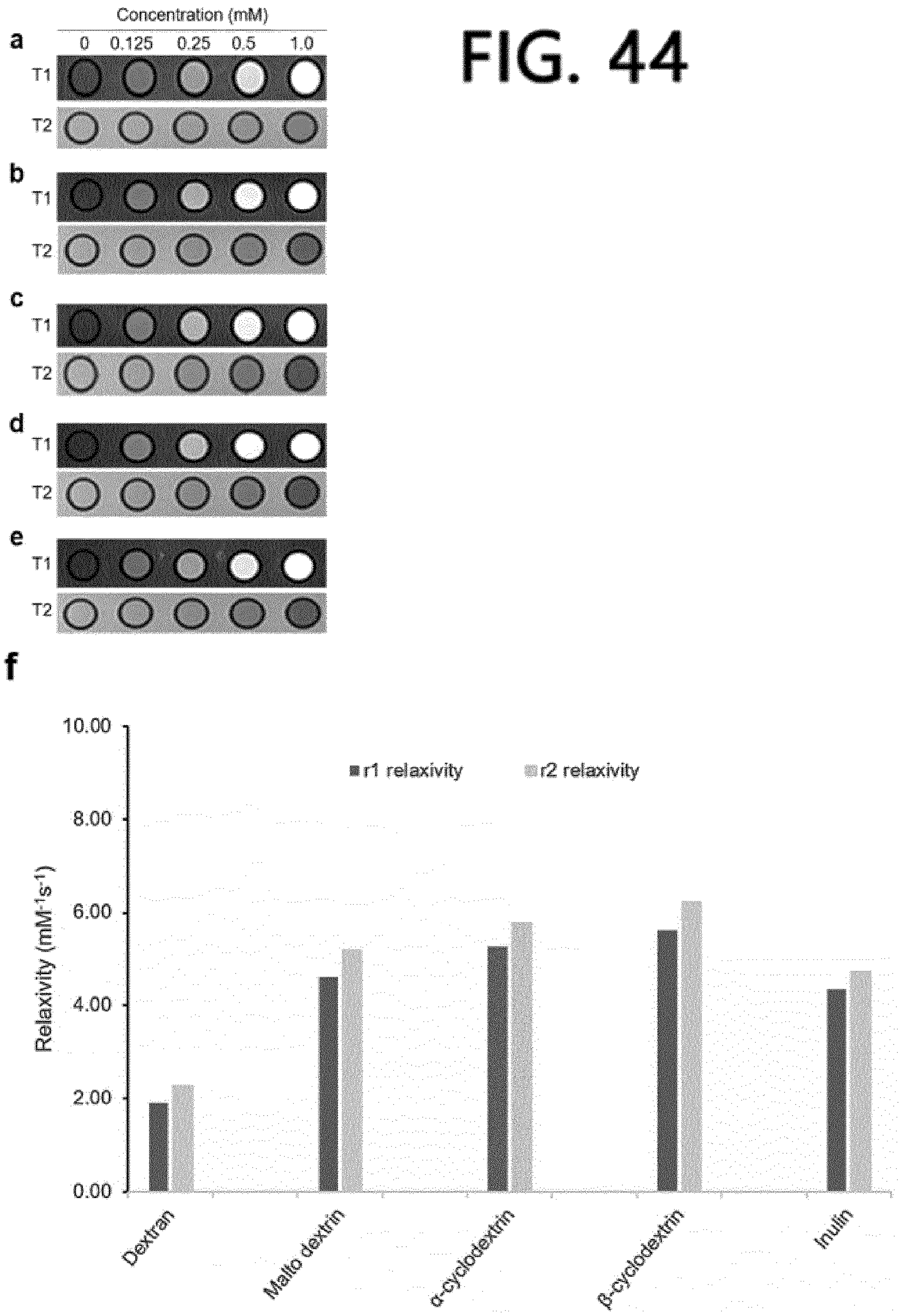
a
b
c
d
e
f
Concentration (mM)
0
0.125
0.25
0.5
1.0
T1
T2
r1 relaxivity
r2 relaxivity
Relaxivity (mM-1s-1)
10.00
8.00
6.00
4.00
2.00
0.00
Dextran
Malto dextrin
α-cyclodextrin
β-cyclodextrin
Inulin

FIG. 45

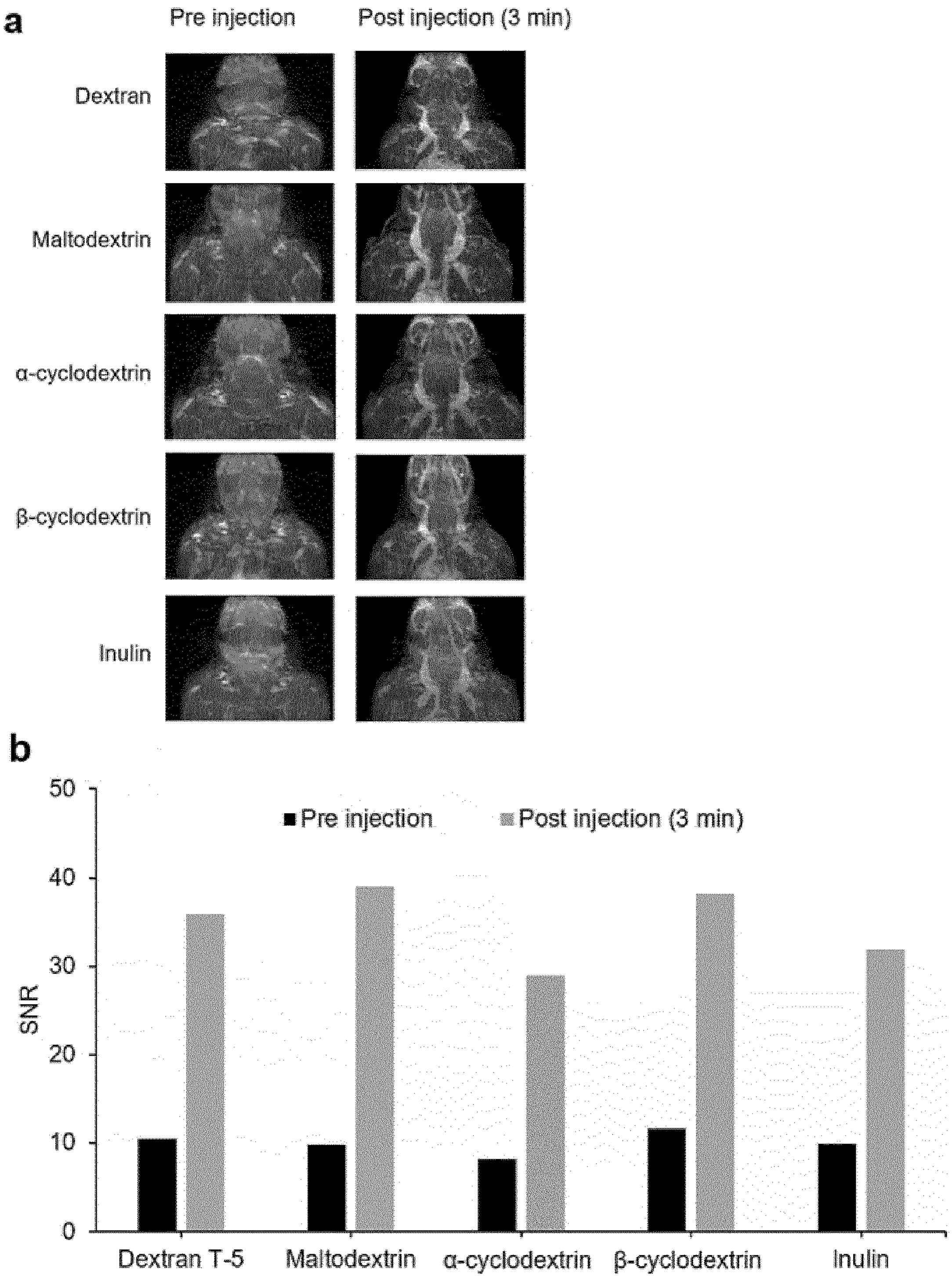
a
Pre injection
Post injection (3 min)
Dextran
Maltodextrin
α-cyclodextrin
β-cyclodextrin
Inulin
b
50
40
30
20
10
0
SNR
Pre injection
Post injection (3 min)
Dextran T-5
Maltodextrin
α-cyclodextrin
β-cyclodextrin
Inulin

FIG. 46

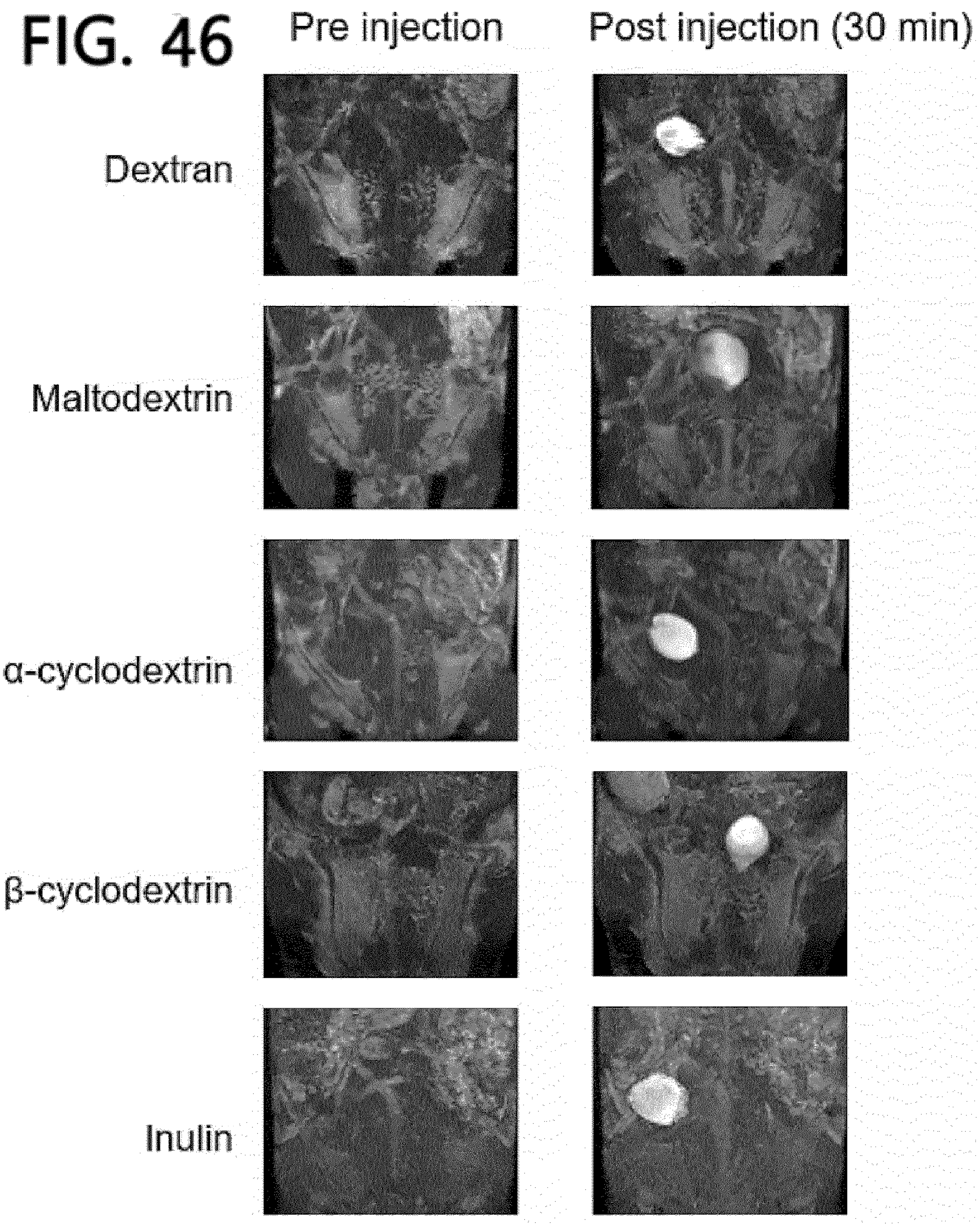
Pre injection
Post injection (30 min)
Dextran
Maltodextrin
α-cyclodextrin
β-cyclodextrin
Inulin

## INTERNATIONAL SEARCH REPORT

International application No.
**PCT/KR2024/001958**

**A. CLASSIFICATION OF SUBJECT MATTER**

**A61K 49/18**(2006.01)i; **A61K 9/00**(2006.01)i; **A61K 49/12**(2006.01)i; **A61K 47/69**(2017.01)i

According to International Patent Classification (IPC) or to both national classification and IPC

**B. FIELDS SEARCHED**

Minimum documentation searched (classification system followed by classification symbols)

A61K 49/18(2006.01); A61K 49/06(2006.01); A61K 49/08(2006.01); A61K 49/10(2006.01); A61M 27/00(2006.01); B82Y 15/00(2011.01); G01R 33/48(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 자기 공명 림프관 조영술(magnetic resonance lymphangiography), 정맥오염(venous contamination), 모세혈관(capillary), 덱스트란(dextran), 나노입자(nano particle), 림프관(lymphatic vessel), T1-MRI, 친수성기(hydrophilic functional group)

**C. DOCUMENTS CONSIDERED TO BE RELEVANT**

| Category* | Citation of document, with indication, where appropriate, of the relevant passages | Relevant to claim No. |
|---|---|---|
| A | WO 2021-146589 A1 (NXT BIOMEDICAL, LLC) 22 July 2021 (2021-07-22)<br>See paragraphs [0147]-[0150]. | 1-26 |
| A | KR 10-2013-0008095 A (HANWHA CHEMICAL CORPORATION) 22 January 2013 (2013-01-22)<br>See entire document. | 1-26 |
| A | KR 10-2018-0088775 A (INDUSTRY-ACADEMIC COOPERATION FOUNDATION, YONSEI UNIVERSITY) 07 August 2018 (2018-08-07)<br>See entire document. | 1-26 |
| A | US 2017-0143853 A1 (UNIVERSITY OF WASHINGTON) 25 May 2017 (2017-05-25)<br>See entire document. | 1-26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 May 2024** | **13 May 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2024/001958

C. DOCUMENTS CONSIDERED TO BE RELEVANT

| Category* | Citation of document, with indication, where appropriate, of the relevant passages | Relevant to claim No. |
|---|---|---|
| A | KR 10-2013-0122585 A (SEOUL NATIONAL UNIVERSITY R&DB FOUNDATION et al.) 07 November 2013 (2013-11-07) See entire document. | 1-26 |

Form PCT/ISA/210 (second sheet) (July 2022)

**INTERNATIONAL SEARCH REPORT**
**Information on patent family members**

International application No.
**PCT/KR2024/001958**

| Patent document cited in search report | Publication date (day/month/year) | Patent family member(s) | Publication date (day/month/year) |
|---|---|---|---|
| WO 2021-146589 A1 | 22 July 2021 | CN 115279446 A | 01 November 2022 |
| | | EP 4090409 A1 | 23 November 2022 |
| | | EP 4090409 A4 | 27 March 2024 |
| | | JP 2023-511071 A | 16 March 2023 |
| | | KR 10-2590683 B1 | 18 October 2023 |
| | | US 2023-0042304 A1 | 09 February 2023 |
| KR 10-2013-0008095 A | 22 January 2013 | CN 103458932 A | 18 December 2013 |
| | | CN 103458932 B | 09 December 2015 |
| | | EP 2723392 A2 | 30 April 2014 |
| | | EP 2723392 A4 | 17 December 2014 |
| | | EP 2723392 B1 | 28 September 2016 |
| | | JP 2014-514301 A | 19 June 2014 |
| | | JP 5799161 B2 | 21 October 2015 |
| | | KR 10-1721570 B1 | 30 March 2017 |
| | | US 2014-0023594 A1 | 23 January 2014 |
| | | US 8906346 B2 | 09 December 2014 |
| | | WO 2012-177039 A2 | 27 December 2012 |
| | | WO 2012-177039 A3 | 04 April 2013 |
| KR 10-2018-0088775 A | 07 August 2018 | CN 115279446 A | 01 November 2022 |
| | | EP 4090409 A1 | 23 November 2022 |
| | | EP 4090409 A4 | 27 March 2024 |
| | | JP 2023-511071 A | 16 March 2023 |
| | | KR 10-2590683 B1 | 18 October 2023 |
| | | US 2023-0042304 A1 | 09 February 2023 |
| US 2017-0143853 A1 | 25 May 2017 | US 10814019 B2 | 27 October 2020 |
| | | WO 2016-004061 A1 | 07 January 2016 |
| KR 10-2013-0122585 A | 07 November 2013 | KR 10-2020870 B1 | 16 September 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014107055 A
- KR 1020210104405